# EUROPÄISCHE PATENTSCHRIFT

(11) **EP 2 576 699 B1**
(45) Veröffentlichungstag und Bekanntmachung des Hinweises auf die Patenterteilung: **12.07.2017**
(21) Anmeldenummer: 11729930.5
(22) Anmeldetag: 30.05.2011
(51) Int. Cl.: C09B 23/06, G01N 33/533, G01N 33/58, A61K 31/4035, A61K 49/00

(54) **NAPHTHOCYANINE ALS KONTRASTMITTEL**
NAPHTHOCYANINES FOR USE AS CONTRAST AGENTS
NAPHTHOCYANINES SERVANT D'AGENTS DE CONTRASTE

(30) Priorität: 31.05.2010 DE 102010022110
(43) Veröffentlichungstag der Anmeldung: 10.04.2013
(73) Patentinhaber: PHARMPUR GmbH, 86343 Königsbrunn (DE)
(72) Erfinder: LANGHALS, Heinz, 85521 Ottobrunn (DE); LAUBICHLER, Peter, 81377 München (DE); HARITOGLOU, Christos, 80469 München (DE); VARJA, Ana, 81241 München (DE)
(74) Vertreter: Leissler-Gerstl, Gabriele
(86) Internationale Anmeldenummer: PCT/EP2011/058837
(87) Internationale Veröffentlichungsnummer: WO 2011/151287

(56) Entgegenhaltungen:
- WO-A1-97/13810
- WO-A1-2007/028163
- WO-A1-2009/121055
- US-A1- 2009 305 410
- HEINZ LANGHALS ET AL: "Cyanine Dyes as Optical Contrast Agents for Ophthalmological Surgery", JOURNAL OF MEDICINAL CHEMISTRY, Bd. 54, Nr. 11, 9. Juni 2011 (2011-06-09), Seiten 3903-3925, XP55010173, ISSN: 0022-2623, DOI: 10.1021/jm2001986

## Beschreibung

Die Augenchirurgie hat in den vergangenen Jahren erhebliche Fortschritte gemacht und stellt an den Chirurgen hohe Anforderungen. Für die milcrochirurgiechen Eingriffe wird das korrekte Erkennen auch sehr kleiner und transparenter Strukturen der Ziel-Gewebe ein wach sendes Problem. Weitere Fortschritte in den chirurgischen Möglichkeiten sind durch eine Erhöhung des visuellen Kontrasts zu erreichen. Eine solche Erhöhung kann durch optische Kontrastmittel erzielt werden, die nach Möglichkeit selektiv auf bestimmte Zielgewebe aufziehen sollen. Hier gab es Versuche (C. Haritoglou, A, Gandorfer, C. A. Gass et al., Am. J. Ophthalmol. 2002; 134: 836-841; C. Haritoglou, A. Gandorfer C. A. Gass et al. Am. J. Ophthalmol. 2003; 135, 328-337) mit Indocyaningrün (ICG, RN 3599-32-4; D. W. Heseltine, L. G. S. Brooker, US 2895955), das in der Humanmedizn bereits als optisches Kontrastmittel in der Kardiologie eingesetzt wird und sich dabei durch seinen raschen Abbau auszeichnet. Mit ICG konnte eine selektive Färbung der Lumina limitans interna (LLI-Membran) erreicht werden.

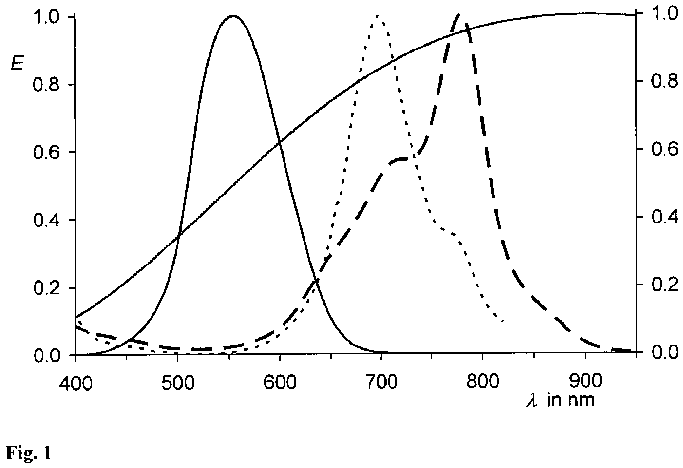

Indocyaningrün ist allerdings kein ideales Kontrastmittel, weil es trotz seiner Selektivität in Bezug auf das Zielgewebe schwierig zu kontrollierende toxische Wirkungen auf die Retina ausübt (C. Haritoglou, A. Gandorfer, A. Kampik, Invest. Ophthalmol. Vis Sci. 2003; 44, 316-323; C. Haritoglou, A. Gandorfer, M. Schaumberger et al., Invest. Ophthalmol. Vis Sci. 2003; 44, 2722-2729; C. Haritoglou, a. Gandorfer, C. A. Gass, A. Kampik, Am. J. Ophthalmol. 2004, 7, 345-348; C. Haritoglou, S. G. Priglinger, A. Gandorfer, U. Welge-Luessen, A. Kampik, Invest. Ophthalmol. Vis Sci. 2005, 46, 1468-1472), deren genaue Entstehung nicht vollständig aufgeklärt ist. Darüber hinaus liegt der Hauptteil der Lichtabsorption des Farbstoffs im nahen Infrarot (NIR)-Bereich, der für das Erkennen von Färbungen nutzlos ist, bzw. im langwellig sichtbaren Bereich, in dem die Lichtempfindlichkeit des menschlichen Auges bereits klein ist; siehe Figur 1. Eine Anpassung solcher Farbstoffe als Kontrastmittel an die speziellen Bedingungen bei Augenoperationen würde einen erheblichen Fortschritt bringen.

Die Aufgabe der vorliegenden Ereindung war es daher, ein toxikologisch unbedenkliches optisches Kontrastmittel für die Chirurgie des Auges, insbesondere am Augenhintergrund, zu entwickeln. Neben einer spektral atigepassten Färbung in Bezug auf die spektrale Augenempfindlichkeit war ein vorteilhaftes Färbeverhalten ein Ziel dieser Arbeit.

Für die Anwendung insbesondere in der Augenchirurgie sollten labile Farbstoffe entwickelt werden, damit nicht eine dauerhafte Färbung des Gewebes erfolgt Naphthocyaninfarbstoffe wurden dabei als interessante Ziel-Strukturen identifiziert Durch ihre Polyen-Teilstruktur wird ein biologischer Abbau erleichtern.

Durch die Verkürzung der zentralen Kette zwischen den Benzindol-Binheiten auf drei Methineinheiten konnte eine hypsochrome Verschiebung erreicht werden, so dass die optische Absorption der Farbstoffe an die Empfindlichkeitscharakteristik des menschlichen Auges angepasst, aber leicht bathochrom verschoben ist. Dies trägt der üblichen Verwendung von Wolfram-Halogenlampen bei Operationen Rechnung, die im längerwelligen Bereich stärker emittieren. Mit optionalen Sulfonsäure-Substituenten konnte dabei zusätzlich die für die Anwendung in der Augenheilkunde vorteilhafte Wasserlöslichkeit befördert werden.

Die vorliegende Erfindung stellt demgemäß einen Farbstoff der folgenden Formel (IIb) zur Anwendung als Kontrastmittel in einem Verfahren zur chirurgischen Behandlung oder in einem diagnostischen Verfahren bereit, 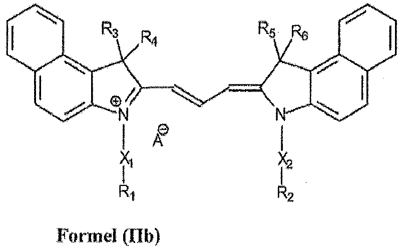 wobei
X₁ und X₂ unabhängig voneinander ausgewählt sind aus einer bis 12 CH₂-Einheiten, bei denen unabhängig voneinander eine oder mehrere ersetzt sein können durch eine Carbonylgruppe, ein Sauerstoffatom, ein Schwefelatom, eine *cis-* oder *trans*-CH=CH-Gruppe, bei der eine CH-Einheit auch durch ein Stickstoffatom ersetzt sein kann, eine acetylenische C≡C-Gruppe, einen divalenten Phenyl-, Pyridin-, oder Thiophenrest, einen divalenten Naphthalinrest, bei dem ein oder zwei CH-Gruppen durch Stickstoffatome ersetzt sein können, einen divalenten Anthracenrest, bei dem ein oder zwei CH-Gruppen durch Stickstoffatome ersetzt sein können; und wobei bis zu 12 einzelne Wasserstoffatome der CH₂-Einheiten jeweils unabhängig voneinander auch an gleichen G-Atomen ersetzt sein können durch die Halogene Fluor, Chlor, Brom oder Iod oder die Cyanogruppe oder durch eine lineare Alkylkette mit bis zu 18 C-Atomen, bei der eine bis 6 CH₂-Einheiten unabhängig voneinander ersetzt sein können durch eine Carbonylgruppe, ein Sauerstoffatom, ein Schwefelatom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenyl-, Pyridin , oder Thiophenrest, einen divalenten Naphthalinrest, bei dem ein oder zwei CH-Gruppen durch Stickstoffatome ersetzt sein können, oder einen divalenten Anthracenrest, bei dem ein oder zwei CH-Gruppen durch Stickstoffatome ersetzt sein können;
R₁ und R₂ unabhängig voneinander ausgewählt sind aus einer Carbonsäuregruppe (-COOH), einer Carbonsäureestergruppe, einer Sulfonsäuregruppe (-SO₃H) oder einem Halogenatom; R₃, R₄, R₅ und R₆ Methylgruppen sind und A⁻ ein optionales Anion darstellt, das die Formalladung am positiv geladenen Stickstoffatom ausgleichen kann.

Verbindungen mit einer derartigen Struktur sind beispielsweise offenbart in WO 2008/046775 A1 (als Farbstoff für eine Druckplatte), in WO 2005/000218 A2 (als Intermediat bei der Synthese von Biokonjugaten), in WO 97/13810 (als LED-Leuchtstoff oder Fluoreszenzmarker) oder in DE 19841985 A1 (als Arzneimittel). Hinweise auf eine Anwendung insbesondere in der Augenchirurgie oder in der Diaknostik am oder im Auge finden sich nicht.
**Figur 1** zeigt UV/Vis-Spektren. Durchgezogene schwarze Linie: Spektrale Augenempfindlichkeit. Graue Linie: Strahlungscharakteristik / einer Wolfram-Halogen-Lampe (3200 K). Gestrichelte Linie: Absorptionsspektrum von Indocyaningrün (ICG). Gepunktete Linie: Absorptionsspektrum des aggregierten ICG (H-Aggregat).

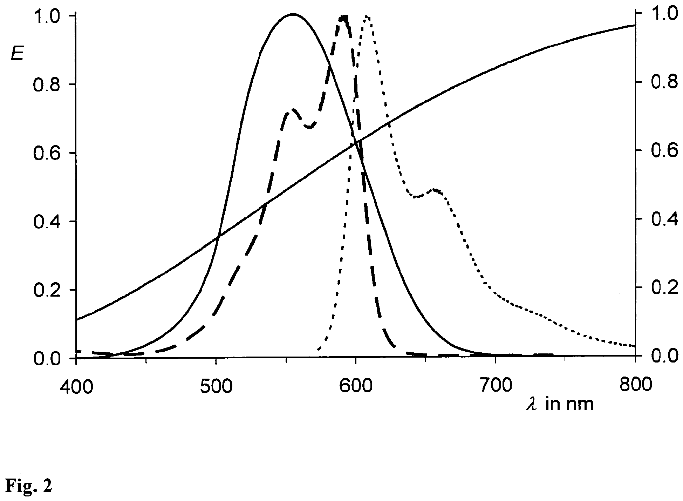

**Figur 2** zeigt UV/Vis-Spektren. Durchgezogene schwarze Linie: Spektrale Augenempfindlichkeit. Graue Linie: Strahlungscharakteristik einer Wolfram-Halogen-Lampe (3200 K). Gestrichelte Linie: Absorptionsspektrum von Verbindung **3i** in Ethanol. Gepunktete Linie: Fluoreszenzspektrum von **3i** in Ethanol.

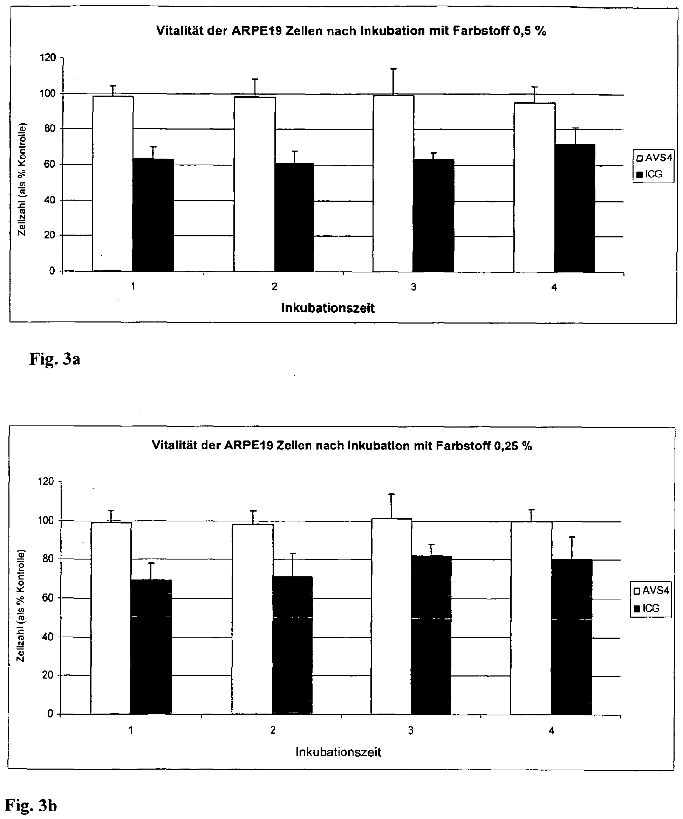

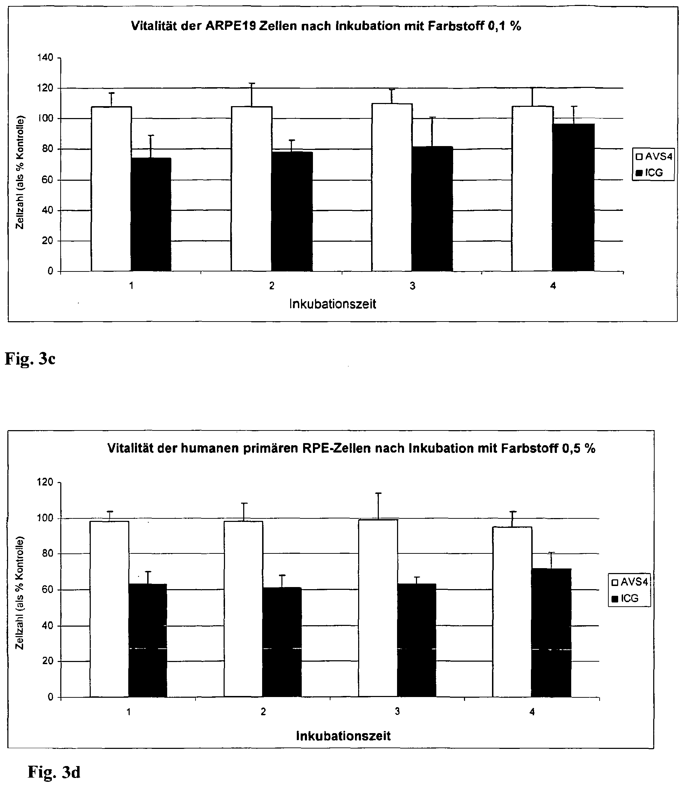

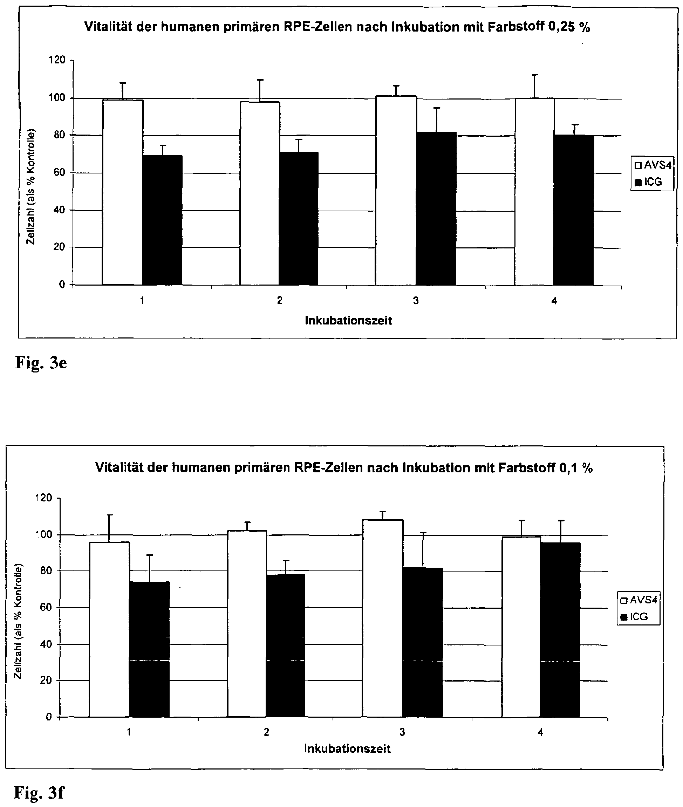

**Figur 3** zeigt die Zahl der ARPE-19 bzw. RPE-Zellen, gemessen mit dem beschriebenen colorimetrischen Verfahren (MTT) nach der Behandlung mit dem Farbstoff: ARPE-19: A: 0.5%, B: 0.25%, C: 0.1% des Farbstoffs 3t (in der dortigen Legende auch als AVS4 bezeichnet); RPE: D: 0.5%, E: 0.25%, F: 0.1% des Farbstoffs 3t.

Naphthocyaninfarbstoffe der Formel (IIb) wie auch der im Folgenden dargestellten bevorzugten Ausfiihrungsformen sind für Färbungen von Geweben für chirurgische Eingriffe und die Diagnostik besonders geeignet, weil ihre Lichtabsorption und ausgeprägte Fluoreszenz in einem für das menschliche Auge relevanten Spektralbereich liegen. Toxische Wirkungen wurden in der Substanzklasse nicht beobachtet. Erzielt Färbungen im Bereich der Basalmembran (im Tiermodell an porcinen Linsenkapseln) belegen eine Bindung im Bereich der Zielstruktur. Durch die Abstimmung von Seitenkette lassen sich die Eigenschaften der Substanz in weiten Grenzen variieren, so dass sie auf diesem Wege insbesondere an die Erfordernisse in der Augenchirurgie oder die Diagnostik am oder im Auge angepasst werden können.

Die UV/Vis-Absorptions- und Fluoreszenzspektren der erfindungsgemäßen Farbstoffe werden nur wenig durch ihre Substitution beeinflusst. Ein typisches Spektrum ist in Figur 2 dargestellt Die Absorption entspricht erstaunlich gut der Empfindlichkeitscharakteristik des menschlichen Auges und ist daher für den Chirurgen besonders interessant, weil sie sehr gut wahrgenommen werden kann. Der Schwerpunkt des Spektrums liegt etwas längerwellig als der Schwerpunkt der Augen- Empfindlichkeitscharakteristik. Dies erweist sich für die Praxis als ausgesprochen günstig, denn in der Augenchirurgie wird das Operationsfeld üblicherweise mit Halogenlampen beleuchtet, deren Strahlungscharakteristik ebenfalls in der Figur 2 dargestellt worden ist. Da hier der längerwellige Bereich etwas stärker betont. wird, wirkt sich die Lage des Spektrums optimal aus. Die blaue Färbung der Cyaninfarbstoffe hebt sich sehr gut von der Farbe des Gewebes ab. Eine Färbung durch das optische Kontrastmittel ist daher gut visuell ohne weitere Hilfsmittel zu erkennen.

Die Farbstoffe zeichnen sich durch ihre starke Lichtabsorption aus, die zu der erwähnten blauen Färbung führt Bei sehr kleinen Konzentrationen an Farbstoff wird die Färbung allerdings schlechter sichtbar. Empfindlicher kann demgegenüber die erstaunlich intensive Fluoreszenz von Substanzen festgestellt werden. Man erkennt in Figur 2, dass große Anteile des intensiv roten Flumeszenzlichts noch in einem für das menschliche Auge relevanten Spektralbereich liegen, so dass diese gut bei einer Färbung von Gewebestrukturen wahrgenommen werden. Das Fluoreszenzspektrum ist bei einem verhältnismäßig kleinen Stokes-Shift noch hinreichend innerhalb der Empfindlichkeitcharakteristik des menschlichen Auges, so dass eine starke rote Fluoreszenz wahrgenommen wird. Die Fluoreszenzquantenausbenten liegen bei 45% in Lösung, und die starke Fluoreszenz bleibt erhalten, wenn die Substanzen auf Oberflächen aufgezogen werden, so dass sich die Substanzen auch als Fluoreszenz-Kontrastmittel eignen.

Die Naphthocyaninfarbstoffe der Formel (IIb) wie auch der im Folgenden dargestellten bevorzugten Ausführungsformen sind in der Regel gut wesserlöslich, bei Bedarf kann ihre Löslichkeit durch die Substitution mit Sulfonsäuregruppen an den Ringstrukturen erhöht werden. Sie können daher in den für die Augenchirurgie üblicherweise verwendeten wässrigen Elektrolytlösungen problemlos eingesetzt werden. Die Toxizität der Farbstoffe ist ausgesprochen gering; es ließen sich bei Zellkulturen auch bei hohen Farbstoff-Konzentrationen keine toxischen Wirkungen nachweisen. Versuche mit biologischen Materialien haben ergeben, dass die Farbstoffen selektiv auf die menschliche LLI-Membran und die Augenkapsel von Schweineaugen aufziehen, die sich sehr gut als Modell für menschliche Augenstrukturen eignet Alle Farbstoffe färben nur das Gewebe der Augenkapsel, während der Glaskörner unbeeinflusst bleibt. Hiermit sind die Voraussetzungen für die Verwendung der Substanzen in der Augenchirurgie gegeben.

Demgemäß stellt die Erfindung die Farbstoffe der Formel (IIb) wie auch der im Folgenden dargestellten bevorzugten Ausführungsformen zur Anwendung oder Verwendung als Kontrastmittel in einem Verfahren zur chirurgischen Behandlung, insbesondere zur chirurgischen Behandlung des Auges, bereit Nach einem weiteren Aspekt stellt die Erfindung die Farbstoffe der Formel (IIb) wie auch der im Folgenden dargestellten bevorzugtes. Ausführungsformen zur Anwendung oder Verwendung als Kontrastmittel in einem Verfahren zur Diagnose, insbesondere zur Diagnostik am oder im Auge, bereit. Wie vorstehend beschrieben handelt es sich dabei in der Regel um den Einsatz als optisches Kontrastmittel. Bevorzugt finden die Farbstoffe Anwendung in der Humanmedizin/-chirurgie.

In Bezug auf die Anwendung als Kontrastmittel zur chirurgischen Behandlung des Auges ist das bevorzugte Einsatzgebiet die Chirurgie des hinteren Augenabschnitts, und insbesondere das Färben einer Basalmembran des Auges, wie der LLI (lamina limitans intema)-Membran. Die Membran kann so gezielt behandelt oder entfernt werden. Darüber hinaus eignen sich die Farbstoffe ausgezeichnet zum Färben der Linsenkapsel. Entsprechend gilt dies für die Anwendung als Kontrastmittel in einem diagnostischen Verfahren, hier ist das bevorzugte Einsatzgebiet die Diagnostik des hinteren Augenabschnitts, und insbesondere das Färben einer Basalmembran des Auges, wie der LLI (lamina limitans intema)-Membnn. Darüber hinaus eignen sich die Farbstoffe ausgezeichnet zum Färben der Linsenkapsel.

Offenbart wird daher auch ein Verfahren zur chirurgischen Behandlung des Auges,umfassend den Schritt des Applizierens eines Farbstoffs der Formel (IIb) oder der im Folgenden dargestellten bevorzugten Ausführungsformen als Kontrastmittel in das Auge, insbesondere des Applizierens zum Färben der LLI-Membran oder der Linsenkapsel.

Offenbart wird darüber hinaus ein Verfahren zur Diagnostik am oder im Auge, umfassend den Schritt des Applizierens eines Farbstoffs der Formel (IIb) oder der im Folgenden dargestellten bevorzugten Ausführungsformen als Kontrastmittel in das Auge, insbesondere des Applizierens zum Färben der LLI-Membran oder der Linsenkapsel. Bei dem Farbstoff handelt es sich um eine Verbindung der **Formel** (IIb).

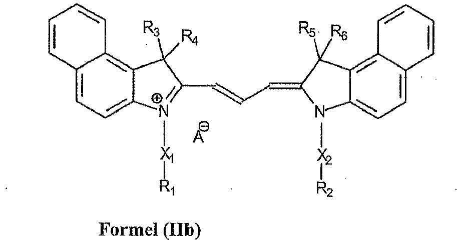

Besonders bevorzugt als Farbstoff der Formel (IIb) sind Verbindungen der folgenden Formeln (III), (IV) oder (V).

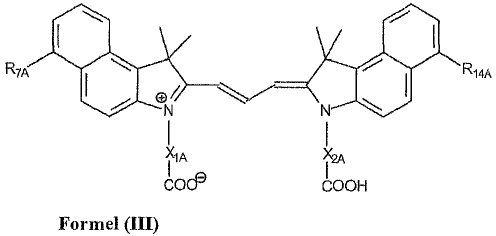

In Formel (III) sind X_{1A} und X_{2A} unabhängig voneinander ausgewählt aus einer bis 12 CH₂-Einheiten, bei denen unabhängig voneinander eine bis drei ersetzt sein können durch ein Sauerstoffatom, oder ein Schwefelatom, und/oder eine ersetzt sein kann durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Iod oder die Cyanogruppe oder durch eine lineare Alkylkette mit bis zu 6 C-Atomen; und
R_{7A} und R_{14A} Wasserstoff sind

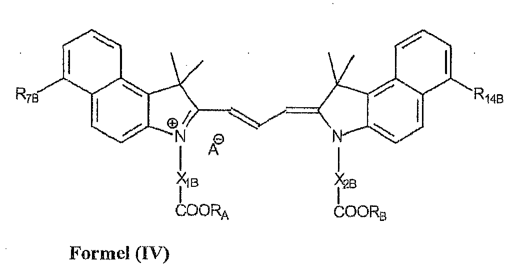

In Formel (IV) sind X_{1B} und X_{2B} unabhängig voneinander ausgewählt aus einer bis 12 CH₂-Einheiten, bei denen unabhängig voneinander eine bis drei ersetzt sein können durch ein Sauerstoffatom, oder ein Schwefelatom, und/oder eine ersetzt sein kann durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Iod oder die Cyanogruppe oder durch eine lineare Alkylkette mit bis zu 6 C-Atomen;
R_{A} und R_{B} sind unabhängig voneinander ausgewählt aus C₁-C₁₂ Alkyl und einer der Reste R_{A} und R_{B} kann auch Wasserstoff sein;
R_{7B} und R_{14B} Wasserstoff sind und
A⁻ stellt ein Anion dar, das die Formalladung am positiv geladenen Stickstoffatom ausgleicht.

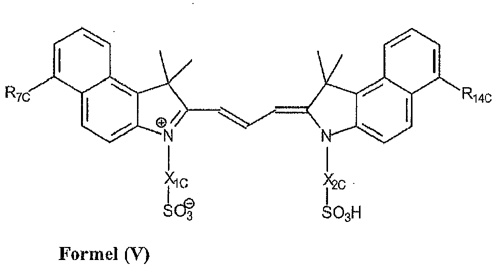

In Formel (V) sind X_{1C} und X_{2C} unabhängig voneinander ausgewählt aus einer bis 12 CH₂-Einheiten, bei denen unabhängig voneinander eine bis drei ersetzt sein können durch ein Sauerstoffatom, oder ein Schwefelatom, und/oder eine ersetzt sein kam durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Iod oder die Cyanogruppe oder durch eine lineare Alkylkette mit bis zu 6 C-Atomen; und
R_{7C} und R_{14C} Wasserstoff sind.

In Bezug auf die Verbindungen der Formel (IIb) gelten darüber hinaus folgende bevorzugte Definitionem.

X₁ und X₂ sind bevorzugt unabhängig voneinander ausgewählt aus 1 bis 12 CH₂-Einheiten, bei denen unabhängig voneinander eine bis drei ersetzt sein können durch ein Sauerstoffatom oder ein Schwefelatom, und/oder eine ersetzt sein kann durch einen divalenten Phenylrest; und wobei bis 4 einzelne Wasserstoffatome der CH₂-Einheten jeweils unabhängig voneinander auch an gleichen C-Atomen ersetzt sein können durch die Halogene Fluor, Chlor, Brom oder Iod oder die Cyanogruppe oder durch eine lineare Alkylkette mit bis zu 6 C-Atomen. Insbesondere sind X₁ und X₂ bevorzugt unabhängig voneinander ausgewählt aus 1 bis 12 CH₂-Einheiten, bei denen unabhängig voneinander eine bis drei ersetzt sein können durch ein Sauerstoffatom und wobei bis zu 4 einzelne Wasserstoffatome der CH₂-Einheiten jeweils unabhängig voneinander auch an gleichen C-Atomen ersetzt sein können durch die Halogene Fluor, Chlor, oder Brom oder durch eine lineare Alkylkette mit bis zu 6 C-Atomen. Ganz besonders bevorzugt sind X₁ und X₂ unabhängig voneinander ausgewählt aus 1 bis 12 CH₂-Einheiten.

R₁ und R₂ sind bevorzugt unabhängig voneinander ausgewählt aus einer Carbonsäuregruppe (-COOH), einer Carbonsäureestergruppe (-COOR), und einer Sulfonsäuregruppe (-SO₃H). Für Verbindungen der Formel (IIb) ist R bevorzugt ein linearer Alkylrest mit 1 bis 37 C-Atomen. In einer alternativen, stärker bevorzugten Ausführungsform ist R sowohl für die Verbindungen der Formel (I) wie auch für die Verbindungen der Formeln (IIa) und (IIb) ein Alkylrest mit 1 bis 12, insbesondere 1 bis 6 C-Atomen, ganz besonders bevorzugt sind Methyl- und Ethylester. Es sollte klar sein, dass die Säuregruppen bei Vorliegen der Verbindung der Formel (I), (IIa) und (IIb) in Lösungsmitteln, insbesondere in Wasser, auch in deprotonierter Form vorliegen können. In diesem Fall wird ihre negative Formalladung in der Regel durch ein Kation ausgeglichen. Dabei kann es sich um das Stickstoffatom mit kationischer Formalladung der Formel (I), (IIa) und (IIb) handeln, oder um ein externes (d.h. nicht in der Verbindung kovalent gebundenes) Kation. Beispiele für ein solches Kation sind ein Alkali- oder Erdalkalimetallkation, oder ein Ammoniumkation.

R₃, R₄, R₅ und R₆ sind bevorzugt unabhängig voneinander ausgewählt aus C₁-C₆ Alkyl, und sind insbesondere bevorzugt Methyl.

A⁻ stellt ein optionales Anion dar, das die Formalladung am positiv geladenen Stickstoffatom ausgleichen kann. Diesbezüglich wurde bereits darauf hin gewiesen, dass beispielsweise die Reste R₁ bzw. R₂ eine Carbonsäuregruppe oder eine Sulfonsäuregruppe darstellen können, die in deprotanierter Form eine negative Ladung tragen. Insofern muss die positive Formalladung nicht notwendigerweise durch ein externes (d.h. nicht in der Verbindung kovalent gebundenes) Anion A⁻ ausgeglichen werden. Statt dessen kann auch ein inneres Salz gebildet werden mit einer kationischen und einer anionischen Ladung in der Verbindung der Formel (I). Liegt ein externes Anion vor, so kann es sich beispielsweise um ein einwertiges Anion, bevorzugt ein Anion von Mineralsäuren wie Fluorid, Chlorid, Bromid, Iodid, Hydrogensulfat, Dihydrogenphosphat, Hydrogencarbamat oder Nitrat handeln. A⁻ kann auch das halbe Äquivalent eines Dianions wie Sulfat, Carbonat oder Hydrogenphosphat oder auch ein Drittel eines Trisanions sein wie Phosphat. A⁻ kann aber auch das Anion einer Sulfonsäure wie Toluolsulfonsäure, Benzolsulfonsäure oder Methansulfonsäure sein oder auch das Anion einer Carbonsäure wie Essigsäure.

Salze der Verbindung der Formel (IIb) können, wie vorstehend beschrieben, durch die Verbindung, mit positiver Formalladung am Stickstoffatom und einem externen Anion A⁻ gebildet werden. Sie können auch durch eine anionische deprotonierte Säuregruppe zusammen mit einem externen Kation gebildet werden. Alle derartigen Salze sind im Umfang der Erfindung eingeschlossen.

Bevorzugt ist darüber hinaus aus Gründen der einfachen Synthese, dass es sich bei den Verbindungen der Formel (IIb) um symmetrische Verbindungen handelt, bei denen die Gruppen X₁ und X₂, R₁ und R₂, R₃ bis R₆, )) jeweils identisch sind.

In Bezug auf die Verbindungen der Formel (III) gelten folgende bevorzugte Definitionen.

X_{1A} und X_{2A} sind bevorzugt unabhängig voneinander ausgewählt sind aus 1 bis 12 CH₂-Einheiten, bei denen unabhängig voneinander eine bis drei ersetzt sein können durch ein Sauerstoffatom und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder durch eine lineare Alkylkette mit bis zu 6 C-Atomen. Insbesondere sind X_{1A} und X_{2A} bevorzugt unabhängig voneinander ausgewählt sind aus 1 bis 12 CH₂-Einheiten.

Stellen R_{7A} und R_{14A} Wasserstoff dar, so ist es insbesondere bevorzugt dass X_{1A} und X_{2A} unabhängig voneinander ausgewählt sind aus 2 bis 9 CH₂-Einheiten, besonders bevorzugt 2 oder 5 bis 9 Einheiten.

In Bezug auf die Verbindungen der Formel (IV) gelten folgende bevorzugte Definitionen.

X_{1B} und X_{2B} sind bevorzugt unabhängig voneinander ausgewählt sind aus 1 bis 12 CH₂-Einheiten, bei denen unabhängig voneinander eine bis drei ersetzt sein können durch ein Sauerstoffatom und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder durch eine lineare Alkylkette mit bis zu 6 C-Atomen. Insbesondere sind X_{1B} und X_{2B} bevorzugt unabhängig voneinander ausgewählt sind aus 1 bis 12 CH₂-Einheiten.

R_{A} und R_{B} sind bevorzugt unabhängig voneinander ausgewählt sind aus C₁-C₆ Alkyl, insbesondere aus Methyl und Ethyl. Dabei sind besonders bevorzugt Kombinationen in denen R_{A} und R_{B} Methyl sind und X_{1B} und X_{1C} ausgewählt sind aus 1 bis 10 CH₂-Einheiten, und in denen R_{A} und R_{B} Ethyl sind und X_{1B} und X_{1C} ausgewählt sind aus 1 bis 5 CH₂-Einheiten. R_{7B} und R_{14B} sind Wasserstoff.

In Bezug auf die Verbindungen der Formel (V) gelten folgende bevorzugte Definitionem.

X_{1C} und X_{2C} sind bevorzugt unabhängig voneinander ausgewählt aus 1 bis 12 CH₂-Einheiten, bei denen unabhängig voneinander eine bis drei ersetzt sein können durch ein Sauerstoffatom und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder durch eine lineare Alkylkette mit bis zu 6 C-Atomen. Insbesondere sind X_{1C} und X_{2C} bevorzugt unabhängig voneinander ausgewählt aus 1 bis 12 CH₂-Einheiten, ganz besonders bevorzugt aus 1 bis 5 CH₂-Einheiten.

Bevorzugt ist darüber hinaus auch für die Verbindungen der Formel (III), (IV) und (V) aus Gründen der einfachen Synthese, dass es sich um symmetrische Verbindungen handelt, bei denen die Gruppen R_{7A} und R_{14A}, R_{7B} und R_{14B} bzw. R_{7C} und R_{14C}, X_{1A} und X_{2A}, X_{1B} und X_{2B} bzw. X_{1C} und X_{2C} sowie R_{A} und R_{B} jeweils identisch sind.

Weiter gilt auch für die Verbindungen der Formeln (III), (IV) und (V), dass Salze durch die Verbindung mit positiver Formalladung am Stickstoffatom und einem externen Anion A⁻ gebildet werden können. Sie können auch durch eine anionische deprotonierte Säuregruppe zusammen mit einem externen Kation gebildet werden. Derartige Salze sind im Umfang der Erfindung eingeschlossen.

Die Stabilitäten der Substanzen werden im Wesentlichen von den funktionellen Gruppen bestimmt, wie aus der folgenden Tab. 1 zu ersehen ist; eine Zersetzung der festen Farbstoffe führt zu einer Rotfärbung der Feststoffe und der Lösungen. Besonders empfindlich sind Carbonsäureester und hier insbesondere Methylester und außerdem die Bromide. Farbstoffe ohne Sulfonsäuregruppen an den aromatischen Kernen sind demgegenüber im Allgemeinen stabiler, so dass auch nach mehreren Jahren Lagerung keine Zersetzung beobachtet wird.

**Tabelle 1. Stabilitätsvergleich der festen Cyaninfarbstoffe**

| **Farbstoff** | **Stabilität** |
|---|---|
| **3t** | 3 Jahre keine Zersetzung |
| **3u** | 4 Monate keine Zersetzung |
| **3v** | 4 Monate keine Zersetzung |
| **3w** | Zersetzung nach wenigen Tagen |
| **3x** | 4 Monate keine Zersetzung |
| **3y** | 4 Monate keine Zersetzung |
| **6a** | 4 Monate keine Zersetzung |
| **6b** | Zersetzung nach wenigen Tagen |
| **6c** | 3 Monate keine Zersetzung |
| **6d** | Zersetzung nach 1 Monat |
| **6e** | Zersetzung nach 2 Monaten |
| **6f** | Zersetzung nach 2 Monaten |
| **6g** | 3 Monate keine Zersetzung |
| **6h** | Zersetzung nach wenigen Tagen |
| **6i** | Zersetzung nach 2 Tagen |

Für die Synthese beispielhafter Verbindungen der Formel (I) kann in Anlehnung an Y. Ye, S. Bloch, S. Achilefu, J. Am. Chem. Soc. 2004, 126, 7740-7741 von Trimethylbenzindol ausgegangen werden, das zu den Immoniumsalzen **2** (Schema 1) substituiert werden kann (C. D. Geddes, Dyes and Pigm. 2001, 50, 151-155). Auf diesem Weg lassen sich diverse Seitenketten mit unterschiedlich langen Alkyl-Spacergruppen und polaren Endgruppen, wie Carboxylgruppen und Carbonsäueestergruppen, auch Alkinreste, einführen. Durch solche Strukturvariationen ist es möglich, geeignete Strukturen bereit zu stellen, die mit mehreren polaren Gruppen möglichst selektiv an Gewebe binden. Die Immoniumsalze **2** können dann mit Orthoameisensäureester in Anlehnung an N. Shigetou, J. Miyazaki, M. Hirai, US Pat. 5922618 A (2.4.1997); Chem. Abstr. 2001, 135, 300684 zu den Cyaninfarbstoffen **3** umgesetzt werden. Die Reindarstellung der Farbstoffe, die für deren medizinische Anwendung essentiell ist, gestaltete sich wegen des überwiegend polaren Charakters der Substanzen als ausgesprochen schwierig. Eine Vorreinigung des Materials gelang dann durch Ausfällen aus polarer Lösung mit stärker lipophilen Solventien wie z.B. Ether. Überraschend gute Ergebnisse wurden bei der Verwendung von Reversed-Phase-Chromatographie erzielt. Hier eignete sich als stationäre Phase insbesondere Kieselgel mit unpolaren Seitenketten, wie RP18, mit dem säulenchromatographisch die Reindarstellung der Substanzen in präparativem Maßstab sehr gute Ergebnisse zeigte. Bevorzugte Laufmittel sind stark polar, wie wasserhaltige kurzkettige Alkohole (z.B. Methanol, Ethanol, Propanol, insbesondere Methanol), ggf. unter Säurezusatz. Beispielhafte Laufmittelgemische enthalten Methanol, Wasser und Eisessig oder Methanol, Wasser und 2N HCl. Bevorzugt ist dabei weiterhin ein Volumenverhältnis Methanol/Wasser/Säure von 1:10:0,4.

Für die Darstellung beispielhafter Verbindungen mit Sulfonsäuregruppen als Gruppe X in Schema 1 kann ebenfalls von Trimethylbenzindol **1** ausgegangen werden, das am Stickstoffatom alkyliert werden kann (**2** in Schema 1), um Funktionalitäten einzuführen. Diese Farbstoff-Vorstufe kann hier direkt mit Sulfonsäuregruppen (X in **2**) versehen werden. Zur Alkylierung mit *n* = 4 eignet sich Butansulton. Für andere Alkyl-Kettenlängen (n = 2, 5 und 10) sind die entsprechenden Bromsulfonsäuren eingesetzt worden. Als Alternative kann zunächst mit einem Überschuß an α,ω-Dibromalkanen zu den entsprechenden Bromiden alkyliert (**2**, X = Br, *n* = 5, 10) und das Brom mit Sulfit zu den Sulfonsäuren **3** substituiert (X = SO₃⁻, *n* = 5, 10) werden.

Sulfonsäuregruppen als Substituenten am aromatischen Ringsystem der Farbstoffe zur erfindungsgemäßen Anwendung können durch Sulfonierung eingeführt werden, indem eine Ausgangssubstanz mit einem Benzindolgerüst, wie z.B. das Trimethylbenzindol **1** in Nitrobenzol mit Oleum zur Sulfonsäure, z.B. Sulfonsäure **4,** umgesetzt wird. Diese einstufige Umsetzung stellte eine Verbesserung gegenüber der vielstufigen Literatursynthese (L. Della-Caiana, A. Grignani, M. Cassullo, G. Caputo, PCT Int. Appl. WO 97/13810) analog zur Fischer-Indolsynthese dar. Das Nitrobenzol kann im Anschluss an die Sulfonierung effizient durch Wasserdampfdestillation entfernt werden. Die Verbindungen **4** können analog zu **1** alkyliert und als Carbonylderivate die Carbonsäure (**z.B. 5c**) und die Ethylester (z.B. **5e, 5g**) hergestellt werden. Auf diesem Weg konnten auch zwei Sulfonsäuregruppen in das Benzindol eingeführt werden, indem die Alkylkette mit einem endständigen Sulfonsäurerest versehen wird (z.B. **5a**).

Die Vorstufen **2, 4** bzw. **5** können mit Ameisensäureorthoester zu den Cyaninfarbstoffen **3** bzw. **6** kondensiert werden. Die Alkylierung und Kondensation von **4** zu **6** gelingt auch in einem Schritt. Auch für die Reinigung der Farbstoffe **6** (Referenzbeispiel) hat sich die Reversed-Phase-Chromatographie, wie vorstehend beschrieben, bewährt.

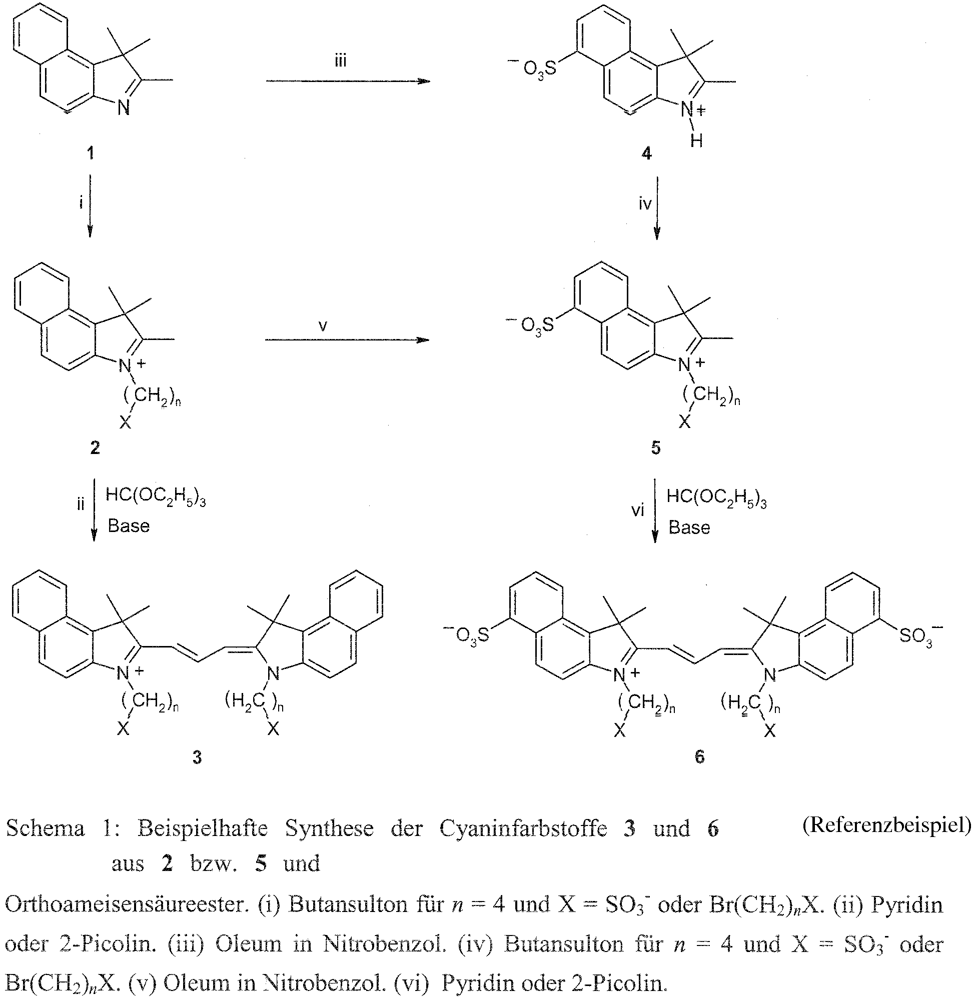

Die Farbstoffe werden zum erfindungsgemäßen Einsatz vorteilhafterweise als Elektrolytlösung, in der Regel als wässrige isotonischen Elektrolytlösung formuliert. Bei Bedarf können dieser Elektrolytlösung bekannte Additive zugegeben werden, wie z.B. Konservierungsmittel oder Mittel zum Einstellen der Viskosität und/oder der Osmolarität. Die Applikation kann durch Eintropfen der Lösung in das Auge erfolgen. Die Konzentration des Farbstoffs in der Lösung kann dabei nach Bedarf angepasst werden, typischerweise werden Konzentrationen in einem Bereich von 0,0025 % (Angaben in Gewichtsprozent des Farbstoffs bezogen auf das Gewicht des Lösungsmittels) bis 2 %, bevorzugt 0,025 % bis 1 % eingesetzt. In den folgenden Punkten 1 und 2 werden ergänzend Farbstoffe offenbart, die zur erfindungsgemäßen Verwendung einsatzbar sind.

Demgemäß stellt die Erfindung auch die Farbstoffe der nachstehende genannten Formeln 5 und 6 (Referenzbeispiel) zur Anwendung oder Verwendung als Kontrastmittel in einem Verfahren zur chirurgischen Behandlung, insbesondere zur chirurgischen Behandlung des Auges, bereit. Wie vorstehend beschrieben handelt es sich dabei in der Regel um den Einsatz als optisches Kontrastmittel. Bevorzugt finden die Farbstoffe Anwendung in der Humanmedizin/-chirurgie.
1. Carbocyaninfarbstoffe der allgemeinen Formel **5**, 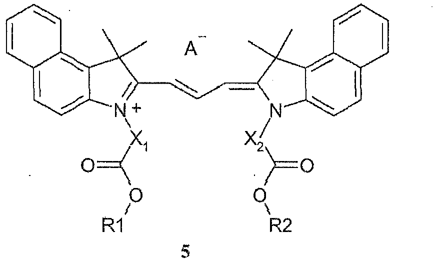 in denen X₁ und X₂ unabhängig voneinander sein können und eine bis 12 CH₂-Enheiten bedeuten, bei denen unabhängig voneinander eine oder mehrere ersetzt sein können durch jeweils Carbonylgruppen, Sauerstoffatome, Schwefelatome, *cis-* oder *trans*-CH=CH-Gruppen, bei der eine CH-Einheit auch durch ein Stickstoffatom ersetzt sein kann, acetylenische C≡C-Gruppen, 1,2-, 1,3- oder 1,4-substituierten Phenylreste, 2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei CH-Gruppen durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei CH-Gruppen durch Stickstoffatome ersetzt sein können. Bis zu 12 einzelne Wasserstoffatome der CH₂-Gruppen können jeweils unabhängig voneinander auch an gleichen C-Atomen ersetzt sein durch die Halogene Fluor, Chlor, Brom oder Iod oder die Cyanogruppe oder eine lineare Alkylkette mit bis zu 18 C-Atomen, bei der eine bis 6 CH₂-Einheiten unabhängig voneinander ersetzt sein können durch Carbonylgruppen, Sauerstoffatome, Schwefelatome, Selenatome, Telluratome, *cis-* oder *trans*-CH=CH-Gruppen, bei denen eine CH-Einheit auch durch ein Stickstoffatom ersetzt sein kann, acetylenische C≡C-Gruppen, 1,2-, 1,3- oder 1,4-substituierte Phenylreste, 2,3-, 2,4-, 2,5-, 2,6-, 3,4- oder 3,5-disubstituierte Pyridinreste 2,3-, 2,4-, 2,5- oder 3,4-disubstituierter Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei Kohlenstoffatome durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei Kohlenstoffatome durch Stickstoffatome ersetzt sein können. Bis zu 12 einzelne Wasserstoffatome der CH₂-Gruppen der Alkylreste können jeweils unabhängig voneinander auch an gleichen C-Atomen ersetzt sein durch die Halogene Fluor, Chlor, Brom oder Iod oder Cyanogruppe oder lineare Alkylketten mit bis zu 18 C-Atomen, bei denen eine bis 6 CH₂-Einheiten unabhängig voneinander ersetzt sein können durch Carbonylgruppen, Sauerstoffatome, Schwefelatome, *cis*- oder *trans*-CH=CH-Gruppen, bei der eine CH-Einheit auch durch ein Stickstoffatom ersetzt sein kann, acetylenische C≡C-Gruppen 1,2-, 1,3- oder 1,4-substituierte Phenylreste, 2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei Kohlenstoffatome durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei Kohlenstoffatome durch Stickstoffatome ersetzt sein können. Statt Substituenten zu tragen können die freien Valenzen der Methingruppen bzw. der quartären C-Atome paarweise verknüpft werden, so dass Ringe entstehen, wie z.B. Cyclohexanringe. Die Reste R¹ bis R² können außerdem unabhängig voneinander die Halogenatome F, Cl, Br oder I bedeuten.

Die Reste R¹ bis R² können gleich oder verschieden voneinander sein und können unabhängig voneinander Wasserstoff oder lineare Alkylreste mit mindestens einem und höchstens 37 C-Atome bedeuten, bei denen eine bis 10 CH₂-Enheiten unabhängig voneinander ersetzt sein können durch jeweils Carbonylgruppen, Sauerstoffatome, Schwefelatome, Selenatome, Telluratome, *cis-* oder *trans*-CH=CH-Gruppen, bei der eine CH-Einheit auch durch ein Stickstoffatom ersetzt sein kann, acetylenische C≡C-Gruppen 1,2-, 1,3- oder 1,4-substituierten Phenylreste, 2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei CH-Gruppen durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei CH-Gruppen durch Stickstoffatome ersetzt sein können. Bis zu 12 einzelne Wasserstoffatome der CH₂-Gruppen können jeweils unabhängig voneinander auch an gleichen C-Atomen ersetzt sein durch die Halogene Fluor, Chlor, Brom oder Iod oder die Cyanogruppe oder eine lineare Alkylkette mit bis zu 18 C-Atomen, bei der eine bis 6 CH₂-Einheiten unabhängig voneinander ersetzt sein können durch Carbonylgruppen, Sauerstoffatome, Schwefelatome, Selenatome, Telluratome, *cis-* oder *trans*-CH=CH-Gruppen, bei denen eine CH-Einheit auch durch ein Stickstoffatom ersetzt sein kann, acetylenische C≡C-Gruppen, 1,2-, 1,3- oder 1,4-substituierte Phenylreste, 2,3-, 2,4-, 2,5-, 2,6-, 3,4- oder 3,5-disubstituierte Pyridinreste, 2,3-, 2,4-, 2,5- oder 3,4-disubstituierter Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei Kohlenstoffatome durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei Kohlenstoffatome durch Stickstoffatome ersetzt sein können. Bis zu 12 einzelne Wasserstoffatome der CH₂-Gruppen der Alkylreste können jeweils unabhängig voneinander auch an gleichen C-Atomen ersetzt sein durch die Halogene Fluor, Chlor, Brom oder Iod oder Cyanogruppen oder lineare Alkylkette mit bis zu 18 C-Atomen, bei denen eine bis 6 CH₂-Einheiten unabhängig voneinander ersetzt sein können durch Carbonylgruppen, Sauerstoffatome, Schwefelatome, Selenatome, Telluratome, *cis-* oder *trans-*CH=CH-Gruppen, bei der eine CH-Einheit auch durch ein Stickstoffatom ersetzt sein kann, acetylenische C≡C-Gruppen 1,2-, 1,3- oder 1,4-substituierte Phenylreste, 2,3-, 2,4-, 2,5-, 2,6-, 3,4- oder 3,5-disubstituierte Pyridinreste, 2,3-, 2,4-, 2,5- oder 3,4-disubstituierte Thiophenreste, 1,2-, 1,3-, 1,4-, 1,5-, 1,6-, 1,7-, 1,8-, 2,3-, 2,6- oder 2,7-disubstituierte Naphthalinreste, bei denen ein oder zwei Kohlenstoffatome durch Stickstoffatome ersetzt sein können, 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-disubstituierte Anthracenreste, bei denen ein oder zwei Kohlenstoffatome durch Stickstoffatome ersetzt sein können. Statt Substituenten zu tragen können die freien Valenzen der Methingruppen bzw. der quartären C-Atome paarweise verknüpft werden, so dass Ringe entstehen, wie z.B. Cyclohexanringe. Die Reste R¹ bis R² können außerdem unabhängig voneinander die Halogenatome F, Cl, Br oder I bedeuten.

A⁻ bedeutet ein einwertiges Anion, bevorzugt ein Anion von Mineralsäuren wie Fluorid, Chlorid, Bromid, Iodid, Hydrogensulfat, Dihydrogenphosphat, Hydrogencarbonat oder Nitrat. A⁻ kann auch das halbe Äquivalent eines Dianions wie Sulfat, Carbonat oder Hydrogenphosphat oder auch ein Drittel eines Trisanions sein wie Phosphat. A⁻ kann aber auch das Anion einer Sulfonsäure wie Toluolsulfonsäure, Benzolsulfonsäure oder Methansulfonsäure sein oder auch das Anion einer Carbonsäure wie Essigsäure.
2. Cyaninfarbstoffe der allgemeinen Formel : 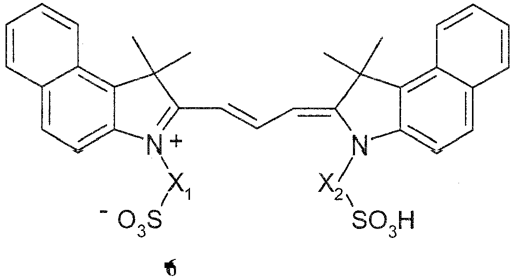 in denen X₁ und X₂ die in 1 genannte Bedeutung haben.
   Wesentliche Aspekte der vorliegenden Erfindung sind darüber hinaus die folgenden:
3. Verfahren dadurch **gekennzeichnet,** dass die Cyaninfarbstoffen nach 1 und 2 über Reversed-Phase-Chromatographie gereingt werden. Die bevorzugte stationäre Phase ist RP18, bevorzugte Laufmittel enthalten Wasser, Methanol und Eisessig bzw. wässrige 2 N HCl und eine bevorzugte Zusammensetzung ist 1:10:0.4.
4. **Verwendung** der Substanzen nach 1 bis 2 als optisches Kontrastmittel in der Medizin, bevorzugt in der Humanmedizin.
5. **Verwendung** der Substanzen nach 1 bis 2 als optisches Kontrastmittel in der Augenheilkunde und hier speziell in der Augenchirurgie. Bevozugte Anwendungen liegen in der Chirurgie des hinteren Augenabschnitts und hier speziell zu Entfernung der LLI-Membran und zur Anfärbung und Behandlung der Linsenkapsel.
6. **Verwendung** der Substanzen nach 1 bis 2 als optisches Fluoreszenz-Kontrastmittel in der Medizin, hier speziell in der Humanmedizin, insbesondere in der Augenheilkunde und hier speziell in der Augenchirurgie. Bevozugte Anwendungen liegen in der Chirurgie des hinteren Augenabschnitts und hier speziell zu Entfernung der LLI-Membran und zur Anfärbung und Behandlung der Linsenkapsel.

### Beispiele

### Allgemeines:

IR-Spektren: Perkin Elmer Spektrum BX II mittels Diamantstempelzelle; UVNis-Spektren: Varian Cary 5000 und Bruins Omega 20; Fluoreszenzspektren: Varian Eclispe; NMR-Spektroskopie: Varian Vnmrs 600 (600 MHz, 400 MHz, 200 MHz); Massenspektrometrie: Thermo Finnigan LQT FT.

### Synthese von Vorstufen der Cyaninfarbstoffe

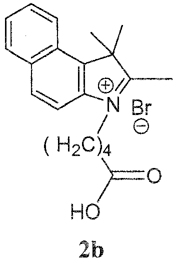

**3-(4-Carboxybutyt)-1,1,2-trimethyl-1*H*-benz[*e*]indoleniumbromid (2b):** 1,1,2-Trimethylbenz[*e*]indol (360 mg, 1.65 mmol) und 5-Bromvaleriansäure (300 mg, 1.65 mmol) wurden 1 h unter Rückfluss erhitzt (Bad 145°C), abkühlen lassen, 3 h mit Diethylether gerührt (50.0 mL), abfiltriert und mit Dichlormethan gewaschen. Ausb. 243 mg (38%) farbloser Feststoff. Schmp. 187°C. IR (ATR): *ṽ* = 2984 (w), 2913 (w), 1713 (s), 1581 (m), 1524 (m), 1474 (m), 1455 (m), 1397 (m), 1368 (w), 1258 (w), 1236 (w), 1208 (m), 1171 (s), 1157 (s), 1084 (w), 896 (w), 869 (w), 811 (s), 791 (m), 762 (m), 747 (w), 692 (w), 641 cm⁻¹ (w). ¹H-NMR (200 MHz, DMSO-*d*₆): *δ* = 8.49-8.31 (m, 1 H, *H*ₐᵣₒₘₐₜ), 8.30-8.13 (m, 2 H, *H*ₐᵣₒₘₐₜ), 8.09-7.96 (m, 1 H, *H*ₐᵣₒₘₐₜ), 7.85-7.70 (m, 1 H, *H*ₐᵣₒₘₐₜ), 7.68-7.50 (m, 1 H, *H*ₐᵣₒₘₐₜ), 4.60 (t, 2 H, NC*H*₂, ³*J* = 7.0 Hz), 2.94 (s, 3 H, C*H*₃), 2.32 (t, 2 H, C*H*₂, ³*J* = 7.2 Hz), 1.99-1.84 (m, 2 H, C*H*₂), 1.76 (s, 6 H, 2 x C*H*₃), 1.73-1.63 ppm (m, 2 H, C*H*₂). HRMS (ESI) (C₂₀H₂₄NO₂⁺): Ber. 310.1802, Gef. 310.1801, *Δ* = -0.1 mmu.

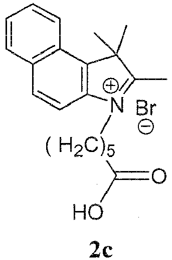

**3-(5-Carboxypentyl)-1,1,2-trimethyl-1*H*-benz[*e*]indoleniumbromid (2c):** 1,1,2-Trimethylbenz[e]indol (500 mg, 2.30 mmol) und 6-Bromhexansäure (1.15 g, 6.00 mmol) wurden in DMPU (10.0 mL) gelöst, 2 d unter Schutzgas auf 120°C erhitzt, abfiltriert und mit Diethylether und Dichlormethan gewaschen. Ausb. 350 mg (37%) leicht bläulicher Feststoff. ¹H-NMR (400 MHz, CD₃OD) *δ* = 8.33 (d, 1 H, *H*ₐᵣₒₘₐₜ, ³*J* = 8.5 Hz), 8.25 (d, 1 H, *H*ₐᵣₒₘₐₜ, ³*J* = 9.1 Hz), 8.17 (d, 1 H, *H*ₐᵣₒₘₐₜ, ³*J* = 8.3 Hz), 8.01 (d, 1 H, *H*ₐᵣₒₘₐₜ, ³*J* = 9.0 Hz), 7.81 (ddd, 1 H, *H*ₐᵣₒₘₐₜ, ⁴*J* = 1.3 Hz, ³*J* = 6.9 Hz, ³*J* = 8.4 Hz), 7.73 (ddd, 1 H, *H*ₐᵣₒₘₐₜ, ⁴*J* = 1.1 Hz, ³*J* = 7.0 Hz, ³*J* = 8.1 Hz), 4.70-4.60 (m, 2 H, NC*H*₂), 2.89 (s, 3 H, CH₃), 2.36 (t, 2 H, C*H₂,* ³*J* = 7.0 Hz), 2.18-1.97 (m, 2 H, C*H*₂), 1.85 (s, 6 H, 2 x C*H*₃), 1.79-1.68 (m, 2 H, C*H*₂), 1.68-1.53 ppm (m, 2 H, C*H*₂). ¹³C-NMR (100 MHz, CD₃OD): *δ* = 196.1, 175.7, 174.2, 138.3, 137.3, 131.0, 129.6, 128.3, 127.7, 127.3, 123.0, 112.3, 55.9, 32.8, 27.3, 25.6, 24.0, 20.9 ppm. HRMS (ESI) (C₂₁H₂₆NO₂⁺): Ber. 324.1958, Gef. 324.1956, *Δ* = -0.2 mmu.

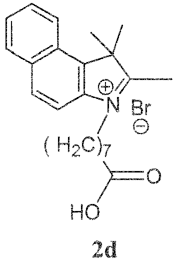

**3-(7-Carboayheptyl)-1,1,2-trimethyl-1*H*-benz[*e*]indoleniumbromid (2d):** 1,1,2-Trimethylbenz[e]indol (938 mg, 4.48 mmol) und 8-Bromoctansäure (1.00 g, 4.48 mmol) wurden 2 h auf 120°C erhitzt, abkühlen lassen, 16 h mit Diethylether (50.0 mL) gerührt, abfiltriert, in wenig Dichlormethan gelöst und mit Diethylether ausgefällt. Ausb. 1.69 mg (87%) schwarzer Feststoff, Schmp. 150°C. IR (ATR): *ṽ* = 3055 (w), 2928 (s), 2854 (w), 1720 (s), 1633 (w), 1580 (m), 1522 (m), 1463 (m), 1382 (w), 1178 (w), 1085 (w), 827 (w), 744 cm⁻¹ (w). ¹H-NMR (600 MHz, CD₃Cl₃): *δ* = 8.12 (d, 1 H, *H*ₐᵣₒₘₐₜ, *³J* = 8.8 Hz), 8.10-8.04 (m, 2 H, *H*ₐᵣₒₘₐₜ, *³J* = 8.3 Hz,), 7.80 (d, 1 H, *H*ₐᵣₒₘₐₜ, ³*J* = 8.9 Hz), 7.73 (ddd, 1 H, *H*ₐᵣₒₘₐₜ, ⁴*J* =1.1 Hz, ³*J* = 7.0 Hz, ³*J* = 8.3 Hz), 7.68-7.65 (m, 1 H, *H*ₐᵣₒₘₐₜ), 4.86-4.82 (m, 2 H, NC*H*₂), 3.22 (s, 3 H, C*H*₃), 2.36 (t, 1 H, C*H*₂, ³*J* = 7.1 Hz), 2.01-1.97 (m, 2 H, C*H*₂), 1.86 (s, 6 H, 2x C*H*₃*),* 1.65-1.60 (m, 2 H, C*H*₂), 1.57-1.51 (m, 2 H, C*H*₂), 1.46-1.40 (m, 2 H, C*H*₂), 1.39-1.33 ppm (m, 2 H, C*H*₂). ¹³C-NMR (150 MHz, CDCl₃): *δ* = 195.6, 176.5, 138.2, 137.1, 133.7, 131.5, 130.1, 128.6, 127.9, 127.6, 122.8, 112.5, 55.9, 49.6, 34.0, 28.3, 28.1, 28.0, 26.2, 24.2, 22.7, 16.0 ppm. HRMS (ESI) (C₂₃H₃₀NO₂⁺): Ber. 352.2271, Gef. 352.2271, *Δ* = 0 mmu.

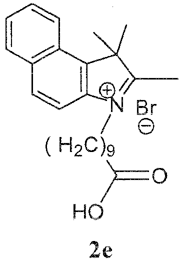

**3-(9-Carbozynonyl)-1,1,2-trimethyl-1*H*-benz[*e*]indoleniumbromid (2e):** 1,1,2-Trimethylbenz[e]indol (345 mg, 1.65 mmol) und 10-Bromdecansäure (414 mg, 1.65 mmol) wurden 1 h auf 120°C erhitzt, abkühlen lassen, mit Diethylether (50 mL) 16 h gerührt, abfiltriert, in Dichlormethan gelöst und mit Ethylacetat ausgefällt (2 d). Ausb. 112 mg (15%) blaues Öl. IR (ATR): *ṽ* = 2925 (s), 2851 (s), 1720 (s), 1633 (w), 1615 (w), 1580 (m), 1522 (m), 1463 (m), 1371 (s), 1242 (w), 1211 (w), 1173 (m), 1098 (w), 1044 (m), 997 (w), 866 (w), 825 (m), 790 (m), 746 (m), 694 cm⁻¹ (w). ¹H-NMR (200 MHz, CDCl₃): *δ* = 8.14-8.05 (m, 3 H, *H*ₐᵣₒₘₐₜ), 7.79-7.65 (m, 3 H, *H*ₐᵣₒₘₐₜ), 4.85 (t, 2 H, NC*H*₂, ³*J* = 8.8 Hz), 3.21 (s, 3 H, CH₃), 2.35 (t, 2 H, C*H*₂*,* ³*J* = 7.1 Hz), 2.03-1.93 (m, 2 H, C*H*₂), 1.87 (s, 6 H, 2 x C*H*₃), 1.69-1.29 ppm (m, 12 H, 6 x C*H*₂). ¹³C-NMR (150 MHz, CDCl₃): *δ* = 195.5, 177.0, 171.2, 138.2, 137.2, 133.7, 131.5, 130.1, 128.7, 127.9, 127.6, 122.8, 112.4, 60.4, 55.8, 49.8, 34.0, 28.5, 26.5, 24.4, 22.7, 21.0, 16.1, 14.2 ppm. HRMS (ESI) (C₂₅H₃₄NO₂⁺): Ber. 380.2584, Gef. 380.2582, *Δ* = -0.2 mmu.

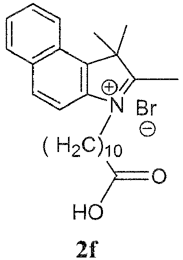

**3-(10-Carboxydecyl)-1,1,2-trimethyl-1*H*-benz[*e*]indoleniumbromid (2f):** 1,1,2-Trimethylbenz[*e*]indol (345 mg, 1.65 mmol) und 11-Bromundecansäure (437 mg, 1.65 mmol) wurden 1 h auf 120°C erhitzt, abkühlen lassen, mit Diethylether (50.0 mL) 16 h gerührt und durch Dekantieren vom Lösungsmittel befreit. Ausb. 640 mg (82%) braunes Öl. IR (ATR): *ṽ* = 3059 (w), 2964 (w), 2925 (s), 2852 (s), 1937 (w), 1709 (s), 1624 (w), 1598 (w), 1574 (m), 1520 (m), 1463 (m), 1429 (w), 1384 (w), 1362 (w), 1350 (w), 1244 (m), 1218 (m), 1118 (w), 1023 (w), 979 (w), 862 (w), 819 (s), 747 (s), 722 (w), 694 (w), 668 (w), 640 (w), 609 cm⁻¹ (w). ¹H-NMR (600 MHz, CDCl₃): *δ* = 8.01 (d, 1 H, *H*ₐᵣₒₘₐₜ, ³*J* = 8.4 Hz), 7.94 (d, 1 H, *Hₐᵣₒₘₐₜ*, ³*J* = 7.8 Hz), 7.85 (d, 1 H, *H*ₐᵣₒₘₐₜ, ³*J* = 8.6 Hz), 7.82 (d, 1 H, ³*J* = 8.5 Hz) 7.54 (ddd, 1 H, *H*ₐᵣₒₘₐₜ, ⁴*J* = 1.3 Hz, ³*J* = 6.8 Hz, ³*J* = 8.3 Hz), 7.44 (ddd, 1 H, *H*ₐᵣₒₘₐₜ, ⁴*J* = 1.1 Hz, ³*J* = 6.8 Hz, ³*J* = 8.0 Hz), 3.40 (t, 2 H, NC*H*₂, ³*J* = 6.9 Hz), 2.41 (s, 3 H, C*H*₃), 2.36 (t, 2H, C*H*₂, ³*J* = 7.5 Hz), 1.87-1.82 (m, 2 H, C*H*₂), 1.71-1.63 (m, 2 H, C*H*₂,), 1.55 (s, 6 H, 2 x C*H*₃), 1.45-1.39 (m, 2 H, C*H*₂), 1.38-1.33 (m, 2 H, C*H*₂), 1.32-1.27 ppm (m, 8 H, 4 x C*H*₂). ¹³C-NMR (150 MHz, CDCl₃): *δ* = 189.9, 178.6, 149.9, 138.4, 132.3, 129.6, 128.9, 128.6, 126.3, 124.4, 122.4, 119.6, 55.2, 34.2, 33.9, 32.8, 29.3, 29.2, 29.1, 28.6, 28.1, 24.8, 22.6, 14.9 ppm. HRMS (ESI) (C₂₆H₃₆NO₂⁺): Ber. 394.2740, Gef. 394.2743, *Δ* = 0.3 mmu.

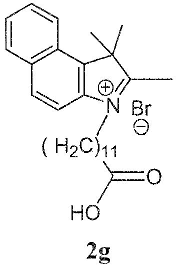

**3-(11-Carboayundecyl)-1,1,2-trimethyl-1*H*-benz[*e*]indoleniumbromid (2g):** 1,1,2-Trimethylbenz[*e*]indol (450 mg, 2.15 mmol) und 12-Bromdodecansäure (200 mg, 0.72 mmol) wurden analog zu 3-(9-Carboxynonyl)-1,1,2-trimethyl-1*H*-benz[*e*]indoleniumbromid (**2e**) umgesetzt (2.5 h) und aufgearbeitet. Ausb. 297 mg (84%) brauner Feststoff. IR (ATR): *ṽ* = 2926 (m), 2850 (m), 2489 (w), 1779 (w), 1750(w), 1716 (s), 1634 (s), 1615 (w), 1581 (s), 1523 (s),1476 (w), 1463 (s), 1388 (m), 1317 (w), 1270 (m), 1212 (w), 1174 (m), 1113 (w), 1101 (w), 1036 (w), 1026 (w), 998 (m), 940 (w), 900 (w), 865 (m), 827 (s), 789 (m), 745 (s), 719 (m), 694 (m), 616 cm⁻¹ (w). ¹H-NMR (200 MHz, CDCl₃): *δ* = 8.14-8.01 (m, 3 H, *H*ₐᵣₒₘₐₜ), 7.82-7.60 (m, 3 H, *H*ₐᵣₒₘₐₜ), 4.84 (t, 2 H, NC*H*₂, ³*J* = 7.4 Hz), 3.20 (s, 3 H, C*H*₃), 2.31 (t, 2 H, C*H*₂, ³*J* = 7.3 Hz), 1.86 (s, 6 H, 2 x C*H*₃), 1.62-1.20 ppm (m, 18 H, 9 x C*H*₂). ¹³C-NMR (150 MHz, CDCl₃): *δ* = 176.0, 138.3, 137.2, 133.7, 131.4, 130.1, 128.7, 128.0, 127.6, 122.8, 112.3, 55.8, 33.6,0 28.7, 28.6, 28.4, 28.4, 28.3, 28.3, 28.1, 26.4, 24.4, 22.7, 16.0, 3.1, 3.0, 2.7 ppm. HRMS (ESI) (C₂₇H₃₈NO₂⁺): Ber. 408.2897, Gef. 408.2895, *Δ* = -0.2 mmu.

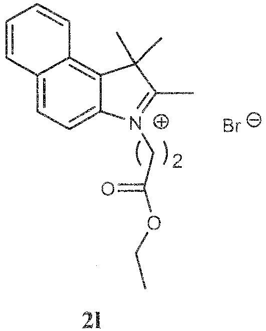

**3-(2-Ethoxycarbonylethyl)-1,1,2-trimethyl-1*H*-benz[*e*]indoleniumbromid (21):** 1,1,2-Trimethylbenz[*e*]indol (462 mg, 2.21 mmol) und 3-Brompropionsäureethylester (2 g, 11 mmol) wurden analog zu 3-(9-Carboxynonyl)-1,1,2-trimethyl-1*H-*benz[*e*]indoleniumbromid (**2e**) umgesetzt und aufgearbeitet. Ausb. 656 mg, (62%, Gehalt 81% neben Ausgangsmaterial entsprechend ¹H-NMR-Spektroskopie) brauner Feststoff. IR (ATR): *ṽ* = 3381 (m), 3056 (w), 2923 (w), 2852 (w), 2350 (w), 2287 (w), 1711 (s), 1626 (m), 1588 (s), 1554 (w), 1520 (s), 1479 (w), 1423 (m), 1357 (m), 1277 (w), 1224 (w), 1168 (w), 1142 (w), 1127 (w), 1012 (m), 971 (w), 930 (w), 898 (w), 867 (w), 806 (s), 786 (m), 746 (s), 726 (w), 685 (w), 676 (w), 652 cm⁻¹ (w). ¹H-NMR (200 MHz, CDCl₃): *δ* = 8.13-8.01 (m, 4 H, C*H*ₐᵣₒₘₐₜ), 7.75-7.60 (m, 2 H, C*H*ₐᵣₒₘₐₜ), 5.23 (t, 2 H, NC*H*₂, ³*J* = 6.0 Hz), 4.09 (q, 2 H, OC*H*₂C*H*₃, ³*J* = 7.2 Hz), 3.0 (s, 3 H, C*H*₃), 2.41-2.39 (m, 2 H, C*H*₂COOCH₂CH₃), 1.77 (s, 6 H, 2 x C*H*₃), 1.14 ppm (t, 3 H, OCH₂C*H*₃, ³*J* = 7.2 Hz). HRMS (ESI) (C₂₀H₂₄NO₂⁺): Ber. 310.1796, Gef. 310.1762, *Δ* = -0.4 mmu.

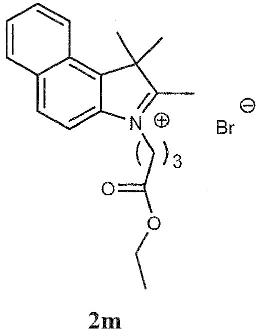

**3-(3-Ethoxycarbonylpropyl)-1,1,2-trimethyl-1*H*-benz[*e*]indoleniumbromid (2m):** 1,1,2-Trimethylbenz[e]indol (429 mg, 2.05 mmol) und 4-Brombuttersäureethylester (2 g, 10 mmol) wurden analog zu 3-(9-Carboxynonyl)-1,1,2-trimethyl-1*H-*benz[*e*]indoleniumbromid (**2e**) umgesetzt (2 h) und aufgearbeitet. Ausb. 496 mg (21%, Gehalt 35% neben Ausgangsmaterial entsprechend ¹H-NMR-Spektroskopie) grauer Feststoff. IR (ATR): *ṽ* = 3381 (w), 3056 (m), 2350 (w), 2287 (w), 1711 (s), 1626 (m), 1588 (s), 1520 (m), 1276 (m), 971 (w), 867 (m), 786 (m), 746 (m), 726 (w), 685 (m), 676 (w), 652 cm⁻¹ (w). ¹H-NMR (200 MHz, CDCl₃): *δ* = 8.13-7.97 (m, 4 H, C*H*ₐᵣₒₘₐₜ), 7.76-7.58 (m, 2 H, C*H*ₐᵣₒₘₐₜ), 5.00 (t, 2 H, NC*H*₂*,* ³*J* = 8.1 Hz), 4.08 (q, 2 H, OC*H*₂CH₃, ³*J* = 7.2 Hz), 3.27 (s, 3 H, C*H*₃), 2.76 (t, 2 H, C*H*₂COOCH_{z}CH₃, ³*J* = 6.8 Hz), 2.37-2.21 (m, 2 H, C*H*₂), 1.85 (s, 6 H, 2 x C*H*₃), 1.21 ppm (t, 3 H, OCH₂C*H*₃, ³*J* = 7.2 Hz). HRMS (ESI) (C₂₁H₂₆NO₂⁺): Ber. 324.1958, Gef. 324.1962, *Δ* = 0.4 mmu.

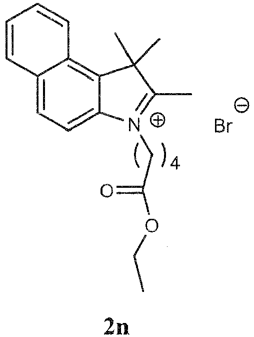

**3-(4-Ethoxycarbonylbutyl)-1,1,2-trimethyl-1*H*-benz[*e*]indoleniumbromid (2n):** 1,1,2-Trimethylbenz[*e*]indol (400 mg, 1.91 mmol) und 5-Brompentansäureethylester (2 g. 9.56 mmol) wurden analog zu 3-(9-Carboxynonyl)-1,1,2-trimethyl-1*H-*benz[e]indoleniumbromid (**2e**) umgesetzt und aufgearbeitet. Ausb. 513 mg (26%, Gehalt 40% neben Ausgangsmaterial entsprechend ¹H-NMR-Spektroskopie) blauer Feststoff. IR (ATR): *ṽ* = 3381 (m), 3056 (w), 2350 (w), 2287 (w), 1711 (s), 1626 (w), 1588 (s), 1520 (m), 1277 (w), 971 (w), 898 (w), 867 (w), 786 (m), 746 (s), 726 (w), 685 (m), 676 (w), 652 cm⁻¹ (w). ¹H-NMR (200 MHz, DMSO-d₆): *δ* = 8.40-8.03 (m, 4 H, C*H*ₐᵣₒₘₐₜ), 7.83-7.53 (m, 2 H, C*H*ₐᵣₒₘₐₜ), 4.61 (t, 2 H, NC*H*₂, ³*J* = 7.2 Hz), 4.03 (q, 2 H, OC*H*₂CH₃, ³*J* = 6.9 Hz), 2.95 (s, 3 H, C*H*₃), 2.10 (t, 2 H, C*H*₂COOCH₂CH₃, ³*J* = 7.3 Hz), 1.99-1.83 (m, 2 H, C*H*₂), 1.76 (s, 6 H, 2 x C*H*₃), 1.73-1.66 (m, 2 H, C*H*₂), 1.14 ppm (t, 3 H, OCH₂C*H*₃, ³*J* = 7.1 Hz). HRMS (ESI) (C₂₂H₂₈NO₂⁺): Ber. 338.2115, Gef. 338.2118, *Δ* = 0.3 mmu.

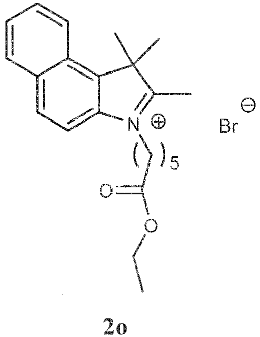

**3-(5-Ethoxycarbonylpentyl)-1,1,2-trimethyl-1*H-*benz[*e*]indoleniumbromid (2o):** 1,1,2-Trimethylbenz[e]indol (31 mg, 0.15 mmol) und 6-Bromhexansäurethylester (100 mg, 0.45 mmol) wurden analog zu 3-(7-Carboxyheptyl)-1,1,2-trimethyl-1*H-*benz[e]indoleniumbromid (**2d**) umgesetzt (1 h) und aufgearbeitet. Ausb. 41 mg (63%) gräulicher Feststoff. IR (ATR): *ṽ* = 3851 (w), 3420 (s), 2921 (m), 2851 (w), 1726 (s), 1622 (w), 1589 (m), 1556 (w), 1520 (m), 1477 (w), 1352 (m), 1227 (w), 1067 (w), 1014 (m), 941 (w), 899 (w), 805 (m), 786 (w), 744 (m), 728 (w), 652 cm⁻¹ (w). ¹H-NMR (200 MHz, CD₃OD): *δ* = 8.36-8.15 (m, 3 H, C*H*ₐᵣₒₘₐₜ), 8.02 (d, 1 H, C*H*ₐᵣₒₘₐₜ), ³*J* = 9.1 Hz), 7.86-7.66 (m, 2 H, C*H*ₐᵣₒₘₐₜ), 4.65 (t, 2 H, NC*H*₂, ³*J* = 7.5 Hz), 4.07 (q, 2 H, OC*H*₃CH₃, ³*J* = 7.2 Hz), 2.38 (t, 2 H, C*H*₂COOCH₂CH₃, ³*J* = 7.0 Hz), 2.10-2.02 (m, 2 H, C*H*₂), 1.99 (s, 3 H, C*H*₃), 1.85 (s, 6 H, 2 x C*H*₃), 1.77-1.52 (m, 4 H, C*H*₂), 1.20 ppm (t, 3 H, OCH₂C*H*₃, ³*J* = 7.1 Hz). HRMS (ESI) (C₂₃H₃₀NO₂⁺): Ber. 352.2271, Gef. 352.2275, *Δ* = 0.4 mmu.

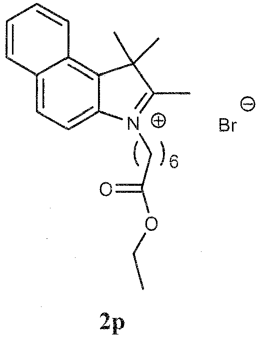

**3-(6-Ethozycarbonylhexyl)-1,1,2-trimethyl-1*H-*benz[*e*]indoleniumbromid (2p):** 1.05 g (5.04 mmol) 1,1,2-Trimethylbenz[*e*]indol und 7-Bromheptansäureethylester (400 mg, 1.68 mmol) wurden 2 h auf 120°C erhitzt. Nach dem Abkühlen wurde Diethylether (50 mL) zugegeben, 16 h gerührt und abfiltriert. Ausb. 348 mg (22%, Gehalt 48% neben Ausgangsmaterial entsprechend ¹H-NMR-Spektroskopie) gräulicher Feststoff. ¹H-NMR (600 MHz, CDCl₃): *δ* = 8.11-8.07 (m, 4 H, C*H*ₐᵣₒₘₐₜ), 7.75-7.70 (m, 2 H, C*H*ₐᵣₒₘₐₜ), 4.88 (t, 2 H, NC*H*₂, ³*J* = 7.3 Hz), 4.09 (q, 2 H, OC*H*₂CH₃, ³*J* = 7.1 Hz), 3.23 (s, 3 H, C*H*₃), 2.28 (t, 2 H, C*H*₂COOCH₂CH₃, ³*J* = 7.3 Hz), 2.03-1.99 (m, 2 H, C*H*₂), 1.88 (s, 6 H, 2 x C*H*₃), 1.65-1.59 (m, 2 H, C*H*₂), 1.56-1.52 (m, 2 H, C*H*₂), 1.46-1.40 (m, 2 H, C*H*₂), 1.22 ppm (t, 3 H, OCH₂C*H*₃, ³*J* = 7.1 Hz). HRMS (ESI) (C₂₄H₃₂NO₂⁺): Ber. 366.2428, Gef. 366.2426, *Δ* =-0.2 mmu.

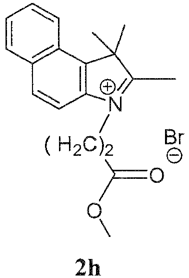

**3-(2-Methoxycarbonylethyl)-1,1,2-trimethyl-1-benz[*e*]indoleniumbromid (2h):** 1,1,2-Trimethylbenz[*e*]indol (250 mg, 1.19 mmol) und 3-Brompropionsäuremethylester (400 mg, 2.39 mmol) wurden 1 h auf 120°C erhitzt, nach dem Abkühlen mit Diethylether (50.0 mL) versetzt, 16 h gerührt, abfiltriert, in Chloroform gelöst, mit Diethylether gefällt und abgesaugt. Ausb. 321 mg (49%, Gehalt 68% neben Ausgangsmaterial entsprechend ¹H-NMR-Spektroskopie) brauner Feststoff. ¹H-NMR (200 MHz, CDCl₃): *δ* = 8.10-7.98 (m, 4 H, C*H*ₐᵣₒₘₐₜ), 7.78-7.59 (m, 2 H, C*H*ₐᵣₒₘₐₜ), 5.24 (t, 2 H, NC*H*₂, ³*J* = 5.9 Hz), 3.58 (s, 3 H, OC*H*₃), 3.27 (t, 2 H, C*H*₂COOCH₃, ³*J* = 6.0 Hz), 3.24 (s, 3 H, C*H*₃), 1.84 ppm (s, 6 H, 2 x C*H*₃). HRMS (ESI) (C₁₉H₂₂NO₂⁺): Ber. 296.1645, Gef. 296.1638, *Δ* = -0.7 mmu.

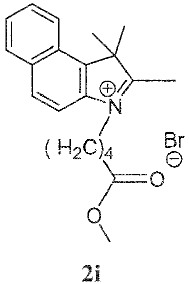

**3-(4-Methoxycarbonylbutyl)-1,1,2-trimethyl-1*H*-benz[*e*]indoleniumbromid (2i):** 1,1,2-Trimethylbenz[e]indol (214 mg, 1.02 mmol) und 5-Brompentansäuremethylester (200 mg, 1.02 mmol) wurden 2 h auf 120°C erhitzt, abkühlen lassen, mit Diethylether (50.0 mL) versetzt, 16 h gerührt und abfiltriert. Ausb. 253 mg (28%, Gehalt 46% neben Ausgangsmaterial entsprechend ¹H-NMR-Spektroskopie) gräulicher Feststoff. ¹H-NMR (200 MHz, CDCl₃): *δ* = 8.15-8.01 (m, 4 H, C*H*ₐᵣₒₘₐₜ), 7.87-7.63 (m, 2 H, C*H*ₐᵣₒₘₐₜ), 4.97 (t, 2 H, NC*H*₂, ³*J* = 7.4 Hz), 3.64 (s, 3 H, OC*H*₃), 3.25 (s, 3 H, C*H*₃), 2.46 (t, 2 H, ³*J* = 6.8 Hz), 2.19-1.98 (m, 2 H, C*H*₂), 1.89 (s, 6 H, 2 x C*H*₃), 1.78-1.70 ppm (m, 2 H, C*H*₂*).* HRMS (ESI) (C₂₁H₂₆NO₂⁺): Ber. 324.1958, Gef. 324.1949, *Δ* = -0.9 mmu.

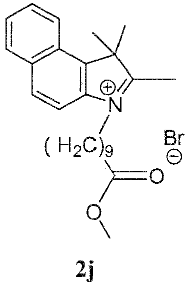

**3-(9-Methoxycarbonylnonyl)-1,1,2-trimethyl-1*H*-benz[*e*]indoleniumbromid (2j):** 1,1,2-Trimethylbenz[*e*]indol (947 mg, 4.52 mmol) und 10-Bromdecansäuremethylester (400 mg, 1.50 mmol) wurden analog zu 3-(4-Methoxycarbonylbutyl)-1,1,2-trimethyl-1*H-*benz[*e*]indoleniumbromid (**2i**) umgesetzt und aufgearbeitet. Ausb. 310 mg (18% Gehalt 40% neben Ausgangsmaterial entsprechend ¹H-NMR-Spektroskopie) gräulicher Feststoff. ¹H-NMR (200 MHz, CDCl₃): *δ* = 8.14-8.00 (m, 4 H, C*H*ₐᵣₒₘₐₜ), 7.79-7.60 (m, 2 H, C*H*ₐᵣₒₘₐₜ), 4.86 (t, 2 H, NC*H*₂, ³*J* = 7.7 Hz), 3.64 (s, 3 H, OC*H*₃), 3.22 (s, 3 H, C*H*₃), 2.27 (t, 2 H, ³*J* = 7.3 Hz), 2.08-1.90 (m, 4 H, 2 x C*H*₂), 1.88 (s, 6 H, 2 x *CH₃*), 1.60-1.30 ppm (m, 10 H, 5 x C*H*₂). HRMS (ESI) (C₂₆H₃₆NO₂⁺): Ber. 394.2741, Gef. 394.2729, *Δ* = -1.2 mmu.

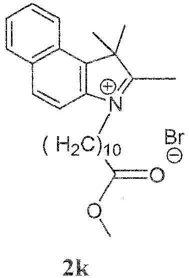

**3-(10-Methozycarbonyldecyl)-1,1,2-trimethyl-1*H-*benz[*e*]indoleniumbromid (2k):** 1,1,2-Trimethylbenz[e]indol (1.2 g, 5.73 mmol) und 11-Bromundecansäuremethylester (400 mg, 1.43 mmol) wurden analog zu 3-(4-Methoxycarbonylbutyl)-1,1,2-trimethyl-1*H-*benz[*e*]indoleniumbromid (**2i**) umgesetzt (1.5 h) und aufgearbeitet. Ausb. 270 mg (25%, Gehalt 65% neben Ausgangsmaterial entsprechend ¹H-NMR-Spektroskopie) bläulich-grauer Feststoff. ¹H-NMR (400 MHz, CDCl₃): *δ* = 8.11-7.99 (m, 4 H, C*H*ₐᵣₒₘₐₜ), 7.75-7.60 (m, 2 H, C*H*ₐᵣₒₘₐₜ), 4.86 (t, 2 H, NC*H*₂, ³*J* = 7.7 Hz), 3.64 (s, 3 H, OC*H*₃), 3.22 (s, 3 H, C*H*₃), 2.27 (t, 2 H, ³*J* = 7.5 Hz), 2.01-1.93 (m, 2 H, C*H*₂), 1.88 (s, 6 H, 2 x C*H*₃), 1.61-1.54 (m, 2 H, C*H*₂), 1.51-1.44 (m, 2 H, C*H*₂), 1.40-1.32 (m, 2 H, C*H*₂), 1.29-1.21 ppm (m, 8 H, 4 x C*H*₂). HRMS (ESI) (C₂₇H₃₈NO₂⁺): Ber. 408.2897, Gef. 408.2895, *Δ* = -0.2 mmu.

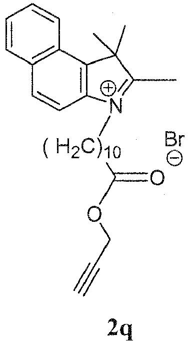

**3-(10-Propargyloxycarbonyldecyl)-1,1,2-trimethyl-1*H-*benz[*e*]indoleniumbromid** (**2q**)**:** 1,1,2-Trimethylbenz[*e*]indol (517 mg, 2.47 mmol) und 11-Bromundecansäurepropargylester (I. Ott et al., J. Med. Chem. 2005, 48, 622-629) (250 mg, 0.82 mmol) wurden analog zu 3-(4-Methoxycarbonylbutyl)-1,1,2-trimethyl-1*H-*benz[*e*]indoleniumbromid (**2i**) umgesetzt (1.5 h) und aufgearbeitet. Ausb. 148 mg (35%) schwarzer Feststoff. HRMS (ESI) (C₂₉H₃₈NO₂⁺): Ber. 432.2897, Gef. 432.2899. *Δ* = 0.2 mmu.

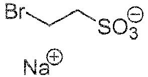

**Natrium-2-bromethansulfonat:** 1,2-Dibromethan (6.2 mL, 72 mmol) und Natriumsulfit (3 g, 24 mmol) wurden in einer Mischung aus Ethanol (25 mL) und destilliertem Wasser (20 mL) gelöst, 6 h unter Rückfluss erhitzt, abkühlen lassen, dreimal mit Chloroform extrahiert, mit Magnesiumsulfat getrocknet und im Vakuum eingedampft. Ausb. 8.3 g (55%) farbloser Feststoff, Schmp. 289 °C. IR (ATR): *ṽ* = 3600 (w), 3528 (w), 3408 (s), 2980 (w), 2946 (w), 2087 (w), 1635 (w), 1615 (s), 1435 (m), 1411 (m), 1294 (m), 1263 (w), 1202 (m), 1168 (m), 1112 (w), 1041 (s) 794 (w), 779 (w), 750(w), 674 (w), 617 cm⁻¹ (w). HRMS (ESI) (C₂H₄BrO₃S⁻): Ber. 186.9070, Gef. 186.9070. *Δ* = 0 mmu.

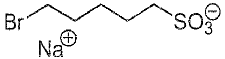

**Natrium-5-brompentansulfonat:** 1,5-Dibrompentan (16.4 g, 71 mmol), Natriumsulfit (3 g, 24 mmol), Ethanol (25 mL) und Wasser (20 mL) wurden analog zu Natrium-2-bromethansulfonat umgesetzt und aufgearbeitet. Ausb. 7.4 g (41%) farbloser Feststoff. IR (ATR): *ṽ* = 3485 (m), 3411 (w), 2978 (w), 2930 (m), 2908 (w), 2895 (w), 2867 (w), 2851 (w), 1636 (w), 1616 (w), 1487 (w), 1466 (m), 1410 (w), 1390 (w), 1329 (w), 1314 (w), 1293 (w), 1262 (w), 1224 (m), 1207 (s), 1180 (s), 1044 (s), 988 (w), 938 (w), 823 (w), 804 (w), 792 (w), 729 (w), 618 cm⁻¹ (w). HRMS (ESI) (C₅H₁₁BrNaO₃S⁺): Ber. 252.9504, Gef. 252.9820, *Δ* = 31.6 mmu.

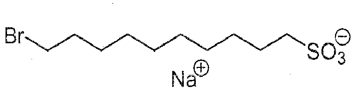

**Natrium-10-bromdecansulfonat:** 1,10-Dibromdecan (7.5 g, 25 mmol), Natriumsulfit (1.4 g, 11 mmol), Ethanol (25 mL) und Wasser (20 mL) wurden analog zu Natrium-2-bromethansulfonat umgesetzt und aufgearbeitet. Ausb. 1.8 g (23%), farbloser Feststoff, Schmp. 342 °C. IR (ATR): *ṽ* = 3541 (s), 3481 (s), 2916 (s), 2874 (m), 2853 (s), 2095 (w), 1627 (m), 1472 (m), 1307 (w), 1281 (w), 1250 (w), 1230 (m), 1197 (m), 1178 (s), 1055 (m), 1044 (m), 968 (w), 796 (m), 721 (w), 640 (w), 608 cm⁻¹ (m). HRMS (ESI) (C₁₀H₂₁BrNaO₃S⁺): Ber. 323.0287, Gef. 323.0602, *Δ* = 31.5 mmu.

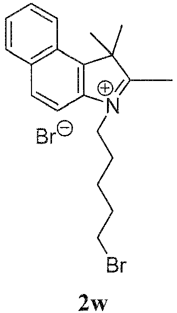

**3-(5-Brompentyl)-1,1,2-trimethyl-1*H-*benz[*e*]indoleniumbromid (2w):** 1,1,2-Trimethylbenz[e]-indol (1.0 g, 4.8 mmol) und 1,5-Dibrompentan (0.22 mL, 1.6 mmol) wurden 2 h auf 120°C erhitzt, abkühlen lassen, mit Diethylether (30 mL) versetzt, 16 h gerührt, abgesaugt, in wenig Dichlormethan gelöst und mit Diethylether gefällt. Ausb. 860 mg (54%, Gehalt 44% neben Ausgangsmaterial, ¹HNMR-spektroskopisch bestimmt) brauner Feststoff, Schmp. 123 °C. IR(ATR):*ṽ* = 3393 (s), 3058 (w), 2976 (m), 2928 (m), 2867 (w), 2714 (w), 2667 (w), 2588 (w), 2362 (m), 2336 (w), 1982 (w), 1739 (w), 1702 (w), 1632 (w), 1618 (w), 1582 (s), 1522 (s), 1464 (s), 1388 (w), 1367 (w), 1352 (w), 1279 (w), 1206 (m), 1148 (w), 1130 (w), 1039 (w), 1007 (w), 938 (w), 897 (w), 871 (w), 817 (s), 803 (s), 790 (m), 746 (s), 711 (w), 692 (w), 668 cm⁻¹ (w). ¹H-NMR (200 MHz, CDCl₃): *δ* = 8.04-7.91 (m, 4H, *H*ₐᵣₒₘₐₜ),7.70-7.49 (m, 2H, *H*ₐᵣₒₘₐₜ), 4.95 (t, 2H, NC*H*₂, ³*J* = 7.3 Hz), 3.30 (s, 3H, C*H*₃), 3.25-3.19 (m, 4H, 2 x C*H*₂), 2.41-2.25 (m, 2H, C*H*₂), 2.17-2.03 (m, 2H, C*H*₂), 1.77 ppm (s, 6H, 2 x C*H*₃).

Zusätzlich sind weitere, zum Edukt gehörende Signale im NMR-Spektrum vorhanden. Daraus ergibt sich ein Umsatz von 44%.

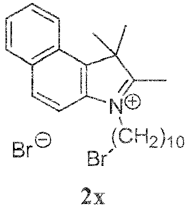

**3-(10-Bromdecyl)-1,1,2-trimethyl-1*H*-benz[*e*]indoleniumbromid (2x):** 1,1,2-Trimethylbenz[e]indol (2.0 g, 10 mmol) und 1,10-Dibromdecan (0.75 mL, 3.3 mmol) wurden 40 min auf 120 °C erhitzt, abkühlen lassen, mit Diethylether (30 mL) versetzt, 16 h gerührt und abgesaugt. Ausb 1.3 g (32%, Gehalt 40% neben Ausgangsmaterial), Schmp. 109 °C. IR(ATR): *ṽ* = 3394 (s, br), 3056 (w), 2977 (w), 2926 (s), 2854 (m), 2717 (w), 2665 (w), 2616 (w), 2591 (w), 2455 (w), 2038 (w), 1824 (w), 1633 (w), 1316 (w), 1582 (s), 1523 (m), 1465 (s), 1391 (w), 1368 (w), 1352 (w), 1334 (w), 1281 (w), 1216 (m), 1175 (w), 1147 (w), 1130 (w), 1040 (w), 1007 (w), 982 (w), 932 (w), 896 (w), 870 (w), 817 (m), 790 (w), 746 (s), 692 (w), 659 cm⁻¹ (w). ¹H-NMR (200 MHz, CDCl₃): *δ* = 8.14-7.98 (m, 4H, *H*ₐᵣₒₘₐₜ), 7.76-7.58 (m, 2H, *H*ₐᵣₒₘₐₜ), 4.93-4.82 (m, 2H, NC*H*₂), 3.29 (s, 3H, C*H*₃), 2.10-1.84 (m, 2H, C*H*₂), 1.86 (s, 6H, 2 x CH₃), 1.84-1.76 (m, 4H, 2 x C*H*₂), 1.70-1.20 ppm (m, 12H, 6 x C*H*₂). Zusätzlich sind die Signale des Ausgangsmaterials im NMR-Spektrum vorhanden und ergeben einen Umsatz von 40%. HRMS (ESI) (C₂₅H₃₅BrN⁺): Ber. 428.1947, Gef. 428.1948, *Δ* = 0.1 mmu.

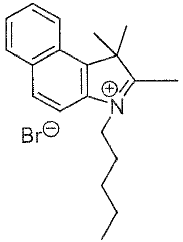

**1,1,2-Trimethyl-3-pentyl-1*H-*benz[*e*]indoleniumbromid:** 1,1,2-Trimethylbenz[*e*]indol (690 mg, 3.31 mmol) und 1-Brompentan (0.20 mL, 1.6 mmol) wurden 1.5 h auf 120 °C erhitzt, abkühlen lassen, mit Diethylether (20 mL) versetzt, 16 h gerührt und abgesaugt. Ausb. 182 mg (24%, Gehalt 80% neben Ausgangsmaterial entsprechend ¹H-NMR-Spektroskopie). brauner Feststoff, der ohne weitere Reiningung für die nachfolgende Kondensation eingesetzt wurde, Schmp. 241°C. IR (ATR): *ṽ* = 3398 (w), 3052 (w), 2929 (w), 2869 (w), 2709 (w), 2588 (w), 1819 (w), 1643 (m), 1584 (s), 1524 (m), 1464 (s), 1394 (m), 1218 (w), 1204 (w), 1148 (w), 1007 (w), 846 (w), 802 (s), 744 cm⁻¹ (s). ¹H-NMR (200 MHz, CDCl₃): *δ* = 8.15-8.01 (m, 4H, *H*ₐᵣₒₘₐₜ), 7.75-7.62 (m, 2H, *H*ₐᵣₒₘₐₜ), 4.88 (t, 2H, NC*H*₂, ³*J* = 7.7 Hz), 3.23 (s, 3H, C*H*₃), 2.05-1.96 (m, 2H, C*H*₂), 1.89 (s, 6H, 2 x C*H*₃), 1.71-1.68 (m, 2H, C*H*₂), 1.56-1.40 (m, 2H, C*H*₂), 0.92 ppm (t, 3H, CH₂C*H*₃. ³*J* = 7.0 Hz). Zusätzlich sind die Signale des Ausgangsmaterials im NMR-Spektrum vorhanden und ergeben einen Umsatz von 80%. HRMS (ESI) (C₂₀H₂₆N⁺): Ber. 280.2060, Gef. 280.2057, *Δ* = -0.3 mmu.

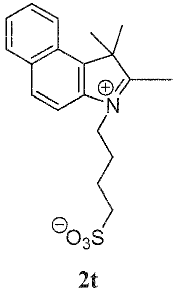

**3-(4-Sulfobutyl)-1,1,2-trimethyl-1*H-*benz[*e*]indol (2t):** 1,1,2-Trimethylbenz[e]indol (1.0 g, 4.8 mmol) und 1,4-Butansulton (0.50 mL, 4.8 mmol) wurden 2.5 h auf 130 °C erhitzt, nach dem Abkühlen abfiltriert, mit Aceton gewaschen und 3 h bei 100 °C getrocknet. Ausb. 1.13 g (70%) grau-weißer Feststoff, Schmp. 263 °C (Lit Review: H. Langhals, C. Haritoglou, Der Ophthalmologe 2009, 106, 16-20: 266 °C). IR (ATR): *ṽ* = 3435 (m), 2939 (w), 1636 (w), 1584 (w), 1523 (w), 1468 (m), 1199 (s), 1034 (s), 872 (w), 824 (m), 791 (w), 758 (m), 737 cm⁻¹ (w). ¹H-NMR (400 MHz, CD₃OD): *δ* = 8.32 (d, 1H, *H*ₐᵣₒₘₐₜ, ³*J* = 8.5 Hz), 8.24 (d, 1H, *H*ₐᵣₒₘₐₜ, ³*J* = 8.9 Hz), 8.16 (d, 1H, *H*ₐᵣₒₘₐₜ, ³*J* = 8.2 Hz), 8.06 (d, 1H, *H*ₐᵣₒₘₐₜ, ³*J* = 9.0 Hz), 7.80 (ddd, 1 H, *H*ₐᵣₒₘₐₜ, ⁴*J* = 1.3 Hz, ³*J* = 6.9 Hz, ³*J* = 8.4 Hz), 7.71 (ddd, 1H, *H*ₐᵣₒₘₐₜ, ⁴*J* = 1.1 Hz, ³*J* = 6.9 Hz, ³*J* = 8.1 Hz), 4.70- 4.64 (m, 2H, NC*H*₂), 2.91 (t, 2H, C*H*₂SO₃⁻ , ³*J* = 7.1 Hz), 2.26- 2.18 (m, 2H, CH₂), 2.03- 1.94 (m, 2H, C*H*₂), 1.84 (s, 6H, 2x CH₃), 1.30 ppm (s, 3H, C*H*₃). ¹³C-NMR (100 MHz, CD₃OD): *δ* = 196.6, 138.7, 137.5, 134.1, 131.3, 129.9, 128.5, 127.9, 127.5, 123.2, 112.7, 56.1, 49.9, 26.4, 23.0, 22.1, 21.2 ppm. HRMS (ESI) (C₁₉H₂₄NO₃S): Ber. 346.1471, Gef. 346.1473, *Δ* = 0.2 mmu.

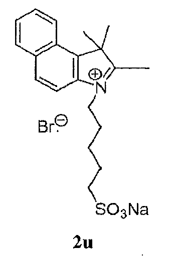

**3-(5-Sulfopentyl)-1,1,2-trimethyl-1*H*-benz[*e*]indoleniumbromid-Natriumsalz (2u):** 1,1,2-Trimethylbenz[*e*]indol (744 mg, 3.56 mmol) und Natrium-5-brompontansulfonat (300 mg, 1.18 mmol) werden 2 h auf 150 °C erhitzt, abkühlen lassen, mit Diethylethor (30 mL) versetzt, 16 h gerührt, abfiltriert, in wenig Methanol gelöst und mit Diethylether gefällt. Ausb. (59%) blau-schwarzer Feststoff, Schmp. 213 °C. IR(ATR): *ṽ* = 3054 (w), 2963 (m), 2928 (m), 2866 (w), 2661 (w), 2614 (w), 2587 (w), 2456 (w), 1960 (w), 1915 (w), 1819 (w), 1782 (w), 1700 (w), 1645 (m), 1621 (m), 1570 (s), 1519 (s), 1466 (s), 1431 (m), 1377 (m), 1346 (m), 1282 (w), 1263 (w), 1242 (m), 1218 (s), 1206 (m), 1180 (m), 1036 (w), 1022 (w), 974 (m), 929 (m), 871 (s), 827 (s), 803 (s), 755 (s), 756 (s), 682 (w), 666 (m), 608 cm⁻¹ (m). ¹H-NMR (200 MHz, CDCl₃): *δ* = 8.35-8.00 (m, 4H, *H*ₐᵣₒₘₐₜ)*,* 7.82-7.64 (m, 2H, *H*ₐᵣₒₘₐₜ), 4.64 (t, 2H, NC*H*₂, ³*J* = 7.5 Hz), 3.56 (t, 2H, C*H*₂SO₃H, ³*J* = 6.3 Hz), 2.11-1.98 (m, 2H, C*H*₂), 1.82 (s, 6H, 2 x C*H*₃), 1.79 (s, 3H, C*H*₃), 1.62-1.50 ppm (m, 4H, 2 x C*H*₂). HRMS (ESI) (C₂₀H₂₅NNaO₃S⁺): Ber. 328.1447, Gef. 328.1447, *Δ* = 0 mmu.

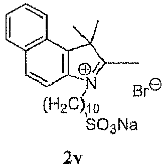

3-(10-Sulfodecyl)-1,1,2-timethyl-1*H*-benz[e]indoleniumbromid-**Natriumsalz (2v):** als Referenz 3-(10-Bromdecyl)-1,1,2-trimethyl-1*H*-benz[*e*]indoleniumbromid (600 mg, 1.18 mmol) und Natriumsulfit (50 mg, 0.39 mmol) wurden in einer Mischung aus Ethenol (5.0 mL) und Wasser (3.0 mL) gelöst, 6 h unter Rückfluss erhitzt und dann abkühlen lassen. Die organische Phase (1,10-Dibromdecan) wurde verworfen und die untere wäßrige Phase 3 Mal mit Chloroform extrahiert. Die vereinigten Chloroform-Phasen wurden über Magnesiumsulfat getrocknet und im Vakuum eingedampft. Ausb. 165 mg (79%). farbloser Feststoff. IR(ATR): *ṽ* = 2923 (s), 2853 (m), 1703 (m), 1521 (m), 1465 (w), 1352 (w), 1208 (m), 1035 (m), 810 (s), 750 cm⁻¹ (m). ¹H-NMR (200 MHz, CDCl₃): *δ* = 8.09-7.91 (m, 4H, *H*ₐᵣₒₘₐₜ), 7.77-7.64 (m, 2H, *H*ₐᵣₒₘₐₜ), 3.93-3.90 (m, 2H, NC*H*₂), 3.65-3.54 (m, 2H, C*H*₂SO₃H), 2.38 (s, 3H, 2 x C*H*₃), 2.07-1.97 (m, 2H, C*H*₂), 1.83-1.76 (4H, 2 x C*H*₂), 1.55 (s, 6H, 2 x C*H*₃), 1.39-1.22 ppm (m, 10H, 5 x C*H*₂). MS (EI⁺): *m*/*z* (%): 556.6 [M⁺ 2HNa].

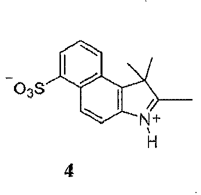

**1,1,2-Trimethyl-1H-sulfobenz[*e*]indol (4)**: als Referenz 1,1,2-Trimethylbenz[*e*]indol (1.0 g, 5.0 mmol) wurde in Nitrobenzol gelöst (5.0 mL), unter Eiskühlung vorsichtig tropfenweise mit rauchender Schwefelsäure versetzt (0.65 mL, 5.0 mmol, SO₃-Gehalt 65%, Temperaturanstieg auf 20 °C, Kühlung bis zum Ende der Rauchentwicklung), auf Raumtemperatur erwärmen lassen, 4 h gerührt, vom Nitrobenzol durch Abdekantieren und anschließender Wasserdampfdestillation befieit (bis zur Geruchlosigkeit) und eingedampft. Ausb. 1.00 g (69%). schwarzes, viskoses Öl, das ohne weitere Reinigung für die nachfolgende Kondensation eingesetzt wird. HRMS (ESI) (C₁₅H₁₄NO₃S⁻): Ber. 288.0700, Gef: 288.0698, *Δ* = -0.2 mmu. 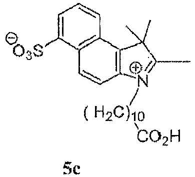 als Referenz

**3-(10-Carboxyldecyl)-1,1,2-trimethyl-1*H*-sulfobenz[*e*]indol (5c):** als Referenz 3-(10-Carboxydecyl)-1,1,2-trimethyl-1*H*-benz[*e*]indoleniumbromid (**2f**, 300mg, 0.632 mmol), Nitrobenzol (5.0 mL) und rauchende Schwefelsäure (0.08 mL, 0.63 mmol, SO₃-Gehalt 65%) wurden analog zu 1,1,2-Trimethyl-1*H*-sulfobenz[*e*]indol (**4**) umgesetzt (3 d Reaktionszeit) und aufgearbeitet. Ausb. 300 mg (85%). schwarzes, viskoses Öl, das ohne weitere Reinigung für die nachfolgende Kondensation eingesetzt wurde. ¹H-NMR (200 MHz, CD₃OD): *δ* = 8.41-8.01 (m, 4H, *H*ₐᵣₒₘₐₜ), 7.87-7.66 (m, 1H, *H*ₐᵣₒₘₐₜ), 4.63 (t, 2H, NC*H*₂, ³*J* = 7.3 Hz), 3.35 (s, 3H, C*H*₃), 2.28 (t, 2H, C*H*₂CO₂CH₃, ³*J* = 7.3 Hz), 2.69-1.93 (m, 2H, C*H*₂), 1.82 (s, 6H, 2 x CH₃), 1.64-1.30 ppm (m, 14H, 7 x C*H*₂). HRMS (ESI) (C₂₆H₃₄NO₅S⁻): Ber. 472.2169, Gef. 472.2159, *Δ* = -1 mmu.

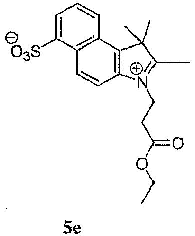

**3-(2-Ethoxycarbonylethyl)-1,1,2-trimetyl-1*H*-sulfobenz[*e*]indol** als Referenz **(5e):** 3-(2-Ethoxycarbonylethyl)-1,1,2-trimethyl-1*H*-benz[*e*]indoleniumbromid (300 mg, 0.768 mmol), Nitrobeozol (5.0 mL) und rauchende Schwefelsäure (0.10 mL, 0.77 mmol, SO₃-Gehalt 65%) wurden analog zu **5c** umgesetzt und aufgearbeitet. Ausb. 250 mg (69%) schwarzer, viskoses Öl, das ohne weitere Reinigung für die nachfolgende Kondensation eingesetzt wurde. ¹H-NMR (200 MHz, CD₃OD): *δ* = 8.37-8.03 (m, 4H, *H*ₐᵣₒₘₐₜ), 7.84-7.66 (m, 1H, *H*ₐᵣₒₘₐₜ), 4.96-4.87 (m, 2H, NC*H*₂), 4.22-4.20 (m, 2H, OC*H*₂CH₃), 3.32-3.13 (m, 2H, CO₂C*H*₂CH₃), 2.95-2.85 (m, 3H, OCH₂C*H*₃), 1.82 ppm (s, 6H, 2 x CH₃). Die 2-Methyl-Protonen tauschen mit dem Lösemitttel aus und erscheinen daher nicht im NMR-Spektrum.

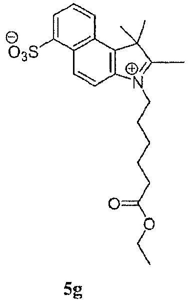

**3-(5-Ethorycarbonylpentyl)-1,1,2-trimethyl-1*H*-sulfobenz[*e*]indol** als Referenz **(5g):** 3-(5-Ethoxycarbonylpentyl)-1,1,2-trimethyl-1*H*-benz[*e*]indoleniumbromid (230 mg, 0.532 mmol) und Nitrobenzol (5.0 mL) und rauchende Schwefelsäure (0.06 mL, 0.53 mmol, SO₃-Gehalt von 65%) wurden analog zu **5c** umgesetzt und aufgearbeitet. Ausb. 111 mg (41%) schwarzes, viskoses Öl, das ohne weitere Reinigung für die nachfolgende Kondensation eingesetzt wurde. ¹H-NMR (200 MHz, CD₃OD): *δ* = 8.39-8.29 (m, 2H, *H*ₐᵣₒₘₐₜ), 8.18-8.01 (m, 2H, *H*ₐᵣₒₘₐₜ), 7.87-7.74 (m, 1H, *H*ₐᵣₒₘₐₜ), 4.64 (t, 2H, NC*H*₂, ³*J* = 7.6 Hz), 4.20-4.17 (m, 2H, OC*H*₂CH₃), 2.95-2.85 (m, 3H, OCH₂C*H*₃), 2.37 (t, 2H, C*H*₂CO₂CH₂CH₃, ³*J* = 6.9 Hz), 2.10-1.95 (m, 2H, C*H*₂), 1.81 (s, 6H, 2 x CH₃), 1.74-1.50 ppm (m, 4H, 2 x C*H*₂). Die Methyl-Protonen in Postition 2 tauschen mit dem Lösungsmittel aus und erscheinen daher nicht im NMR-Spektrum. HRMS (ESI) (C₂₃H₃₀O₅S⁺): Ber. 432.1839, Gef. 432.1484, *Δ* = 35.5 mmu.

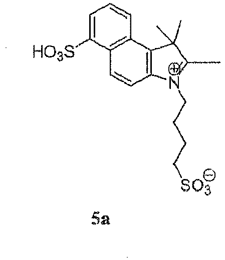

**3-(4-Sulfobutyl)-1,1,2-trimethyl-1*H*-sulfobenz[*e*]indol (5a):** als Referenz 3-(4-Sufobutyl)-1,1,2-trimethyl-1*H*-benz[*e*]indol (300 mg, 0.868 mmol), Nitrobenzol (5.0 mL) und rauchende Schwefelsäure (0.11 mL, 0.87 mmol, SO₃-Gehalt 65%) wurden analog zu 1,1,2-Trimethyl-1*H-*sulfobenz[e]indol (4) umgesetzt (2 d rühren bei Raumtemperatur) und aufgearbeitet. Ausb. 200 mg (46%) schwarzes, viskoses Öl, das ohne weitere Reinigung für die nachfolgende Kondensation eingesetzt wurde. HRMS (ESI) (C₁₉H₂₄NO₆S₂⁺):Ber. 426.1040, Gef. 426.1039, *Δ* = -0.1 mmu.

### Synthese von Carbocyaninfarbstoffen

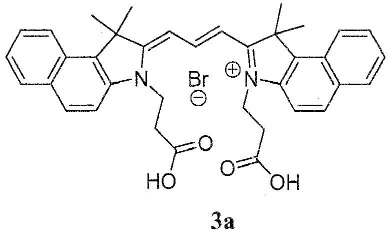

**3,3'-Di-(2-carboryethyl)-1,1,1',1'-tetramethyl-1*H*-dibenz[*e*]indocarbocyaninbromid** (3a): 3-(2-Carboxyethyl)-1,1,2-trimethyl-1*H*-benz[*e*]-indoleniumbromid (2a, 540mg, 1.50 mmol) wurde unter Argon-Atmosphäre in Pyridin (2.00 mL) gelöst, auf 116°C erhitzt, langsam tropfenweise mit Orthoameisensäuretriethylester (0.250 mL, 3.00 mmol) versetzt (Blauviolettfärbung), 2 h unter Rückfluss erhitzt, nach dem Abkühlen mit Diethylether (10.0 mL) versetzt (Ausfällen des Farbstoffs als goldglänzender Feststoff), dekantiert, mehrfach in wenig Ethanol gelöst und mit Diethylether gefällt, schließlich abfiltriert, im Vakuum getrocknet und mit Flashchromatographie gereinigt (RP 18, Methanol/H₂O/Eisessig 1:1:0.4 zum Auftragen des Farbstoffs, Methanol/H₂O/Eisessig 2:1:0.4 zur Elution von Nebenprodukten, Methanol/H₂O/Eisessig 10:1:0.4 zur Elution des Farbstoffs). Ausb. 600 mg (70%) goldglänzender Feststoff, der violette, rot fluoreszierende Lösungen bildet, Schmp. 199°C. *R_{f}* (RP 18, Methanol/H₂O/Eisessig 10:1:0.4) = 0.62. IR (ATR): *ṽ* = 3344 (w, br), 2921 (m, br), 1723 (s), 1633 (w), 1586 (w), 1552 (s), 1519 (m), 1471 (m), 1422 (s), 1350 (m), 1279 (w), 1226 (m), 1153 (m), 1127 (m), 1011 (m), 924 (s), 876 (w), 803 (w), 787 (w), 747 (w), 729 (w), 681 (w), 651 cm⁻¹ (w). ¹H-NMR (200 MHz, CD₃OD): *δ* = 8.27 (d, 2 H, *H*ₐᵣₒₘₐₜ, ³*J* = 8.7 Hz), 8.01 (t, 4 H, *H*ₐᵣₒₘₐₜ, ³*J* = 7.6 Hz), 7.67 (t, 5 H, *Hₐᵣₒₘₐₜ, ³J* = 6.6 Hz), 7.50 (t, 2 H, *H*ₐᵣ*ₒₘₐₜ, H*_{ally}*, ³J* = 7.5 Hz), 6.57 (d, 2 H, *H*_{allyl}, ³*J_{E}* = 12.9 Hz), 4.68-4.45 (m, 4 H, 2 x NC*H*₂), 2.95-2.75 (m, 4 H, 2 x C*H*₂), 2.06 ppm (s, 12 H, 4 x C*H*₃). ¹³C-NMR (100 MHz, CD₃OD): *δ* = 176.1, 149.4, 139.2, 133.5, 132.2, 130.4, 129.7, 127.9, 127.4, 124.9, 121.9, 110.9, 110.9, 110.8, 65.5, 51.0, 26.6, 14.0 ppm. UV/VIS (EtOH): *λ*ₘₐₓ (*E*ᵣₑₗ): 591 (1.0), 554 nm (0.68). UV/VIS (Feststoff/Baumwolle): *λ*ₘₐₓ (*E*ᵣₑₗ): 599 (1.0), 555 nm (0.98). UV/VIS (Feststoff/Wolle): *λ*ₘₐₓ (*E*ᵣₑₗ): 559 (1.0), 595 nm (0.98). UV/VIS (Feststoff/Haare): *λ*ₘₐₓ (*E*ᵣₑₗ): 595 (1.0), 555 nm (0.98). Fluoreszenz (EtOH): *λ*ₘₐₓ (*I*ᵣₑₗ): 622 (1.0), 664 nm (0.70). Fluoreszenz (Feststoff/Baumwolle): *λ*ₘₐₓ (*I*ᵣₑₗ): 667 (1.0), 643 nm (0.85). Fluoreszenz (Feststoff/Wolle): *λ*ₘₐₓ (*I*ᵣₑₗ): 635 (1.0), 667 nm (0.95). Fluoreszenz (Feststoff/Haare): *λ*ₘₐₓ (*I*ᵣₑₗ): 624 (1.0), 665 nm (0.69). Fluoreszenzquantenausb. (CHCl₃, *λ*ₑₓₑ = 562 nm, *E*_{562/1 cm} = 0.0146; Referenz: S-13 mit *Φ* = 1.00): 0.35. HRMS (ESI) (C₃₇H₃₇N₂O₄⁺): Ber. 573.2748, Gef. 573.2747, *Δ* = -0.1 mmu.

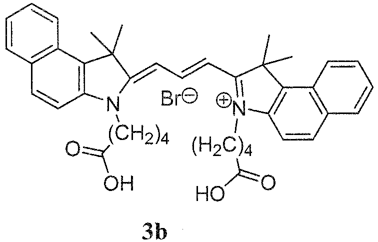

**3,3'-Di-(4-carboxybutyl)-1,1,1',1'-tetramethyl-1*H*-dibenz[*e*]indocarbocyaninbromid** (3b): 3-(4-Carboxybutyl)-1,1,2-trimethyl-1*H*-benz[*e*]indoleniumbromid (2b, 140 mg, 0.36 mmol), 3-Picolin (1.00 mL) und Orthoameisensäuretriethylester (0.12 mL, 0.72 mmol) wurden wie bei 3,3'-Di-(2-carboxyethyl)-1,1,1',1'-tetramethyl-1*H*-dibenz[*e*]indocarbo-cyaninbromid (3a) beschrieben umgesetzt und aufgearbeitet. Ausb. 100 mg (39%) goldglänzender Feststoff, der violette, rot fluoreszierende Lösungen bildet. *R_{f}* (RP 18, Methanol/H₂O/Eisessig 10:1:0.4) = 0.43. IR (ATR): *ṽ* = 2922 (s), 2348 (m), 2213 (w), 1707 (m), 1555 (s), 1519 (s), 1479 (m), 1427 8s), 1360 (m), 1226 (m), 1154 (m), 1014 (m), 936 (m), 805 8w), 746 cm⁻¹ (w). ¹H-NMR (400 MHz, CD₃OD): *δ* = 8.28 (d, 2 H, *H*ₐᵣₒₘₐₜ, *J* = 8.5 Hz), 8.03 (dd, 5 H, *H*ₐᵣₒₘₐₜ, *H*_{allyl}, ³*J* = 8.5 Hz, ³*J_{E}* = 12.3 Hz), 7.70-7.64 (m, 4 H, *H*ₐᵣₒₘₐₜ), 7.55-7.48 (m, 2 H, *H*ₐᵣₒₘₐₜ), 6.55 (d, 2 H, *H*_{ally},*³J_{E}* = 13.0 Hz), 4.39-4.28 (m, 4 H, 2 x NC*H*₂), 2.49-2.35 (m, 4 H, 2 x C*H*₂), 2.09 (s, 12 H, 4 x C*H*₃), 2.00-1.91 (m, 4 H, 2 x C*H*₂) 1.90-1.80 ppm (m, 4 H, 2 x C*H*₂). ¹³C-NMR (100 MHz, CD₃OD): *δ* = 175.9, 149.3, 39.4, 133.5, 132.2, 130.5, 129.7, 127.9, 127.4, 124.8, 121.9, 110.8, 101.9, 101.9, 50.9, 43.8, 26.6 ppm. UV/VIS (EtOH): *λ*ₘₐₓ (*E*ᵣₑₗ): 591 (100), 552 nm (69). UV/VIS (Feststoff/Baumwolle): *λ*ₘₐₓ (*E*ᵣₑₗ): 598 (1.0), 559 nm (0.98). UV/VIS (Feststoff/Wolle): *λ*ₘₐₓ (*E*ᵣₑₗ): 558 (1.0), 591 nm (0.95). UV/VIS (Feststoff/Haare): λₘₐₓ (*E*ᵣₑₗ): 598 (1.0), 560 nm (0.81). Fluoreszenz (EtOH): *λ*ₘₐₓ (*I*ᵣₑₗ): 608 (1.0), 652 nm (0.30). Fluoreszenz (Feststoff/Baumwolle): *λ*ₘₐₓ (*I*ᵣₑₗ): 667 (1.0), 628 nm (0.66). Fluoreszenz (Feststoff/Wolle): *λ*ₘₐₓ (*I*ᵣₑₗ): 635 (1.0), 668 nm (0.97). Fluoreszenz (Feststoff/Haare): *λ*ₘₐₓ (*I*ᵣₑₗ): 624 (1.0), 665 nm (0.68). Fluoreszenzquantenausb. (CHCl₃, *λ*_{exc} = 559 nm, *E*_{559/1 cm} = 0.0120, Referenz: S-13 mit *Φ* = 1.00): 0.49. HRMS (ESI) (C₄₁H₄₅N₂O₄): Ber. 629.3374 Gef. 629.3380, *Δ* = 0.6 mmu.

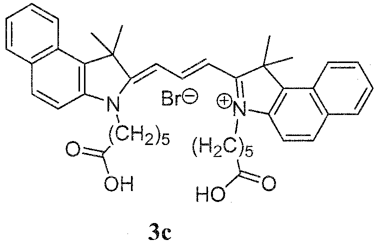

**3,3'-Di-(5-carbozypentyl)-1,1,1',1'-tetramethyl-1*H*-dibenz[*e*]indocarbocyaninbromid** (3c): 3-(5-Carboxypentyl)-1,1,2-trimethyl-1*H*-benz[*e*]indoleniumbromid (**2c,** 150 mg, 0.37 mmol), 3-Picolin (2.00 mL) und Orthoameisensäuretriethylester (0.10 mL, 0.74 mmol) wurden wie bei 3,3'-Di-(2-carboxyethyl)-1,1,1',1'-tetramethyl-1*H*-dibenz[*e*]indocarbo-cyaninbromid (**3a**) beschrieben umgesetzt und aufgearbeitet. Ausb. 150 mg (55%) goldglänzender Feststoff, der violette, rot fluoreszierende Lösungen bildet. *R_{f}* (RP 18, Methanol/H₂O/Eisessig 10:1:0.4) = 0.66. IR (ATR): *ṽ* = 3416 (w, br), 3054 (w), 2978 (w), 2935 (w), 2859 (w), 1727 (s), 1626 (w), 1588 (w), 1547 (s), 1519 (m), 1487 (m), 1469 (w), 1424 (s), 1370 (w), 1348 (w), 1275 (w), 1227 (s), 1170 (m), 1140 (m), 1125 (m), 1031 (w), 1011 (m), 977 (m), 926 (s), 891 (w), 878 (w), 865 (w), 829 (w), 805 (m), 787 (w), 769 (w), 748 (w), 725 (w), 684 (w), 652 cm⁻¹ (w). ¹H-NMR (400 MHz, CD₃OD): *δ* = 8.78 (t, 1 H, *H*_{Allyh}, ³*J* = 13.5 Hz), 8.30 (d, 2 H, *H*ₐᵣₒₘₐₜ*,* ³*J* = 8.5 Hz), 8.06 (d, 2 H, *H*ₐᵣₒₘₐₜₒ ³*J* = 8.8 Hz), 8.02 (d, 2 H, *H*ₐᵣₒₘₐₜ, ³*J* = 8.2 Hz), 7.68 (ddd, 2 H, *H*ₐᵣₒₘₐₜ, ⁴*J =* 1.3 Hz, ³*J* = 6.9 Hz, ³*J* = 8.4 Hz), 7.64 (d, 2 H, *H*ₐᵣₒₘₐₜ, ³*J* = 8.9 Hz), 7.52 (ddd, 2 H, *H*ₐᵣₒₘₐₜ, ⁴*J* = 1.0 Hz, ³*J* = 6.9 Hz, ³*J* = 8.1 Hz), 6.53 (d, 2 H, *H*_{Allyl}, ³*J_{E}* = 13.5 Hz), 4.30 (t, 4 H, 2 x NC*H*₂, ³*J* = 7.5 Hz), 2.35 (t, 4 H, 2 x C*H*₂CO₂H, ³*J* = 7.2 Hz), 2.11 (s, 12H, 4 x C*H*₃), 2.04-1.86 (m, 4H, 2 x C*H*₂), 1.79-1.70 (m, 4H, 2 x C*H*₂), 1.63-1.55 ppm (m, 4 H, 2 x C*H*₂). ¹³C-NMR (100 MHz, CD₃OD): *δ* = 175.9, 149.3, 139.4, 133.5, 132.2, 130.5, 129.7, 127.9, 127.4, 124.9, 121.9, 110.8, 101.7, 51.0, 43.9, 27.1, 26.6, 26.1, 22.8, 16.9 ppm. UV/VIS (EtOH): *λ*ₘₐₓ(*E*ᵣₑₗ): 591 (1.0), 554 nm (0.68). UV/VIS (Feststoff/Baumwolle): *λ*ₘₐₓ (*E*ᵣₑₗ): 598 (1.0), 554 nm (0.99). UV/VIS (Feststoff/Wolle): *λ*ₘₐₓ (*E*ᵣₑₗ): 558 (1.0), 594 nm (0.97). UV/VIS (Feststoff/Haare): *λ*ₘₐₓ (*E*ᵣₑₗ): 598 (1.0), 556 nm (0.90). Fluoreszenz (EtOH): *λ*ₘₐₓ (*I*ᵣₑₗ): 611 (1.0), 655 nm (0.50). Fluoreszenz (Feststoff/Baumwolle): *λ*ₘₐₓ (*I*ᵣₑₗ): 661 (1.0), 644 nm (0.93). Fluoreszenz (Feststoff/Wolle): *λ*ₘₐₓ (*I*rel): 632 (1.0), 667 nm (0.88). Fluoreszenz (Feststoff/Haare): *λ*ₘₐₓ (*I*ᵣₑₗ): 623 (1.0), 660 nm (0.71). Fluoreszenzquantenausb. (CHCl₃, *λ*ₑₓₑ = 559 nm, E_{562/1} um = 0.0126; Referenz: S-13 mit *Φ* = 1.00): 0.50. HRMS (ESI) (C₄₃H₄₉N₂O₄⁺): Ber. 657.3687, Gef. 657.3691, *Δ* = 0.4 mmu.

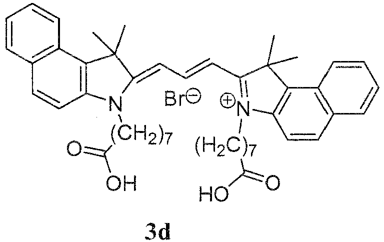

**3,3'-Di-(7-carboxyheptyl)-1,1,1',1'-tetramethyl-1*H*-dibenz[*e*]indocarbocyaninbromid** (**3d**): 3-(7-Carboxyheptyl)-1,1,2-trimethyl-1*H*-benz[*e*]-indoleniumbromid (**2d**, 150 mg, 0.35 mmol), 3-Picolin (1.00 mL) und Orthoameisensäuretriethylester (0.12 mL, 0.70 mmol) wurden wie bei 3,3'-Di-(2-carboxyethyl)-1,1,1',1'-tetramethyl-1*H*-dibenz[*e*]indocarbocyaninbromid (**3a**) beschrieben umgesetzt und aufgearbeitet. Ausb. 80.0 mg (54%) goldglänzender Feststoff, der violette, rot fluoreszierende Lösungen bildet. IR (ATR): *ṽ* = 2927 (s), 2348 (w), 2233 (w), 1720 (s, br), 1552 (s), 1519 (m), 1479 (w), 1422 (s), 1351 (m), 1225 (m), 1141 (m), 1011 (m), 931 (m), 806 cm⁻¹ (w). ¹H-NMR (400 MHz, CD₃OD): *δ* = 8.78 (t, 1 H, *H*_{Allyl}, ³*J_{E}* = 13.5 Hz), 8.30 (d, 2 H, *H*ₐᵣₒₘₐₜ*,* ³*J* = 8.3 Hz), 8.05 (t, 4 H, *H*ₐᵣₒₘₐₜ, ³*J* = 7.8 Hz), 7.73-7.61 (m, 4 H, *H*ₐᵣₒₘₐₜ), 7.57-7.49 (m, 2 H, *H*ₐᵣₒₘₐₜ), 6.53 (d, 2 H, *H*_{Allyl}*,* ³*J_{E}* = 13.3 Hz), 4.29 (t, 4 H, 2 x NC*H*₂, ³*J* = 7.7 Hz), 2.29 (t, 4 H, 2 x CH₂CO₂H, ³*J* = 7.3 Hz), 2.11 (s, 12 H, 4 x C*H*₃), 1.99-1.84 (m, 4 H, 2 x C*H*₂), 1.63-1.40 ppm (m, 16 H, 8 x C*H*₂). ¹³C-NMR (100 MHz, CD₃OD): *δ* = 175.9, 133.5, 132.2, 130.5, 129.7, 127.9, 127.4, 125.2, 124.9, 121.9, 112.4, 110.7, 101.7, 57.0, 51.0, 26.6, 16.9 ppm. UV/VIS (EtOH): *λ*ₘₐₓ (*E*ᵣₑₗ): 591 (1.0), 554 nm (0.70). UV/VIS (Feststoff/Baumwolle): *λ*ₘₐₓ (*E*ᵣₑₗ): 599 (1.0), 554 nm (0.99). UV/VIS (Feststoff/Wolle): *λ*ₘₐₓ (*E*ᵣₑₗ): 558 (1.0), 595 nm (0.97). UV/VIS (Feststoff/Haare): *λ*ₘₐₓ (*E*ᵣₑₗ): 599 (1.0), 555 nm (0.92). Fluoreszenz (EtOH): *λ*ₘₐₓ (*I*ᵣₑₗ): 602 (1.0), 661 nm (0.25). Fluoreszenz (Feststoff/Baumwolle): *λ*ₘₐₓ (*I*ᵣₑₗ): 669 (1.0), 646 nm (0.94). Fluoreszenz (Feststoff/Wolle): *λ*ₘₐₓ (*I*ᵣₑₗ): 632 (1.0), 668 nm (0.89). Fluoreszenz (Feststoff/Haare): *λ*ₘₐₓ (*I*ᵣₑₗ): 635 (1.0), 666 nm (0.86). Fluoreszenzquantenausb. (CHCl₃, *λ*ₑₓₑ = 558 nm, *E*_{562/1 cm} = 0.0161, Referenz: S-13 mit *Φ* = 1.00): 0.43.HRMS (ESI) (C₄₇H₅₇N₂O₄⁺): Ber. 713.4313, Gef. 713.4324, *Δ* = 1.1 mmu.

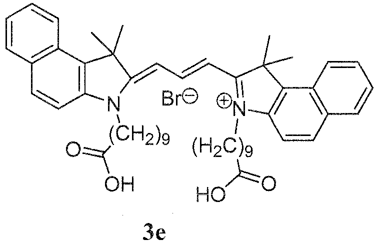

**3,3'-Di-(9-carboxynonyl)-1,1,1',1'-tetramethyl-1*H*-dibenz[*e*]indocarbocyaninbromid** (**3e**): 3-(9-Carboxynonyl)-1,1,2-trimethyl-1*H*-benz[*e*]indoleniubromid (**2e**, 100 mg, 0.21 mmol), 3-Picolin (1.00 mL) und Orthoameisensäuretriethylester (0.07 mL, 0.43 mmol) wurden wie bei 3,3'-Di-(2-carboxyethyl)-1,1,1',1'-tetramethyl-1*H*-dibenz[*e*]indocarbocyaninbromid (**3a**) beschrieben umgesetzt und aufgearbeitet. Ausb. 41.0 mg (23%) goldglänzender Feststoff, der violette, rot fluoreszierende Lösungen bildet. *R_{f}* (RP 18, Methanol/H₂O/Eisessig 10 : 1 : 0.4) = 0.4. IR (ATR): *ṽ* = 3381 (w), 3056 (w), 2923 (s), 2852 (s), 2350 (w), 2287 (w), 1711 (m), 1626 (w), 1588 (w), 1554 (s), 1520 (m), 1479 (m), 1423 (s), 1357 (m), 1277 (w), 1224 (m), 1168 (m), 1142 (m), 1127 (m), 1012 (m), 971 (w), 930 (s), 898 (w), 867 (w), 806 (m), 786 (w), 746 (w), 726 (w), 685 (w), 676 (w), 652 cm⁻¹ (m). ¹H-NMR (400 MHz, CD₃OD): *δ* = 8.78 (t, 1 H, *H*_{Allyl}, ³*J_{E}* = 13.2 Hz), 8.29 (d, 2 H, *H*ₐᵣₒₘₐₜ, ³*J* = 8.6 Hz), 8.06 (d, 2 H, *H*ₐᵣₒₘₐₜ, ³*J* = 8.8 Hz), 8.03 (d, 2 H, *H*ₐᵣₒₘₐₜ, ³*J* = 8.1 Hz), 7.72-7.62 (m, 4 H, *H*ₐᵣₒₘₐₜ), 7.55-7.50 (m, 2 H, *H*ₐᵣₒₘₐₜ), 6.51 (d, 2 H, *H*_{Allyl}*,* ³*J_{E}* = 13.5 Hz), 4.29 (t, 4 H, 2 x NC*H*₂, ³*J* = 7.5 Hz), 2.20 (t, 4 H, 2 x C*H*₂CO₂H, ³*J* = 7.2 Hz), 2.11 (s, 12 H, 4 x C*H*₃), 1.96-1.88 (m, 8 H, 4 x C*H*₂), 1.60-1.50 (m, 8 H, 4 x C*H*₂), 1.49-1.40 (m, 4 H, 2 x C*H*₂), 1.33-1.30 ppm (m, 8 H, 4 x C*H*₂). ¹³C-NMR (100 MHz, CD₃OD): *δ* = 175.9, 149.3, 139.4, 133.5, 132.2, 130.5, 129.8, 127.9, 127.5, 124.9, 121.9, 110.8, 101.6, 51.0, 44.0, 28.9, 28.9, 28.8, 27.4, 26.6, 26.3, 25.2, 16.9 ppm. UV/VIS (EtOH): *λ*ₘₐₓ (*E*ᵣₑₗ): 594 (1.0), 555 nm (0.65). UV/VIS (Feststoff/Baumwolle): *λ*ₘₐₓ (*E*ᵣₑₗ): 569 (1.0), 557 nm (0.99). UV/VIS (Feststoff/Wolle): *λ*ₘₐₓ (*E*ᵣₑₗ): 565 (1.0), 597 nm (0.90). UV/VIS (Feststoff/Haare): *λ*ₘₐₓ (*E*ᵣₑₗ) 603 (1.0), 557 nm (0.95). Fluoreszenz (EtOH): *λ*ₘₐₓ (*I*ᵣₑₗ): 604 (1.0), 651 nm (0.25). Fluoreszenz (Feststoff/Baumwolle): *λ*ₘₐₓ (*I*ᵣₑₗ): 669 (1.0), 645 nm (0.94). Fluoreszenz (Feststoff/Wolle): *λ*ₘₐₓ (*I*ᵣₑₗ): 632 (1.0), 668 nm (0.89). Fluoreszenz (Feststoff/Haare): *λ*ₘₐₓ (*I*ᵣₑₗ): 635 (1.0), 666 nm (0.87). Fluoreszenzquantenausb. (CHCl₃, *λ*_{exc} = 557 nm, *E*_{562/1 cm} = 0.0180, Referenz: S-13 mit *Φ* = 1.00): 0.59. HRMS (ESI) (C₅₁H₆₅N₂O₄⁺): Ber. 769.4939, Gef. 769.4941, *Δ* = 0.2 mmu.

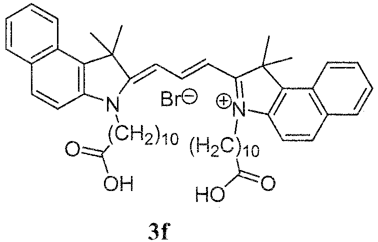

**3,3'-Di-(10-carboxydecyl)-1,1',1'-tetramethyl-1*H*-dibenz[*e*]indocarbocyaninbromid** (3f): 3-(10-Carboxy-decyl)-1,1,2-trimethyl-1*H*-benz[*e*]indoleniumbromid (2f, 117 mg, 0.25 mmol), Pyridin (2.5 mL) und Orthoameisensäuretriethylester (0.08 mL, 0.5 mmol) wurden wie bei 3,3'-Di-(2-carboxyethyl)-1,1,1',1'-tetramethyl-1*H* dibenz[*e*]indocarbocyaninbromid (**3a**) beschrieben umgesetzt (120°C) und aufgearbeitet. Ausb. 118 mg (54%) goldglänzender Feststoff, der violette, rot fluoreszierende Lösungen bildet. IR (ATR): *ṽ* = 3381 (w), 3056 (w), 2923 (s), 2852 (s), 2350 (w), 2287 (w), 1711 (m), 1626 (w), 1588 (w), 1554 (s), 1520 (m), 1479 (m), 1423 (s), 1357 (m), 1277 (w), 1224 (m), 1012 (m), 971 (w), 930 (s), 898 (w), 867 (w), 806 (m), 786 (w), 746 (w), 726 (w), 685 (w), 676 (w), 652 cm⁻¹ (m). ¹H-NMR (400 MHz, CD₃OD): *δ* = 8.77 (t, 1 H, C*H*_{Allyl}, ³*J_{E}* = 13.4 Hz), 8.29 (d, 2 H, C*H*ₐᵣₒₘₐₜ, ³*J* = 8.6 Hz), 8.05 (d, 2 H, C*H*ₐᵣₒₘₐₜ, ³*J* = 8.7 Hz), 8.02 (d, 2 H, C*H*_{aromat,} ³*J* = 8.4 Hz), 7.70-7.62 (m, 4 H, C*H*ₐᵣₒₘₐₜ), 7.52 (t, 2 H, C*H*ₐᵣₒₘₐₜ, ³*J* = 7.5 Hz), 6.50 (d, 2 H, C*H*_{Allyl}, ³*J_{E}* = 13.6 Hz), 4.28 (t, 4 H, 2 x NC*H*₂*,* ³*J* = 7.1 Hz), 2.30-2.13 (m, 4 H, 2 x C*H*₂CO₂H), 2.10 (s, 12 H, 4 x C*H*₃), 1.98-1.88 (m, 4 H, 2 x C*H*₂), 1.58-1.50 (m, 8 H, 4 x C*H*₂), 1.35-1.22 ppm (m, 20 H, 10 x C*H*₂). ¹³C-NMR (100 MHz, CD₃OD): *δ* = 175.9, 149.2, 139.4, 133.5, 132.2, 131.3, 130.5, 129.7, 127.9, 127.5, 124.9, 121.9, 114.7, 110.8, 101.6, 51.0, 44.0, 29.0, 29.0, 28.9, 27.4 26.6, 26.3, 22.8 ppm. UV/VIS (EtOH): *λ*ₘₐₓ (*E*ᵣₑₗ): 590 (1.0), 553 nm (0.70). UV/VIS (Feststoff/Baumwolle): *λ*ₘₐₓ (*E*ᵣₑₗ): 597 (1.0), 555 nm (0.99). UV/VIS (Feststoff/Wolle): *λ*ₘₐₓ (*E*ᵣₑₗ): 557 (1.0), 596 nm (0.96). UV/VIS (Feststoff/Haare): *λ*ₘₐₓ (*E*ᵣₑₗ): 601 (1.0), 556 nm (0.97). Fluoreszenz (EtOH): *λ*ₘₐₓ (*I*ᵣₑₗ): 610 (1.0), 656 nm (0.54). Fluoreszenz (Feststoff/Baumwolle): *λ*ₘₐₓ (*I*ᵣₑₗ): 670 (1.0), 641 nm (0.83). Fluoreszenz (Feststoff/Wolle): *λ*ₘₐₓ (*I*ᵣₑₗ): 667 (1.0), 641 nm (0.98). Fluoreszenz (Feststoff/Haare): *λ*ₘₐₓ (*I*ᵣₑₗ): 640 (1.0), 667 nm (0.93). Fluoreszenzquantenausb. (CHCl₃, *λ*_{exc} = 557 nm, *E*_{562/1 cm} = 0.0131, Referenz: S-13 mit *Φ* = 1.00): 0.33. HRMS (ESI) (C₅₃H₆₉N₂O₄⁺): Ber. 797.5252, Gef. 797.5267, *Δ* = 1.5 mmu.

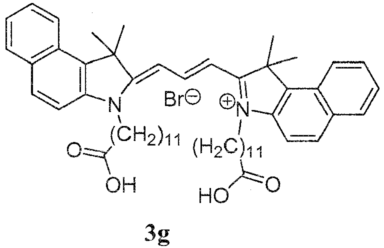

**3,3'-Di-(11-carboxyundecyl)-1,1,1',1'-tetramethyl-1*H-*dibenz[*e*]indocarbocyaninbromid** (**3g**): 3-(11-Carboxyundecyl)-1,1,2-trimethyl-1*H*-benz[*e*]indoleniumbromid (**2g**, 340mg, 7.00 µmol), 3-Picolin (1.00 mL) und Orthoameisensäuretriethylester (0.020 mg, 0.020 mL, 0.014 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt und aufgearbeitet (Chromatographie mit wässriger 2 N HCl statt Eisessig). Ausb. 12.0 mg (19%) goldglänzender Feststoff, der violette, rot fluoreszierende Lösungen bildet. *R_{f}* (RP 18, Methanol/H₂O/HCl 10:1:0.4) = 0.8. IR (ATR): *ṽ* = 3350 (w), 2922 (s), 2851 (m), 1710 (m), 1625 (w), 1587 (w), 1553 (s), 1519 (m), 1479 (m), 1422 (s), 1356 (m), 1278 (w), 1259 (w), 1224 (m), 1169 (m), 1141 (w), 1127 (m), 1012 (m), 973 (w), 931 (s), 896 (w), 806 (m), 786 (w), 746 (w), 726 (w), 685 (w), 675 cm⁻¹ (w). ¹H-NMR (400 MHz, CD₃OD): *δ* = 8.78 (t, 2 H, *H*_{Allyl}, ³*J_{E}* = 13.5 Hz), 8.29 (d, 2 H, *H*ₐᵣₒₘₐₜ, ³*J* = 8.5 Hz), 8.06 (d, 2 H, *H*ₐᵣₒₘₐₜ, ³*J* = 8.8 Hz), 8.03 (d, 2 H, *H*ₐᵣₒₘₐₜₒ ³*J* = 8.2 Hz), 7.68 (ddd, 3 H, *H*ₐᵣₒₘₐₜ, ⁴*J* = 1.2 Hz*,* ³*J* = 6.9 H_{z}, ³*J* = 8.4 Hz), 7.64 (d, 2 H, *Hₐᵣₒₘₐₜ,* ³*J* = 8.9 Hz), 7.53 (ddd, 2 H, *H*ₐᵣₒₘₐₜ, ⁴*J* = 0.9 Hz, ³*J* = 6.9 Hz, ³*J* = 8.0 Hz), 6.50 (d, 2 H, *H*_{Allyl}, ³*J_{E}* = 13.5 Hz), 4.29 (t, 4 H, 2 x NC*H*₂, ³*J* = 7.4 Hz), 2.18 (t, 4 H, 2 x CH₂CO₂H, ³*J* = 7.4 Hz), 2.11 (s, 12 H, 4 x C*H*₃), 1.96-1.88 (m, 4 H, 2 x C*H*₂), 1.57-1.49 (m, 8 H, 4 x C*H*₂), 1.47-1.40 (m, 4 H, 2 x C*H*₂), 1.36-1.28 ppm (m, 20 H, 10 x C*H*₂). ¹³C-NMR (100 MHz, CD₃OD): *δ* = 178.6, 175.9, 149.2, 139.4, 133.5, 132.2, 130.5, 129.7, 127.9, 127.5, 124.9, 121.9, 110.8, 101.7, 56.9, 51.0, 44.0, 29.1, 29.0, 29.0, 29.0, 28.9, 27.3, 26.6, 26.3, 25.4, 16.9 ppm. UV/VIS (EtOH): *λ*ₘₐₓ (*E*ᵣₑₗ): 590 (1.0), 553 nm (0.70). UV/VIS (Feststoff/Baumwolle): *λ*ₘₐₓ (*E*ᵣₑₗ): 600 (1.0), 555 nm (0.97). UV/VIS (Feststoff/Wolle): *λ*ₘₐₓ (*E*ᵣₑₗ): 603 (1.0), 556 nm (0.95). UV/VIS (Feststoff/Haare): *λ*ₘₐₓ (*E*ᵣₑₗ): 607 (1.0), 556 nm (0.93). Fluoreszenz (EtOH): *λ*ₘₐₓ (*I*ᵣₑₗ): 604 (1.0), 656 nm (0.20). Fluoreszenz (Feststoff/Baumwolle): *λ*ₘₐₓ (*I*ᵣₑₗ): 667 (1.0), 643 nm (0.94). Fluoreszenz (Feststoff/Wolle): *λ*ₘₐₓ (*I*ᵣₑₗ 638 (1.0), 667 nm (0.93). Fluoreszenz (Feststoff/Haare): *λ*ₘₐₓ (*I*ᵣₑₗ): 634 (1.0), 668 nm (0.88). Fluoreszenzquantenausb. (CHCl₃, *λ*ₘₐₓ = 560 nm, *E*_{562/1 cm} = 0.0127, Referenz: S-13 mit *Φ* = 1.00): 0.35. HRMS (ESI) (C₅₅H₇₃N₂O₄⁺): Ber. 825.5565, Gef. 825.5567, *Δ* = 0.2 mmu.

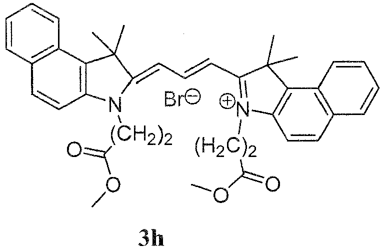

**3,3'-Di-(2-methoxycarbonylethyl)-1,1,1',1'-tetramethyt-1*H*-dibenz[*e*]indocarbocyanin**bromid (3h): 3-(2-Methoxycarbonylethyl)-1,1,2-trimethyl-1*H*-benz[*e*]indoleniumbromid (**2h**, 300 mg, 0.797 mmol), 3-Picolin (2.0 mL) und Orthoameisensäuretriethylester (0.26 mL, 1.60 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und ohne Chromatographie aufgearbeitet. Ausb. 290 mg (53 %) goldglänzender Feststoff, der violette, rot fluoreszierende Lösungen bildet. IR (ATR): *ṽ* = 2925 (s), 2856 (w), 2541 (w), 1960(w), 1722 (s), 1625 (m), 1567 (m), 1555 (s), 1519 (m), 1476 (m), 1422 (s), 1352 (m), 1278 (m), 1232 (w), 1155 (m), 1126 (w), 1011 (m), 924 (m, br), 875 (w), 804 (m), 786 (w), 804 (m), 786 (w), 744 cm⁻¹ (m). ¹H-NMR (200 MHz, CD₃OD) *δ* = 8.42 (t, 2 H, *H*_{Allyl}*,* ³*J_{E}* = 13.4 Hz), 8.11 (d, 4 H, *H*_{aromat,} *H*_{Allyl}*,* ³*J* = 8.0 Hz), 7.96 (d, 2 H, *H*_{aromat,} ³*J* = 8.5 Hz), 7.40-7.30 (m, 4 H, *H*ₐᵣₒₘₐₜ), 7.16 (t, 2 H, *H*ₐᵣₒₘₐₜ, ³*J* = 7.5 Hz), 6.38 (d, 2 H, *H*_{Allyl}, ³*J_{E}* = 13.4 Hz), 4.35-4.30 (m, 4 H, 2 x NC*H*₂), 3.31 (s, 6 H, 2 x OC*H*₃), 2.68 (t, 4 H, 2 x C*H*₂CO₂CH₃, ³*J* = 6.6 Hz), 1.71 ppm (s, 12 H, 4 x C*H*₃). ¹³C-NMR (100 MHz, CD₃OD): *δ* = 177.7, 172.5, 151.1, 140.5, 134.9, 133.7, 131.9, 131.3, 131.2, 129.3, 129.0, 126.5, 123.5, 112.3, 52.5, 41.5, 33.0, 28.2, 24.2 ppm. UV/VIS (EtOH): *λ*ₘₐₓ (*E*ᵣₑₗ): 592 (1.0), 553 nm (0.67). UV/VIS (Feststoff/Baumwolle): *λ*ₘₐₓ (*E*ᵣₑₗ): 600 (1.0), 554 nm (0.99). UV/VIS (Feststoff/Wolle): *λ*ₘₐₓ (*E*ᵣₑₗ): 597 (1.0), 560 nm (0.97). UV/VIS (Feststoff/Haare): *λ*ₘₐₓ (*E*ᵣₑₗ): 597 (1.0), 558 nm (0.98). Fluoreszenz (EtOH): *λ*ₘₐₓ (*I*ᵣₑₗ): 609 (1.0), 656 nm (0.53). Fluoreszenzquantenausb. (CHCl₃, *λ*ₘₐₓ = 562 nm, *E*_{562/1 cm} = 0.0178, Referenz: S-13 mit *Φ* = 1.00): 0.24. HRMS (ESI) (C₃₉H₄₁N₂O₄⁺): Ber. 601.3061, Gef. 601.3044. *Δ* = -1.7 mmu.

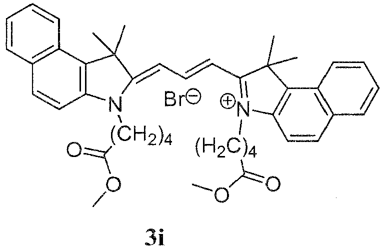

**3,3'-Di-(4-methorycarbonylbutyl)-1,1,1',1'-tetramethyl-1*H*-dibenz[*e*]indocarbocyanin**bromid (3i): 3-(4-Methoxycarbonylbutyl)-1,1,2-trimethyl-1*H-*benz[*e*]indoleniumbromid (2i, 253 mg, 0.626 mmol), 3-Picolin (2.2 mL) und Orthoameisensäuretriethylester (0.2 mL, 1.3 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und aufgearbeitet (Chromatographie mit wässriger 2 N HCl statt Eisessig). Ausb. 50 mg (11%) goldglänzender Feststoff, der violette, rot fluoreszierende Lösungen bildet. IR (ATR): *ṽ* = 3354 (w, br), 3140(w), 3030(w), 2960(w), 2924 (s), 2855 (w), 2167 (w), 1705 (m, br), 1623 (w), 1587 (w), 1554 (s), 1520 (m), 1479 (m), 1425 (s), 1353 (m), 1278 (w), 1260 (w), 1226 (m), 1153 (s), 1128 (m), 1066 (w), 1013 (m), 982 (w), 937 (m), 917 (w), 895 (w), 865 (w), 807 (m), 786 (w), 746 (w), 727 cm⁻¹ (w). ¹H-NMR (400 MHz, CD₃OD): *δ* = 8.79-8.76 (m, 1 H, *H*ₐᵣₒₘₐₜ), 8.30 (d, 2 H, *H*ₐᵣₒₘₐₜ, ³*J* = 8.4 Hz), 8.04 (dd, 4 H, *H*ₐᵣₒₘₐₜ, *H*_{Allyl}, ³*J* = 8.5 Hz, ³*J_{E}* = 14.9 Hz), 7.71-7.64 (m, 4 H, *H*ₐᵣₒₘₐₜ), 7.55-7.51 (m, 2 H, *H*ₐᵣₒₘₐₜ), 6.54 (d, 2 H, *H*_{Allyl}, ³*J_{E}* = 13.5 Hz), 4.31 (t, 4 H, 2 x NC*H*₂ ³*J* = 7.3 Hz), 2.48 (t, 4 H, 2 x CH₂CO₂CH₃, ³*J* = 7.0 Hz), 2.10 (s, 12 H, 4 x C*H*₃), 1.99-1.92 (m, 4 H, 2 x C*H*₂), 1.87-1.80 ppm (m, 4 H, 2 x C*H*₂). ¹³C-NMR (100 MHz, CD₃OD): *δ* = 175.9, 174.0, 149.3, 149.2, 139.4, 133.5, 132.2, 130.5, 129.7, 127.5, 125.0, 121.9, 110.8, 101.8, 51.0, 43.6, 32.7, 26.6, 21.7 ppm. UV/VIS (EtOH): *λ*ₘₐₓ (*E*ᵣₑₗ): 592 (1.0), 555 nm (0.72). UV/VIS (Feststoff/Baumwolle): *λ*ₘₐₓ (*E*ᵣₑₗ): 602(1.0), 555 nm (0.98). UV/VIS (Feststoff/Wolle): *λ*ₘₐₓ (*E*ᵣₑₗ): 557 (1.0), 556 nm (0.98). UV/VIS (Feststoff/Haare): *λ*ₘₐₓ (*E*ᵣₑₗ): 602 (1.0), 558 (0.92), 631 nm (0.89). Fluoreszenz (EtOH): *λ*ₘₐₓ (*I*ᵣₑₗ): 608 (1.0), 658 nm (0.49). Fluoreszenz (Feststoff/Baumwolle): *λ*ₘₐₓ (*I*ᵣₑₗ): 668 (1.0), 630 nm (0.55). Fluoreszenz (Feststoff/Wolle): *λ*ₘₐₓ (*I*ᵣₑₗ): 667 (1.0), 635 nm (0.98). Fluoreszenz (Feststoff/Haare): *λ*ₘₐₓ (*I*ᵣₑₗ): 667 (1.0), 623 nm (0.66). Fluoreszenzquantenausb. (CHCl₃, *λ*ₘₐₓ = 558 nm, E_{552/1 cm} = 0.0114, Referenz: S-13 mit *Φ* = 1.00): 0.40. HRMS (ESI) (C₄₃H₄₉N₂O₄⁺): Ber. 657.3687, Gef. 657.3675, *Δ* = -1.2 mmu.

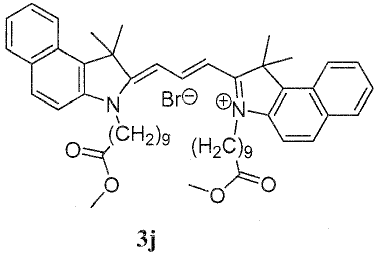

**3,3'-Di-(9-methoxycarbonylnonyl)-1,1,1',1'-tetramethyl-1*H*-dibenz[*e*]indocarbocyanin**bromid (3j): 3-(9-Methoxycarbonylnonyl)-1,1,2-trimethyl-1*H*-benz[*e*]indoleniumbromid (**2j**, 300 mg, 0.633 mmol), 3-Picolin (2.0 mL) und Orthoameisensäuretriethylester (0.20 mL, 1.26 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und ohne Chromatographie aufgearbeitet. Ausb. 113 mg (20%) goldglänzender Feststoff, der violette, rot fluoreszierende Lösungen bildet. IR (ATR): *ṽ* = 3413 (s, br), 3032 (m), 2971 (w), 2932 (m), 2855 (w), 2361 (s), 2339 (m), 2066 (w), 1729 (m), 1634 (s), 1591 (w), 1555 (m), 1505 (s), 1483 (w), 1429 (s), 1385 (w), 1357 (w), 1324 (w), 1246 (w), 1230 (w), 1204 (w), 1160 (s), 1096 (w), 1051 (w), 1014 (w), 981 (w), 934 (m), 893 (w), 810 (s), 751 (w), 728 (w), 685 cm⁻¹ (s). UV/VIS (EtOH): *λ*ₘₐₓ (*E*ᵣₑₗ): 591 (1.0), 551 nm (0.73). UV/VIS (Feststoff/Baumwolle): *λ*ₘₐₓ (*E*ᵣₑₗ): 600 (1.0), 559 nm (0.94). UV/VIS (Feststoff/Wolle): *λ*ₘₐₓ (*E*ᵣₑₗ): 599 (1.0), 559 nm (0.92). UV/VIS (Feststoff/Haare): *λ*ₘₐₓ (*E*ᵣₑₗ): 699 (1.0), 558 nm (0.95). Fluoreszenz (EtOH): *λ*ₘₐₓ (*I*ᵣₑₗ): 610 (1.0), 658 nm (0.51). Fluoreszenzquantenausb. (CHCl₃, *λ*_{exc} = 559 nm, *E*_{559/1 cm} = 0.0145, Referenz: S-13 mit *Φ* = 1.00): 0.47. HRMS (ESI) (C₅₃H₆₉N₂O₄⁺): Ber. 797.5252, Gcf. 797.5252, *Δ* = 0.03 mmu.

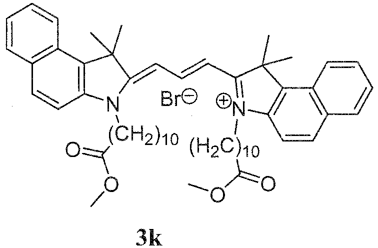

**3,3'-Di-(10-methorycarbonyldecyl)-1,1,1',1'-tetramethyl-1*H*-dibenz[*e*]indocarbocyanin-bromid (3k)**: 3-(10-Methoxycarbonyldecyl)-1,1,2-trimethyl-1*H*-benz[*e*]indoleniumbromid **(2k**, 300 mg, 0.633 mmol), Pyridin (1.0 mL) und Orthoameisensäuretriethylester (0.16 mL, 0.94 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und ohne Chromatographie aufgearbeitet. Ausb. 149 mg (35%) goldglänzender Feststoff, der violette, rot fluoreszierende Lösungen bildet. IR (ATR): *ṽ* = 3403 (s, br), 3131 (w), 3055 (w), 2972 (w), 2927 (s), 2853 (m), 2361 (w), 2337 (w), 2170 (w), 1731 (s), 1633 (m), 1586 (w), 1553 (s), 1519 (m), 1484 (m), 1425 (s), 1392 (w), 1356 (w), 1277 (w), 1227 (m), 1171 (s), 1143 (w), 1128 (w), 1050 (w), 1012 (m), 975 (w), 930 (m), 897 (w), 808 (w), 780 (w), 748 (w), 726 (w), 682 cm⁻¹ (w). UV/VIS (EtOH): *λ*ₘₐₓ (*E*ᵣₑₗ): 591 (1.0), 550 nm (0.69). UV/VIS (Feststoff/Baumwolle): *λ*ₘₐₓ (*E*ᵣₑₗ): 603(1.0), 559 nm (0.95). UV/VIS (Feststoff/Wolle): *λ*ₘₐₓ (*E*ᵣₑₗ): 596 (1.0), 557 nm (0.97). UV/VIS (Feststoff/Haare): *λ*ₘₐₓ (*E*ᵣₑₗ): 600 (1.0), 557 nm (0.95). Fluoreszenz (EtOH): *λ*ₘₐₓ (*I*ᵣₑₗ): 610 (1.0), 657 nm (0.53). Fluoreszenzquantenausb. (CHCl₃, *λ*_{exc} = 560 nm, *E*_{562/1 cm} = 0.0174, Referenz: S-13 mit *Φ* = 1.00): 0.36. HRMS (ESI) (C₅₅H₇₃N₂O₄⁺): Ber. 825.5565, Gef. 8525.5569, *Δ* = 0.4 mmu.

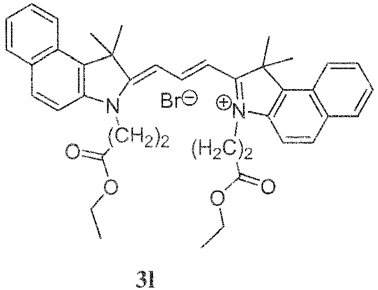

**3,3'-Di-(2-ethoxycarbonylethyl)-1,1,1',1'-tetramethyl-1*H*-dibenz[*e*]indocarbocyanin-bromid (31):** 3-(2-Ethoxycarbonylethyl)-1,1,2-trimethyl-1*H*-benz[*e*]indoleniumbromid (**21**, 200 mg, 0.51 mmol), 3-Picolin (1.1 mL) und Orthoameisensäuretriethylester (0.17 mL, 1.02 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (120°C) und aufgearbeitet (Chromatographie mit wässriger 2 N HCl statt Eisessig). Ausb. 121 mg (33 %) goldglänzender Feststoff, der violette, rot fluoreszierende Lösungen bildet. IR (ATR): *ṽ* = 3054 (w), 2973 (w), 2927 (w), 2574 (w), 2366 (w), 1723 (s), 1626 (w), 1587 (w), 1554 (s), 1520 (m), 1477 (m), 1425 (s), 1393 (w), 1353 (m), 1279 (w), 1227 (m), 1154 (m), 1128 (w), 1047 (w), 1012 (m), 987 (w), 925 (s), 877 (w), 806 (m), 786 (w), 746 (w), 728 (w), 684 cm⁻¹ (w). ¹H-NMR (400 MHz, CD₃OD): *δ* = 8.80-8.75 (m, 1 H, *H*ₐᵣₒₘₐₜ), 8.28 (d, 2 H, *H*ₐᵣₒₘₐₜ, ³*J* = 8.6 Hz), 8.03 (dd, 4 H, *H*ₐᵣₒₘₐₜ, *H*_{Allyl}, ³*J* = 8.6 Hz, ³*J_{E}* = 13.5 Hz), 7.70-7.65 (m, 4 H, *H*ₐᵣₒₘₐₜ), 7.54-7.49 (m, 2 H, *H*ₐᵣₒₘₐₜ), 6.54 (d, 2 H, *H*_{Allyl}, ³*J_{E}* = 13.4 Hz), 4.13 (t, 4 H, 2 x NC*H*₂, ³*J* = 6.8 Hz), 3.60 (q, 4 H, 2 x *CH*₂CH₃, ³*J* = 7.0 Hz), 2.73 (t, 4 H, 2 x C*H*₂CO₂CH₂, ³*J* = 6.9 Hz), 1.58 (s, 12 H, 4 x C*H*₃), 1.17 ppm (t, 6 H, 2 x CH₂C*H*₃, ³*J* = 7.1 Hz). ¹³C-NMR (100 MHz, CD₃OD): *δ* = 178.1, 172.9, 151.4, 140.8, 135.2, 134.0, 132.2, 131.5, 129.6, 129.3, 126.8, 123.1, 112.6, 111.6, 52.8, 41.7, 37.6, 33.2, 28.4, 24.5 ppm. UV/VIS (EtOH): *λ*ₘₐₓ (*E*ᵣₑₗ): 598 (1.0), 561 nm (0.69). UV/VIS (Feststoff/Baumwolle): *λ*ₘₐₓ (*E*ᵣₑₗ): 560 (1.0), 555 nm (0.99). UV/VIS (Feststoff/Wolle): *λ*ₘₐₓ (*E*ᵣₑₗ): 597 (1.0), 555 nm (0.98). UV/VIS (Feststoff/Haare): *λ*ₘₐₓ (*E*ᵣₑₗ): 560 (1.0), 554 nm (0.94). Fluoreszenz (EtOH): *λ*ₘₐₓ (*I*ᵣₑₗ): 608 (1.0), 659 nm (0.54). Fluoreszenz (FeststoffBaumwolle): *λ*ₘₐₓ (*I*ᵣₑₗ): 666 (1.0), 642 nm (0.93). Fluoreszenz (Feststoff/Wolle): *λ*ₘₐₓ (*I*ᵣₑₗ): 664 (1.0), 639 nm (0.98). Fluoreszenz (Feststoff/Haare): *λ*ₘₐₓ (*I*ᵣₑₗ): 636 (1.0), 664 nm (0.92). Fluoreszenzquantenausb. (CHCl₃, *λ*_{exc} = 561 nm, *E*_{561/1} cm = 0.0135, Referenz: S-13 mit *Φ* = 1.00): 0.33. HRMS (ESI): (C₄₁H₄₅N₂O₄⁺): Ber. 629.3374, Gef. 629.3385, *Δ* = 1.1 mmu.

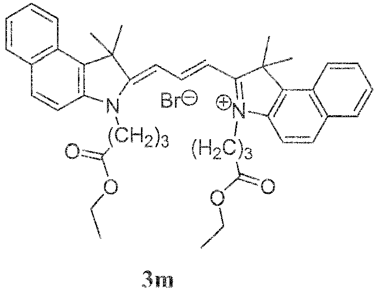

**3,3'-Di-(3-ethoxycarbonylpropyl)-1,1,1',1'-tetramethyl-1*H*-dibenz[*e*]indocarbocyanin-bromid (3m):** 3-(3-Ethoxycarbonylpropyl)-1,1,2-trimethyl-1*H*-benz[*e*]indoleniumbromid (**2m**, 250 mg, 0.620 mmol), 3-Picolin (1.0 mL) und Orthoameisensäuretriethylester (0.200 mL, 1.24 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und aufgearbeitet (Chromatographie mit wässriger 2 N HCl statt Eisessig). Ausb. 215 mg (47%) goldglänzender Feststoff, der violette, rot fluoreszierende Lösungen bildet. IR (ATR): *ṽ* = 3391 (w), 2969 (w), 2921 (m), 1726 (s), 1622 (m), 1589 (w), 1557 (s), 1520 (m), 1477 (m), 1427 (s), 1352 (m), 1227 (m), 1154 (s), 1123 (w), 1067 (w), 1014 (m), 941 (s), 899 (w), 805 (m), 791 (w), 757 (m), 728 (w), 675 (w), 652 (m), 638 cm⁻¹ (w). ¹H-NMR (400 MHz, CD₃OD): *δ*= 8.81-8.73 (m, 1 H, *H*ₐᵣₒₘₐₜ), 8.28 (d, 2 H, *H*ₐᵣₒₘₐₜ, ³*J* = 8.4 Hz), 8.01 (dd, 4 H, *H*ₐᵣₒₘₐₜ, *H*_{Allyl}, ³*J =* 8.3 Hz, ³*J_{E}* = 14.5 Hz), 7.71-7.62 (m, 4 H, *H*ₐᵣₒₘₐₜ), 7.52-7.47 (m, 2 H, *H*ₐᵣₒₘₐₜ), 6.54 (d, 2 H, *H*_{Allyl}, ³*J_{E}* = 13.8 Hz), 4.34-4.28 (m, 4 H, 2 x NC*H*₂), 4.11 (q, 4 H, 2 x C*H*₂CH₃, ³*J* = 7.1 Hz), 2.62-2.57 (m, 4 H, 2 x C*H*₂CO₂CH₂), 2.20-2.13 (m, 4 H, 2 x C*H*₂), 2.07 (s, 12 H, 4 x C*H*₃), 1.20 ppm (t, 6 H, 2 x CH₂C*H*₃, ³*J* = 7.1 Hz). ¹³C-NMR (100 MHz, CD₃OD): *δ* = 176.1,173.0, 149.5, 139.3, 133.5, 132.2, 130.6, 129.8, 127.9, 125.0, 121.9, 110.7, 101.8, 60.4, 51.0, 46.9, 43.2, 30.2, 26.6, 22.8, 13.1 ppm. UV/VIS (EtOH): *λ*ₘₐₓ (*E*ᵣₑₗ): 598 (1.0), 559 nm (0.63). UV/VIS (Feststoff/Baumwolle): *λ*ₘₐₓ (*E*ᵣₑₗ): 600 (1.0), 555 nm (0.99). UV/VIS (Feststoff/Wolle): *λ*ₘₐₓ (*E*ᵣₑₗ): 559 (1.0), 594 nm (0.98). UV/VIS (Feststoff/Haare): *λ*ₘₐₓ (*E*ᵣₑₗ): 597 (1.0), 554 nm (0.96). Fluoreszenz (EtOH): *λ*ₘₐₓ (*I*ᵣₑₗ): 610 (1.0), 657 nm (0.56). Fluoreszenz (Feststoff/Baumwolle): *λ*ₘₐₓ (*I*ᵣₑₗ): 667 (1.0), 642 nm (0.88). Fluoreszenz (Feststoff/Wolle): *λ*ₘₐₓ (*I*ᵣₑₗ): 635 (1.0), 665 nm (0.97). Fluoreszenz (Feststoff/Haare): *λ*ₘₐₓ (*I*ᵣₑₗ): 665 (1.0), 639 nm (0.97). Fluoreszenzquantenausb. (CHCl₃, *λ*_{exc} = 560 nm, *E*_{562/1} cm = 0.0132, Referenz: S-13 mit *Φ* = 1.00): 0.61. HRMS (ESI) (C₄₃H₄₉N₂O₄⁺): Ber. 657.3687, Gef. 657.3695, *Δ* = 0.8 mmu.

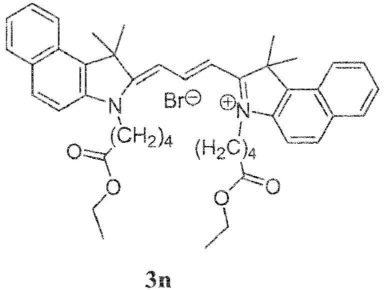

**3,3'-Di-(4-Ethoxycarbonylbutyl)-1,1,1',1'-tetramethyl-1*H*-dibenz[*e*]indocarbocyanin-bromid (3n):** 3-(4-Ethoxycarbonylbutyl)-1,1,2-trimethyl-1*H*-benz[*e*]indoleniumbromid (**2n**, 250 mg, 0.6 mmol), 3-Picolin (1.1 mL) und Orthoameisensäuretriethylester (0.2 mL, 1.2 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und aufgearbeitet (Chromatographie mit wässriger 2 N HCl statt Eisessig). Ausb. 105 mg (23%) goldglänzender Feststoff, der violette, rot fluoreszierende Lösungen bildet. IR (ATR): *ṽ* = 3420 (w), 2921 (s), 2851 (m), 1726 (s), 1622 (m), 1589 (w), 1555 (s), 1520 (s), 1477 (m), 1428 (s), 1352 (m), 1227 (m), 1154 (s), 1123 (w), 1067 (w), 1014 (m), 941 (m), 899 (w), 805 (m), 785 (w), 744 (m), 728 (w), 685 (w), 652 cm⁻¹ (m). ¹H-NMR (400 MHz, CD₃OD): *δ* = 8.76 (t, 1 H, *H*ₐᵣₒₘₐₜ, ³*J* = 13.1 Hz), 8.28 (d, 2 H, *H*ₐᵣₒₘₐₜ, ³*J* = 8.3 Hz), 8.03 (dd, 4 H, *H*ₐᵣₒₘₐₜ, *H*_{Allyl}, *³J* = 8.6 Hz, ³*J_{E}* = 12.3 Hz), 7.75-7.60 (m, 4 H, *H*ₐᵣₒₘₐₜ), 7.55-7.49 (m, 2 H, *H*ₐᵣₒₘₐₜ), 6.51 (d, 2 H, H_{Allyl}, ³*J_{E}* = 13.0 Hz), 4.35-4.28 (m, 4 H, 2 x NC*H*₂), 4.10 (q, 4 H, 2 x C*H*₂CH₃, ³*J* = 7.1 Hz), 2.45 (t, 4 H, 2 x C*H*₂CO₂CH₂, ³*J* = 6.5 Hz), 2.09 (s, 12 H, 4 x C*H*₃), 2.00-1.92 (m, 4 H, 2 x C*H*₂), 1.20 ppm (t, 6 H, 2 x CH₂C*H*₃, ³*J* = 7.1 Hz). ¹³C-NMR (100 MHz, CD₃OD): *δ* = 177.5, 174.9, 169.4, 150.8, 140.8, 133.7, 132.0, 131.2, 129.3, 129.0, 126.4, 123.4, 112.2, 103.2, 79.5, 69.2, 61.6, 52.5, 34.5, 28.1, 23.2, 14.5 ppm. UV/VIS (EtOH): *λ*ₘₐₓ (*E*ᵣₑₗ): 598 (1.0), 558 nm (0.64). UV/VIS (Feststoff/Baumwolle): *λ*ₘₐₓ (*E*ᵣₑₗ): 560 (1.0), 556 nm (0.97). UV/VIS (Feststoff/Wolle): *λ*ₘₐₓ (*E*ᵣₑₗ): 598 (1.0), 557 nm (0.98). UV/VIS (Feststoff/Haare): *λ*ₘₐₓ (*E*ᵣₑₗ): 598 (1.0), 555 nm (0.94). Fluoreszenz (EtOH): *λ*ₘₐₓ (*I*ᵣₑₗ): 610 (1.0), 660 nm (0.52). Fluoreszenz (Feststoff/Baumwolle): *λ*ₘₐₓ (*I*ᵣₑₗ): 670 (1.0), 643 nm (0.95). Fluoreszenz (Feststoff/Wolle): *λ*ₘₐₓ (*I*ᵣₑₗ): 631 (1.0), 664 nm (0.95). Fluoreszenz (Feststoff/Haare): *λ*ₘₐₓ (*I*ᵣₑₗ): 625 (1.0), 664 nm (0.96). Fluoreszenzquantenausb. (CHCl₃, *λ*exc = 559 nm, *E*_{562/1 cm} = 0.0128, Referenz: S-13 mit *Φ* = 1.00): 0.43. HRMS (ESI) (C₄₅H₅₃N₂O₄⁺): Ber. 685.4000, Gef. 685.4010, *Δ* = 1.0 mmu.

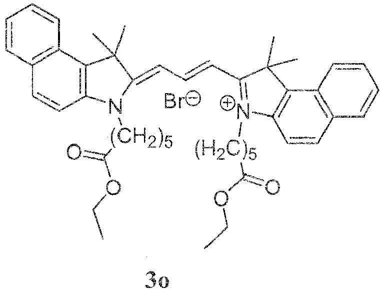

**3,3'-Di-(5-ethoxycarbonylpentyl)-1,1,1',1'-tetramethyl-1*H*-dibenz[*e*]indocarbocyanin-bromid (3o):** 3-(5-Ethoxycarbonylpentyl)-1,1,2-trimethyl-1*H*-benz[*e*]indoleniumbromid (**2o**, 400 mg, 0.93 mmol), Pyridin (1.5 mL) und Orthoameisensäuretriethylester (0.30 mL, 1.85 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und aufgearbeitet. Auf eine Reinigung durch Flashchromatographie wurde hier verzichtet, da sich dabei das Produkt in die entsprechende Säure zersetzt. Ausb. 363 mg (50%) goldglänzender Feststoff, der violette, rot fluoreszierende Lösungen bildet. IR (ATR): *ṽ* = 3312 (w), 2930 (m), 2859 (m), 2361 (w), 2338 (w), 1724 (s), 1626 (w), 1587 (w), 1553 (s), 1520 (m), 1480 (m), 1423 (s), 1356 (m), 1278 (w), 1225 (m), 1169 (w), 1143 (w), 1126 (w), 1071 (w), 1012 (m), 972 (w), 927 (m), 898 (w), 877 (w), 806 (m), 747 (w), 726 (w), 685 cm⁻¹ (w). ¹H-NMR (200 MHz, CD₃OD): *δ* = 8.78 (t, 1 H, *H*ₐᵣₒₘₐₜ*, ³J* = 13.6 Hz), 8.31 (d, 2 H, *H*ₐᵣₒₘₐₜ*,* ³*J* = 7.8 Hz), 8.10-8.00 (m, 4 H, *H*_{aromat,} *H*_{Allyl}), 7.75-7.63 (m, 4 H, *H*ₐᵣₒₘₐₜ), 7.57-7.49 (m, 2 H, *H*ₐᵣₒₘₐₜ), 6.55 (d, 2 H, *H*_{Allyl}, ³*J_{E}* = 13.4 Hz), 4.30 (t, 4 H, 2 x NC*H*₂, ³*J* = 7.5 Hz), 4.07 (q, 4 H, 2 x C*H*₂CH₃, ³*J* = 7.1 Hz), 2.37 (t, 4 H, 2 x C*H*₂CO₂CH₂, ³*J* = 7.0 Hz), 2.11 (s, 12 H, 4 x C*H*₃), 2.00-1.81 (m, 4 H, 2 x C*H*₂), 1.79-1.55 (m, 8 H, 4 x C*H*₂), 1.18 ppm (t, 6 H, 2 x CH₂C*H*₃, ³*J* = 7.1 Hz). ¹³C-NMR (100 MHz, CD₃OD): *δ* = 177.4, 175.3, 150.8, 145.7, 140.9, 135.0, 133.7, 132.0, 131.2, 129.6, 126.4, 123.4, 112.3, 103.3, 61.5, 52.5, 45.9, 34.9, 28.6, 28.1, 27.3, 25.8, 16.8, 14.8 ppm. UV/VIS (EtOH): *λ*ₘₐₓ (*E*ᵣₑₗ): 591 (1.0), 554 nm (0.73). UV/VIS (Feststoff/Baumwolle): *λ*ₘₐₓ (*E*ᵣₑₗ): 598 (1.0), 554 nm (0.97). UV/VIS (Feststoff/Wolle): *λ*ₘₐₓ (*E*ᵣₑₗ): 599 (1.0), 554 nm (0.99). UV/VIS (Feststoff/Haare): *λ*ₘₐₓ (*E*ᵣₑₗ): 600 (1.0), 554 nm (0.95). Fluoreszenz (EtOH): *λ*ₘₐₓ (*I*ᵣₑₗ): 615 (1.0), 655 nm (0.57). Fluoreszenz (Feststoff/Baumwolle): *λ*ₘₐₓ (*I*ᵣₑₗ): 666 (1.0), 642 nm (0.93). Fluoreszenz (Feststoff/Wolle): *λ*ₘₐₓ (*I*ᵣₑₗ): 664 (1.0), 638 nm (0.98). Fluoreszenz (Feststoff/Haare): *λ*ₘₐₓ (*I*ᵣₑₗ): 636 (1.0), 665 nm (0.92). Fluoreszenzquantenausb. (CHCl₃, *λ*_{exc} = 558 nm, *E*_{558/1 cm} = 0.0138, Referenz: S-13 mit *Φ* = 1.00): 0.36. HRMS (ESI) (C₄₇H₅₇N₂O₄⁺): Ber. 713.4313, Gef. 713.4331, *Δ* = 1.8 mmu.

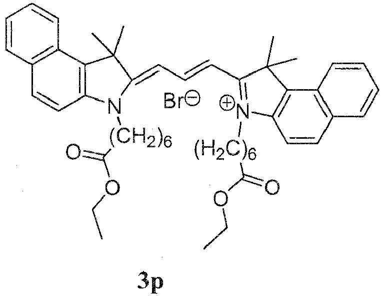

**3,3'-Di-(6-ethozycarbonylhexyl)-1,1,1',1'-tetramethyl-1*H*-dibenz[*e*]indocarbocyanin-bromid (3p):** 3-(6-Ethoxycarbonylhexyl)-1,1,2-trimethyl-1*H*-benz[*e*]indoleniumbromid (**2p**, 95.0 mg, 0.213 mmol), 3-Picolin (1.00 mL) und Orthoameisensäuretriethylester (0.070 mL, 0.426 mmol) wurden wie bei 3,3'-Di-(2-carboxyethyl)-1,1,1',1'-tetramethyl-1*H-*dibenz[*e*]indocarbocyaninbromid (**3a**) beschrieben umgesetzt (100°C) und aufgearbeitet. Auf eine Reinigung durch Flashchromatographie wurde hier verzichtet, da sich dabei das Produkt in die entsprechende Säure zersetzt. Ausb. 49 mg (28%). IR (ATR): *ṽ* = 3064 (w), 2936 (s), 2864 (w), 2540 (w), 2361 (s), 2338 (s), 2162 (w), 1718 (s, br), 1654 (w), 1636 (w), 1558 (m), 1522 (m), 1507 (w), 1431 (s), 1362 (w), 1226 (w), 1174 (m), 1155 (w), 1017 (w), 938 (m), 814 (w), 750 (w), 668 cm⁻¹ (m). UV/VIS (EtOH): *λ*ₘₐₓ (*E*ᵣₑₗ): 588 (1.0), 553 nm (0.76). Fluoreszenz (EtOH): *λ*ₘₐₓ (*I*ᵣₑₗ): 608 (1.0), 655 nm (0.51). Fluoreszenzquantenausb. (CHCl₃, *λ*_{exc} = 561 nm, *E*_{502/1 cm} = 0.0154, Referenz: S-13 mit *Φ* = 1.00): 0.35. HRMS (ESI) (C₄₉H₆₁N₂O₄⁺): Ber. 741.4626, Gef. 741.4613, *Δ* = 1.3 mmu.

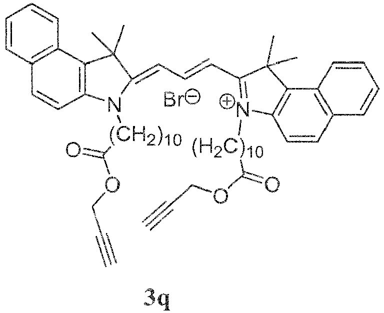

**3,3'-Di-(10-propargyloaycarbonyldecyl)-1,1,1',1'-tetramethyl-1*H*-dibenz[*e*]indo-carbocyaninbromid (3q):** 3-(10-Propargyloxycarbonyldecyl)-1,1,2-trimethyl-1*H-*benz[*e*]indoleniumbromid (**2q**) (900 mg, 1.76 mmol), Pyridin (2.0 mL) und Orthoameisensäuretriethylester (0.6 mL, 3.51 mmol) wurden wie bei 3,3'-Di-(2-carboxyethyl)-1,1,1',1'-tetramethyl-1*H*-dibenz[*e*]indocarbocyaninbromid (**3a**) beschrieben umgesetzt (100°C) und aufgearbeitet. Auf eine Reinigung durch Flashchromatographie wurde hier verzichtet, da sich das Produkt dabei zersetzt. Ausb. 621 mg (37%) goldglänzender Feststoff, der violette, rot fluoreszierende Lösungen bildet. UV/VIS (EtOH): *λ*ₘₐₓ (*E*ᵣₑₗ): 591 (1.0), 550 nm (0.77). Fluoreszenz (EtOH): *λ*ₘₐₓ (*I*ᵣₑₗ): 609 (1.0), 655 nm (0.50). HRMS (ESI) (C₅₉H₇₃N₂O₄⁺): Ber. 873.5565, Gef. 873.5571, *Δ* = 0.6 mmu.

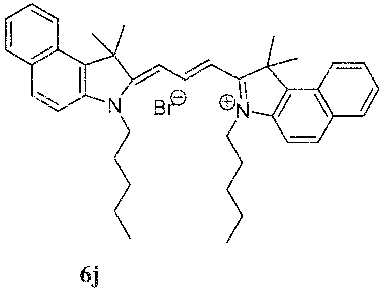

**3,3'-Di-(pentyl)-1,1,1',1'-tetramethyl-1*H*-dibenz[*e*]indocarbocyaninbromid (6j; Referenzverbindung):** 1,1,2-Trimethyl-3-pentyl-1*H*-benz[*e*]indoleniumbromid (100 mg, 0.277 mmol) wurde unter N₂-Schutzatmosphäre in Pyridin (1.0 mL) gelöst, auf 100 °C erhitzt, langsam tropfenweise mit Orthoameisensäuretriethylester (0.1 mL, 0.5 mmol) versetzt, 2 h auf 100 °C erhitzt, abkühlen lassen, mit Diethylether (20 mL) versetzt und abgesaugt. Ausb. 71 mg (79%), goldglänzender Feststoff, der violette Lösungen bildet, die stark rot fluoreszieren, Schmp. > 150 °C Zers. IR(ATR): *ṽ* = 3393 (s), 3133 (w), 3060 (m), 2979 (w), 2941 (w), 2871 (w), 2054 (w), 1634 (s), 1584 (w), 1555 (m), 1519 (w), 1487 (s), 1466 (w), 1446 (w), 1428 (w), 1388 (w), 1356 (w), 1318 (w), 1227 (w), 1173 (s), 1127 (w), 1060 (w), 1029 (w), 973 (w), 937 (w), 880 (w), 807 (w), 778 (m), 680 cm⁻¹ (s). ¹H-NMR (200 MHz, CD₃OD): *δ* = 8.79 (t, 1H, *H*_{dien}, ³*J_{E}* = 13.5 Hz), 8.30 (d, 2H, *H*ₐᵣₒₘₐₜ*,* ³*J* = 8.5 Hz), 8.07-8.02 (m, 4H, *H*ₐᵣₒₘₐₜ), 7.71-7.67 (m, 2H, *H*ₐᵣₒₘₐₜ), 7.64 (d, 2H, *H*ₐᵣₒₘₐₜ ³*J* = 8.8 Hz)7.55-7.51 (m, 2H, *H*ₐᵣₒₘₐₜ), 6.54 (d, 2H, *H*_{dien}, ³*J_{E}* = 13.5 Hz), 4.29 (t, 4H, 2 x NC*H*₂,³*J* = 7.5 Hz), 2.11 (s, 12H, 4 x C*H*₃), 1.96-1.89 (m, 4H, 2 x C*H*₂), 1.55-1.44 (m, 8H, 4 x C*H*₂), 0.97 ppm (t, 6H, 2 x C*H*₃, ³*J* = 7.2 Hz). ¹³C-NMR (100 MHz, CD₃OD): *δ* = 177.8, 147.2, 146.1, 141.3, 134.1, 132.4, 131.6, 130.0, 126.8, 112.6, 103.5, 59.0, 52.8, 45.9, 30.5, 29.0, 28.5, 24.0, 17.1, 14.7 ppm. UV/Vis (EtOH): *λ*ₘₐₓ (*E*ᵣₑₗ): 590 (1.0), 552 nm (0.70). UV/Vis (Feststoff/Cellulose): *λ*ₘₐₓ (*E*ᵣₑₗ): 600 (1.0), 553 nm (0.98). UV/Vis (Feststoff/Wolle): *λ*ₘₐₓ (*E*ᵣₑₗ): 539 (1.0), 600 nm (0.93). Fluoreszenz (EtOH): *λ*ₘₐₓ (*I*ᵣₑₗ): 607 (1.0), 657 nm (0.50). Fluoreszenz (Feststoff/Cellulose): *λ*ₘₐₓ (*I*ᵣₑₗ): 671 (1.0). Fluoreszenz (Feststoff/Wolle): *λ*ₘₐₓ (*I*ᵣₑₗ): 623 (1.0), 662 nm (0.79). Fluoreszenzquantenausbeute (CHCl₃, *λ*_{exc} = 553 nm, *E*_{553 nm/1 cm} = 0.0133; Referenz: **S-13** mit *Φ* = 1.00): 0.20. HRMS (ESI) (C₄₁H₄₉N₂⁺): Ber. 569.3890, Gef. 569.3884, *Δ* = -0.6 mmu.

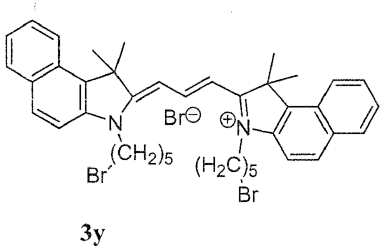

### 3,3'-Di-(5-brompentyl)-1,1,1',1'-tetramethyl-1H-dibenz[e]indocarbocyaninbromid (3y):

3-(5-Brompentyl)-1,1,2-trimethyl-1*H*-benz[*e*]indoleniumbromid (400 mg, 0.911 mmol), Pyridin (2.0 mL) und Orthoameisensäuretriethylester (0.300 mL, 1.82 mmol) wurden analog zu 3,3'-Di-(pentyl)-1,1,1',1'-tetramethyl-1*H*-dibenz[e]indocarbocyaninbromid (**6j**) umgesetzt (2.5 h bei 120°C) und aufgearbeitet. Eine Flashchromatographie (RP 18, Methanol/H₂O/l M HCl 10 :1:0.4) führt zur Zersetzung des Farbstoffs. Er wird daher ohne chromatographische Reinigung verwendet. Schmp. > 100 °C. IR(ATR): *ṽ* = 3408 (m), 2970 (s), 2927 (m), 2865 (m), 2362 (w), 1728 (w), 1627 (w), 1588 (w), 1558 (m), 1520 (w), 1430 (m), 1372 (m), 1296 (w), 1278 (w), 1249 (w), 1226 (w), 1191 (w), 1100 (s), 1013 (m), 936 (m), 863 (w), 809 (w), 787 (w), 748 (w), 728(w), 701 (w), 653 cm⁻¹ (w). ¹H-NMR (200 MHz, CD₃OD): *δ* = 8.82 (t, 1H, *H*_{dien}, ³*J_{E}* = 13.7 Hz), 8.27-8.26 (m, 2H, *H*ₐᵣₒₘₐₜ), 8.16-8.09 (m, 4H, *H*ₐᵣₒₘₐₜ), 7.71-7.64 (m, 4H, *H*ₐᵣₒₘₐₜ), 7.56-7.51 (m, 2H, *H*ₐᵣₒₘₐₜ), 7.08 (d, 2H, *H*_{dien,} ³*J_{E}* = 13.8 Hz), 4.34-4.33 (m, 8H, 2 x NC*H*₂, 2 x C*H*₂Br), 2.11 (s, 12H, 4 x C*H*₃), 2.05-1.94 ppm (m, 12H, 6 x C*H*₂). UV/Vis (EtOH): *λ*ₘₐₓ (*E*ᵣₑₗ): 592 (1.0), 553 nm (0.86). UV/Vis (Feststoff/Cellulose): *λ*ₘₐₓ (*E*ᵣₑₗ): 558 (1.0), 597 nm (0.98). UV/Vis (Feststoff/Wolle): *λ*ₘₐₓ (*E*ᵣₑₗ): 556 (1.0), 598 nm (0.97). Fluoreszenz (EtOH): *λ*ₘₐₓ (*I*ᵣₑₗ): 612 (1.0), 662 nm (0.61). Fluoreszenz (Feststoff/Cellulose): *λ*ₘₐₓ (*I*ᵣₑₗ): 678 (1.0), 632 nm (0.51). Fluoreszenz (Feststoff/Wolle): *λ*ₘₐₓ (*I*ᵣₑₗ): 678 (1.0), 628 nm (0.46). Fluoreszenzquantenausbeute (CHCl₃, *λ*_{exc} = 549 nm, *E*_{549 nm/1 cm} = 0.0254; Referenz S-13 mit *Φ* = 1.00): 0.15. HRMS (ESI): (M⁺ -C₃₆H₃₇N₂⁺) Ber. 497.2951, Gef. 497.2951, *Δ* = 0 mmu.

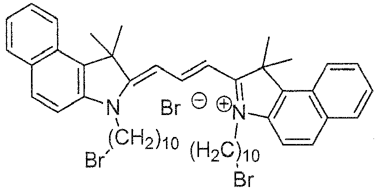

### 3,3'-Di-(5-bromdecyl)-1,1,1',1'-tetramethyl-1H-dibenz[e]indocarbocyaninbromid (3x):

3-(10-Bromdecyl)-1,1,2-trimethyl-1*H*-benz[*e*]indoleniumbromid (250 mg, 0.491 mmol), Pyridin (2.0 mL) und Orthoameisensäuretriethylester (0.16 mL, 0.98 mmol) wurden analog zu 3,3'-Di-(pentyl)-1,1,1',1'-tetramethyl-1*H*-dibenz[*e*]indocarbocyaninbromid (**6j**) umgesetzt (2 h bei 120°C) und aufgearbeitet und durch Flashchromatographie gereinigt (RP 18, Methanol/H₂O/1 M HCl 1:1:0.4 zum Auftragen, Methanol/H₂O/1 M HCl 2:1:0.4 zum Entfernen von Nebenprodukten und Methanol/H₂O/1 M HCl 10:1:0.4 zur Elution des Farbstoffs). Ausb. 170 mg (73%), goldglänzender Feststoff, der violette, stark rot fluoreszierende Lösungen bildet, Schmp. 113°C. IR(ATR): *ṽ* = 3387 (m), 3058 (w), 2919 (s), 2851 (s), 1722 (m), 1627 (w), 1588 (w), 1555 (m), 1520 (m), 1479 (w), 1468 (w), 1427 (s), 1358 (m), 1226 (m), 1171 (w), 1144 (w), 1111(m), 1013 (w), 933 (m), 898 (w), 808 (m), 787 (w), 748 (w), 727 (w), 700(w), 686 (w), 653 cm⁻¹ (w). ¹H-NMR: starke Aggregation in konzentrierter Lösung, daher stark berbreiterte Signale. UV/Vis (EtOH): *λ*ₘₐₓ (*E*ᵣₑₗ): 593 (1.0), 553 nm (0.73). UV/Vis (Feststoff/Cellulose): *λ*ₘₐₓ (*E*ᵣₑₗ): 601 (1.0), 557 nm (0.96). UV/Vis (Feststoff/Wolle): *λ*ₘₐₓ (*E*ᵣₑₗ): 602 (1.0), 570 nm (0.97). Fluoreszenz (EtOH): *λ*ₘₐₓ (*I*ᵣₑₗ): 608 (1.0), 658 nm (0.53). Fluoreszenz (Feststoff/Cellulose): *λ*ₘₐₓ (*I*ᵣₑₗ): 672 (1.0), 650 nm (0.89). Fluoreszenz (Feststoff/Wolle): *λ*ₘₐₓ (*I*ᵣₑₗ): 671 (1.0), 651 nm (0.87). Fluoreszenzquantenausbeute (CHCl₃, *λ*exc = 558 nm, *E*_{558 nm/l cm} = 0.0125; Referenz S-13 mit *Φ* = 1.00): 0.10. HRMS (ESI): (M⁺ -C₄₁H₄₇N₂⁺): Ber. 567.3734, Gef. 567.3736, *Δ* = 0.2 mmu.

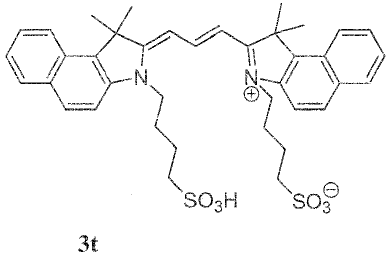

**3,3'-Di-(4-sulfobutyl)-1,1,1',1'-tetramethyl-1*H*-dibenz[*e*]indocarbocyanin (3t):** 3-(4-Sulfobutyl)-1,1,2-trimethyl-1*H*-benz[*e*]indol (500 mg, 1.40 mmol) wurde unter N₂ Schutzatmosphäre in Pyridin (2.0 mL) gelöst, auf 116 °C erhitzt. Langsam tropfenweise mit Orthoameisensäuretriethylester (0.50 mL, 2.9 mmol) versetzt (blau-violett Färbung), 2 h unter Rückfluss erhitzt, abkühlen lassen, mit Diethylether gefällt, durch Abdekantieren vom Lösungsmittel befreit, im Vakuum getrocknet und durch Flashchromatographie gereinigt (RP 18, Methanol/H₂O/1 M HCl 1:1:0.4 zum Auftragen, Methanol/H₂O/1 M HCl 2:1:0.4 zum Entfernen von Nebenprodukten und Methanol/H₂O/1 M HCl 10:1:0.4 zur Elution des Farbstoffs). Ausb. 430 mg (88%) goldglänzender Feststoff, der violette, stark rot fluoreszierende Lösungen bildet, Schmp. >250°C. IR (ATR): *ṽ* = 3454 (w), 3054 (m), 2973 (w), 2933 (w), 2860 (w), 1626 (w), 1588 (w), 1555 (s), 1519 (m), 1490 (s), 1426 (s), 1369 (m), 1348 (w), 1124 (w), 1030 (m), 1012 (m), 977 (w), 948 (m), 925 (m), 891 (w), 805 (w), 767 (w), 740 (w), 728 (w), 688 (w), 650 cm⁻¹ (w). ¹H-NMR (200 MHz, CD₃OD): *δ* = 8.74 (t, 1H, *H*_{dien}, ³*J_{E}* = 13.5 Hz), 8.28 (d, 2H, *H*ₐᵣₒₘₐₜ, ³*J* = 8.2 Hz), 8.02 (t, 4H, *H*ₐᵣₒₘₐₜ, ³*J* = 7.5 Hz), 7.67 (t, 4H, *H*ₐᵣₒₘₐₜ, ³*J* = 8.5 Hz), 7.50 (t, 2H, *H*ₐᵣₒₘₐₜ, ³*J* = 7.5 Hz), 6.59 (d, 2H, *H*_{dien}, ³*J_{E} =* 13.7 Hz), 4.42-4.26 (m, 4H, 2 x NC*H*₂), 3.05-2.87 (m, 4H, 2 x C*H*₂SO₃H), 2.02-2.19 ppm (m, 20H, 4 x C*H*₃, 4 x C*H*₂). ¹³C-NMR (100 MHz, CD₃OD): *δ* = 176.8, 149.3, 139.5, 133.4, 132.2, 130.5, 129.7, 127.9, 127.4, 124.8, 121.9, 110.9, 56.9, 51.0, 43.8, 26.6, 26.2, 22.8, 22.2, 16.9 ppm. UV/Vis (EtOH): *λ*ₘₐₓ (*E*ᵣₑₗ): 591 (1.0), 553 nm (0.68). UV/Vis (Feststoff/Cellulose): *λ*ₘₐₓ (*E*ᵣₑₗ): 593 (1.0), 559 nm (0.99). UV/Vis (Feststoff/Haare): *λ*ₘₐₓ (*E*ᵣₑₗ): 553 (1.0), 593 nm (0.90). Fluoreszenz (EtOH): *λ*ₘₐₓ (*I*ᵣₑₗ): 620 (1.0), 660 nm (0.50). Fluoreszenz (Feststoff/Cellulose): *λ*ₘₐₓ (*I*ᵣₑₗ): 662 (1.0), 630 nm (0.96). Fluoreszenz (Feststoff/Wolle): *λ*ₘₐₓ (*I*ᵣₑₗ): 667 (1.0), 640 nm (0.98). Fluoreszenz (Feststoff/Haare): *λ*ₘₐₓ (*I*ᵣₑₗ): 621 (1.0), 661 nm (0.67). Fluoreszenzquantenausbeute (CHCl₃, *λ*_{exc} = 559 nm, *E*_{559 nm/l} cm = 0.0124; Referenz: S-13 mit *Φ* = 1.00): 0.32. HRMS (ESI) (C₃₉H₄₅N₂O₆S₂): Ber. 701.2719, Gef. 701.2726 *Δ* - 0.7 mmu.

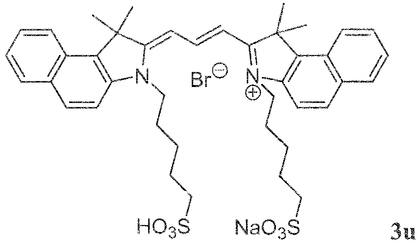

### 3,3'-Di-(5-sulfopentyl)-1,1,1',1'-tetramethyl-1H-dibenz[e]indocarbocyaninbromid (3u):

3-(5-Sulfopentyl)-1,1,2-trimethyl-1*H*-benz[*e*]indoleniumbromid-Natriumsalz (122 mg, 0.264 mmol), Pyridin (1.3 mL), Methanol (1 mL) und Orthoameisensäuretriethylester (0.080 mL, 0.52 mmol) wurden analog zu 3,3'-Di-(pentyl)-1,1,1',1'-tetramethyl-1*H-*dibenz[*e*]indocarbocyaninbromid (**6j**) umgesetzt (2 h bei 100°C) und aufgearbeitet, mehrfach in wenig Ethanol gelöst, mit Diethylether gefällt und abdekantiert und durch Flashchromatographie gereinigt (RP 18, Methanol/H₂O/1 M HCl 1:1:0.4 zum Auftragen, Methanol/H₂O/1 M HCl 2:1:0.4 zum Entfernen von Nebenprodukten und Methanol/H₂O/1 M HCl 10:1:0.4 zur Elution des Farbstoffs). Ausb. 56.0 mg (51%) goldglänzender Feststoff, der violette, stark rot fluoreszierende Lösungen bildet. IR (ATR): *ṽ* = 3376 (w), 3058 (w), 2969 (w), 2931 (m), 2860 (w), 1702 (w), 1626 (w), 1586 (w), 1553 (s), 1520 (s), 1479 (m), 1422 (s), 1357 (m), 1224 (m), 1184 (m), 1171 (m), 1142 (m), 1126 (m), 1074 (w), 1036 (w), 1013 (m), 968 (w), 926 (s), 899 (w), 884 (w), 870 (w), 810 (m), 786 (w), 745 (w), 698 (w), 686 (w), 676 (w), 652 cm⁻¹ (w). ¹H-NMR (200 MHz, CD₃OD): *δ*= 8.78 (t, 1H, *H*_{dien}, ³*J_{E}* = 13.8 Hz), 8.30 (d, 2H, *H*_{aromat,} ³*J* = 8.7 Hz), 8.04 (t, 4H, *H*_{aromat,} ³*J* = 7.8 Hz), 7.68 (t, 4H, *H*ₐᵣₒₘₐₜ, ³*J* = 8.0 Hz), 7.57-7.48 (m, 2H, *H*ₐᵣₒₘₐₜ), 6.60 (d, 2H, *H*_{dien}, ³*J_{E}* = 13.9 Hz), 4.32 (t, 4H, 2 x NC*H*₂, ³*J* = 7.8 Hz), 2.85-2.81 (m, 4H, 2 x C*H*₂SO₃H), 2.11 (s, 12H, 4 x C*H*₃), 1.99-1.69 ppm (m, 12H, 6 x C*H*₂). UV/Vis (EtOH): *λ*ₘₐₓ (*E*ᵣₑₗ): 590 (1.0), 553 nm (0.68). UV/Vis (Feststoff/Cellulose): *λ*ₘₐₓ (*E*ᵣₑₗ): 599 (1.0), 553 nm (0.97). UV/Vis (Feststoff/Wolle): *λ*ₘₐₓ (*E*ᵣₑₗ): 600 (1.0), 553 nm (0.96). Fluoreszenz (EtOH): *λ*ₘₐₓ (*I*ᵣₑₗ): 611 (1.0), 655 nm (0.54). Fluoreszenz (Feststoff/Cellulose): *λ*ₘₐₓ (*I*ᵣₑₗ): 664 (1.0), 636 nm (0.93). Fluoreszenz (Feststoff/Wolle): *λ*ₘₐₓ (*I*ᵣₑₗ): 667 (1.0), 644 nm (0.84). Fluoreszenzquantenausbeute (CHCl₃, *λ*_{exc} = 553 nm, *E*_{553 nm/l cm} = 0.0158; Referenz: S-13 mit *Φ* = 1.00): 0.13. HRMS (ESI) (C₄₁H₄₇N₂Na₂O₆S₂⁺): Ber. 773.2671, Gef. 773.2669, *Δ* = -0.2 mmu.

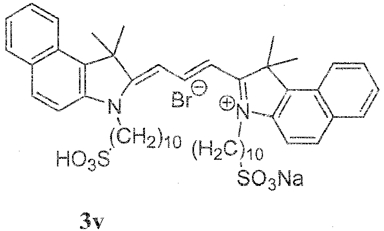

### 3,3'-Di-(10-sulfodecyl)-1,1,1',1'-tetramethyl-1H-dibenz[e]indocarbocyanin-bromid (3v)

3-(10-Sulfodecyl)-1,1,2-trimethyl-1*H*-benz[*e*]indoleniumbromid-Natriumsalz (152 mg, 0.285 mmol), 3-Picolin (1.5 mL) und Orthoameisensäuretriethylester (0.09 mL, 0.57 mmol) wurden analog zu 3,3'-Di-(pentyl)-1,1,1',1'-tetmmethyl-1*H*-dibenz[*e*]indocarbocyaninbromid (**6j**) umgesetzt (2 h bei 100°C) und aufgearbeitet. Da sich der Farbstoff auf der Chromatographiesäule zersetzt (RP 18, Methanol/H₂O/1 M HCl), wurde er ohne weitere Reinigung verwendet. Ausb. 185 mg. IR (ATR): *ṽ* = 3409 (w), 3055 (w), 2974 (w), 2926 (s), 2853 (m), 1705 (m), 1624 (w), 1588 (w), 1554 (s), 1519 (m), 1479 (m), 1425 (s), 1352 (m), 1278 (w), 1227 (m), 1169 (w), 1144 (w), 1124 (w), 1044 (w), 1012 (m), 974 (w), 931 (s), 898 (w), 806 (m), 787 (w), 747 (w), 727 (w), 684 (w), 652 (w), 637 (w), 612 cm⁻¹ (w). ¹H-NMR (200 MHz, CD₃OD): *δ* = 8.76-8.64 (m, 1H, *H*_{dien}), 8.34-8.32 (m, 2H, *H*ₐᵣₒₘₐₜ), 8.03-7.96 (m, 4H, *H*ₐᵣₒₘₐₜ), 7.71-7.67 (m, 4H, *H*ₐᵣₒₘₐₜ), 7.37-7.31 (m, 2H, *H*ₐᵣₒₘₐₜ), 6.55 (d, 2H, *H*_{dien}, ³*J_{E}* = 13.9 Hz), 4.38-4.20 (m, 4H, 2 x NC*H*₂), 2.51-2.40 (m, 4H, 2 x C*H*₂SO₃H), 2.10-1.99 (m, 16H, 4 x CH₃, 2 x C*H*₂), 1.95-1.80 (m, 8H, 4 x C*H*₂), 1.52-1.25 ppm (m, 20H, 10 x C*H*₂). UV/Vis (EtOH): *λ*ₘₐₓ (*E*ᵣₑₗ): 592 (1.0), 553 nm (0.72). UV/Vis (Feststoff/Cellulose): *λ*ₘₐₓ (*E*ᵣₑₗ): 601 (1.0), 559 nm (0.95). UV/Vis (Feststoff/Wolle): *λ*ₘₐₓ (*E*ᵣₑₗ): 599 (1.0), 556 nm (0.97). Fluoreszenz (EtOH): *λ*ₘₐₓ (*I*ᵣₑₗ): 609 (1.0), 658 nm (0.52). Fluoreszenz (Feststoff/Cellulose): *λ*ₘₐₓ (*I*ᵣₑₗ): 637 (1.0), 665 nm (0.97). Fluoreszenz (Feststoff/Wolle): *λ*ₘₐₓ (*I*ᵣₑₗ): 630 (1.0), 662 nm (0.92). Fluoreszenzquantenausbeute (CHCl₃, *λ*_{exc} = 554 nm, *E*_{554 nm/l cm} = 0.0170; Referenz: S-13 mit *Φ* = 1.00): 0.14.

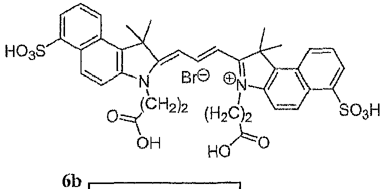

**3,3'-Di-(2-carboxyethyl)-1,1,1',1'-tetramethyl-1*H*-disulfobenz[*e*]indocarbocyanin-bromid (6b):** als Referenz 1,1,2-Trimethyl-1H-sulfobenz[*e*]indol (**4**, 245 mg, 0.847 mmol) wurde unter N₂-Schutzatmosphäre in 3-Picolin (1.0 mL) und Methanol (0.3 mL) gelöst, auf 120 °C erhitzt, mit 3-Brompropansäure (388 mg, 2.54 mmol) versetzt, 10 min bei 120°C gerührt, mit Orthoameisensäuretriethylester (0.300 mL, 1.69 mmol) versetzt (Blauviolettfärbung), 2 h auf 120°C, abkühlen lassen, mit Diethylether (20 mL) gefällt, abgesaugt und durch Flashchromatographie gereinigt (RP 18, Methanol/H₂O/1 M HCl 1:1:0.4 zum Auftragen, Methanol/H₂O/1 M HCl 2:1:0.4 zum Entfernen von Nebenprodukten und Methanol/H₂O/1 M HCl 10:1:0.4 zur Elution des Farbstoffs). Ausb. 200 mg (58%) goldglänzender Feststoff, der violette, stark rot fluoreszierende Lösungen bildet, Schmp. > 180 °C (Zers.). IR(ATR): *ṽ* = 3387 (w), 2972 (w), 2927 (w), 2858 (w), 2367 (w), 1719 (w), 1626 (w), 1562 (s), 1490 (s), 1453 (s), 1414 (m), 1384 (m), 1168 (m, br), 1124 (w), 1097 (w), 1060 (w), 1046 (w), 1025 (s), 979 (m), 927 (m), 808 (m), 761 (w), 735 (w), 689 (m), 643 cm⁻¹ (m). ¹H-NMR (200 MHz, CD₃OD): *δ* = 8.80 (t, 1H, *H*_{allyl}, ³*J_{E}* = 13.4 Hz), 8.35-8.25 (m, 4H, *H*ₐᵣₒₘₐₜ), 8.08-7.97 (m, 4H, *H*ₐᵣₒₘₐₜ), 7.58-7.47 (m, 2H, *H*ₐᵣₒₘₐₜ), 6.56 (d, 2H, *H*_{allyl}, ³*J_{E}* = 13.6 Hz), 4.59-4.53 (m, 4H, 2 x NC*H*₂), 2.94 (t, 4H, 2 x C*H*₂CO₂H, ³*J* = 7.3 Hz), 2.10 ppm (s, 12H, 4 x C*H*₃). UV/Vis (EtOH): *λ*ₘₐₓ (*E*ᵣₑₗ): 588 (1.0), 554 nm (0.74). UV/Vis (Feststoff/Cellulose): *λ*ₘₐₓ (*E*ᵣₑₗ): 591 (1.0), 550 nm (0.99). UV/Vis (Feststoff/Wolle): *λ*ₘₐₓ (*E*ᵣₑₗ): 565 (1.0), 589 nm (0.99). Fluoreszenz (EtOH): *λ*ₘₐₓ (*I*ᵣₑₗ): 612 (1.0), 657 nm (0.41). Fluoreszenz (Feststoff/Cellulose): *λ*ₘₐₓ (*I*ᵣₑₗ): 648 (1.0), 662 nm (0.94). Fluoreszenz (Feststoff/Wolle): *λ*ₘₐₓ (*I*ᵣₑₗ): 629 (1.0), 664 nm (0.72). Fluoteszenzquantenausbeute (CHCl₃, *λ*_{exc} = 482 nm, *E*_{482 nm/l cm} = 0.0197; Referenz: S-13 mit *Φ* = 1.00): 0.21. MS (FAB⁺): *m*/*z*: 733.6 [M⁺ (C₃₇H₃₇N₂O₁₀S⁺].

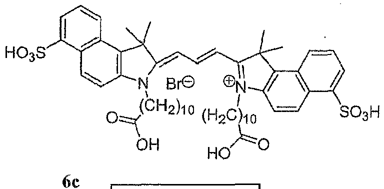

**3,3'-Di-(10-carboxydecyl)-1,1,1',1'-tetramethyl-1*H*-disulfobenz[*e*]indocarbocyanin-bromid** (**6c**): als Referenz 3-(10-Carboxyldecyl)-1,1,2-trimethyl-1*H*-sulfobenz[*e*]indol (**2f**, 100 mg, 0.180 mmol) wurde unter N₂-Schutzatmosphäre in Pyridin (1.0 mL) und Methanol (0.3 mL) gelöst, auf 120°C erhitzt, sehr langsam tropfenweise mit Orthoameisensäuretriethylester (0.06 mL, 0.36 mmol) versetzt, mit einer zweiten Portion Pyridin (0.5 mL) und Orthoameisensäuretriethylester (0.03 mL, 0.18 mmol) versetzt. 2 h auf 120 °C erhitzt, abkühlen lassen, mit Diethylether (20 mL) gefällt, abgesaugt und durch Flashchromatographie gereinigt (RP 18, Methanol/H₂O/1 M HCl 1:1:0.4 zum Auftragen, Methanol/H₂O/1 M HCl 2:1:0.4 zum Entfernen von Nebenprodukten und Methanol/H₂O/1 M HCl 10:1:0.4 zur Elution des Farbstoffs). Ausb. 66.0 mg (71%) goldglänzender Feststoff, der violette, stark rot fluoreszierende Lösungen bildet, Schmp. > 150 °C (Zers.). IR(ATR): *ṽ* = 3392 (w), 2922 (s), 2852 (s), 2362 (w), 1718 (s, br), 1585 (w), 1554 (s), 1515 (m), 1480 (m), 1420 (s), 1361 (m), 1274 (w), 1224 (w), 1166 (m), 1131 (m), 1100 (m), 1029 (w), 1015 (w), 932 (s), 898 (w), 827 (w), 806 (w), 746 (w), 689 (m), 657 (w), 630 cm⁻¹ (w). ¹H-NMR (200 MHz, CD₃OD): *δ* = 8.79 (t, 1H, *H*_{dien}*,* ³*J_{E}* = 13.4 Hz), 8.47-8.35 (m, 4H, *H*ₐᵣₒₘₐₜ), 8.22-8.05 (m, 4H, *H*ₐᵣₒₘₐₜ), 7.72 (t, 2H, *H*ₐᵣₒₘₐₜ, ³*J* = 7.7 Hz), 6.54 (d, 2H, *H*_{dien}, ³*J_{E}* = 13.7 Hz), 4.29 (t, 4H, 2 x NC*H*₂, ³*J* = 8.7 Hz), 2.31-2.21 (m, 4H, 2 x C*H*₂CO₂H), 2.11 (s, 12H, 4 x C*H*₃), 1.97-1.85 (m, 4H, 2 x C*H*₂) 1.59-1.13 ppm (m, 28H, 14 x C*H*₂). UV/Vis (EtOH): *λ*ₘₐₓ (*E*ᵣₑₗ): 593 (1.0), 554 nm (0.68). UV/Vis (Feststoff/Cellulose): *λ*ₘₐₓ (*E*ᵣₑₗ): 594 (1.0); 553 mm (094). UV/Vis (Feststoff/Wolle): *λ*ₘₐₓ (*E*ᵣₑₗ): 598 (1.0), 556 nm (0.94). Fluoreszenz (EtOH): *λ*ₘₐₓ (*I*ᵣₑₗ): 608 (1.0), 656 nm (0.48). Fluoreszenz (Feststoff/Cellulose): *λ*ₘₐₓ (*I*ᵣₑₗ): 663 (1.0), 646 nm (0.89). Fluoreszenz (Feststoff/Wolle): *λ*ₘₐₓ (*I*ᵣₑₗ): 664 (1.0), 641nm (0.91). Fluoreszenzquantenausbeute (CHCl₃, λ_{exc} = 554 nm, *E*_{554m/l cm} = 0.0157; Referenz: S-13 mit *Φ* = 1.00): 0.31. HRMS (ESI) (C₅₃H₆₉N₂O₁₀S₂⁺): Ber. 957.4388, Gef. 957.4392 *Δ* = -0.4 mmu.

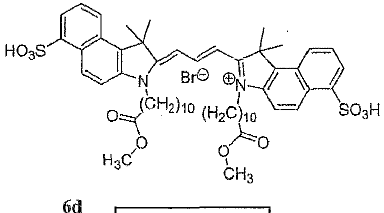

**3,3'-Di-(10-methoxycarbonyldecyl)-1,1,1',1'-tetramethyl-1*H*-disulfobenz[*e*]indo-carbocyaninbromid (6d):** als Referenz Unter Stickstoff-Atmosphäre wurde 1,1,2-Trimethyl-1H-sulfobenz[*e*]indol (**4**, 267 mg, 0.923 mmol), Pyridin (0.5 mL), Methanol (0.25 mL), 11-Bromundecansäuremethylester (0.700 mL, 2.78 mmol, 10 min, 120°C), Orthoameisensäuretriethylester (0.300 mL, 1.86 mmol) und Diethylether (20 mL) wurden analog zu 3,3'-Di-(2-carboxyethyl)-1,1,1',1'-tetramethyl-1*H*-disulfobenz[*e*]indocarbocyanin-bromid (**6b**) umgesetzt (1.5 h bei 120°C) und aufgearbeitet, Da sich der Farbstoff auf der Chromatographiesäule zersetzt (RP 18, Methanol/H₂O/1 M HCl), wurde stattdessen mehrfach in wenig Ethanol gelöst und mit Diethylether ausgefällt. Ausb. 200 mg (41%) goldglänzender Feststoff, der violette, stark rot fluoreszierende Lösungen bildet, Schmp. >150°C (Zers.). IR(ATR): *ṽ* = 3430 (m), 3180 (w), 3140 (w), 3072 (m), 2927 (m), 2855 (w), 2158 (w), 1712 (m), 1637 (s), 1583 (w), 1544 (m), 1489 (s), 1466 (w), 1393 (w), 1316 (w), 1176 (s), 1100 (m), 1044 (w), 1021 (s), 931 (w), 857 (w), 754 (s), 681 (s), 658 (m), 643 (w), 608 cm⁻¹ (w). ¹H-NMR (200 MHz, CD₃OD): *δ* = 8.50-8.42 (m, 1H, *H*_{dien}), 8.40-8.24 (m, 6H, *H*ₐᵣₒₘₐₜ), 7.90-7.64 (m, 4H, *H*ₐᵣₒₘₐₜ), 6.56 (d, 2H, *H*_{dien}, ³*J_{E}* = 13.9 Hz), 4.35-4.30 (m, 4H, 2 x NC*H*₂), 3.31 (s, 6H, 2 x OC*H*₃), 2.29-2.20 (m, 4H, 2 x C*H*₂CO₂CH₃), 2.10 (s, 12H, 4 x C*H*₃), 1.98-1.88 (m, 4H, C*H*₂), 1.65-1.45 (m, 12H, 6 x C*H*₂), 1.38-1.30 ppm (m, 16H, 8 x C*H*₂). UV/Vis (EtOH): *λ*ₘₐₓ (*E*ᵣₑₗ): 587 (1.0), 556 nm (0.78). UV/Vis (Foststoff/Cellulose): *λ*ₘₐₓ (*E*ᵣₑₗ): 588 (1.0), 555 nm (0.97). UV/Vis (Festgoff/Wolle): *λ*ₘₐₓ (*E*ᵣₑₗ): 551 (1.0), 586 nm (0.87). Fluoreszenz (EtOH): *λ*ₘₐₓ (*I*ᵣₑₗ): 607 (1.0), 657 nm (0.48). Fluoreszenz (Feststoff/Cellulose): *λ*ₘₐₓ (*I*ᵣₑₗ): 624 (1.0), 660 nm (0.74). Fluoreszenz (Feststoff/Wolle): *λ*ₘₐₓ (*I*ᵣₑₗ): 617 (1.0), 660 nm (0.60). Fluarteszenzquantenausbeute (CHCl₃, *λ*_{exc} = 556 nm, *E*_{556nm/1 cm} = 0.0159; Referenz S-13 mit *Φ* = 1.00): 0.25. MS (FAB⁻): *m*/*z*: 1063.3 [M⁻ (C₅₅H₇₂BrN₂O₁₀S].

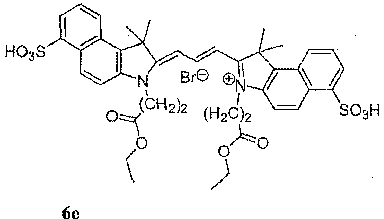

**3,3'-Di-(2-ethorycarbonylethyl)-1,1,1',1'-tetramethyl-1*H*-disulfobenz[*e*]indocarbocyaninbromid (6e):** als Referenz 3-(2-Ethoxycarbonylethyl)-1,1,2-trimethyl-1*H*-sulfobenz[*e*]indol (**5e**, 210 mg, 0.446 mmol), 3-Picolin (1.0 mL) und Orthoameisensäuretriethylester (0.15 mL, 0.89 mmol) wurden analog zu 3,3'-Di-(pentyl)-1,1,1',1'-tetramethyl-1*H-*dibenz[*e*]indocerbocyaninbromid (**6j**) umgesetzt (2 h bei 120°C) und aufgearbeitet und durch Flashchromatographie gereinigt (RP 18, Methanol/H₂O/1 M HCl 1:1:0.4 zum Auftragen und Methanol/H₂O/1 M HCl 10:1:0.4 zur Elution des Farbstoffe). Ausb. 100 mg (52%) goldglänzender Feststoff, der violette, stark rot fluoreszierende Lösungen bildet, Schmp. >150 °C (Zers.). IR(ATR): *ṽ* = 3411 (w), 3068 (w), 2951 (w), 2924 (s), 2854 (m), 1725 (m), 1623 (w), 1584 (w), 1559 (w), 1514 (w), 1478 (w), 1458 (w), 1426 (m), 1390 (w), 1362 (w), 1168 (s), 1100 (m), 1060 (w), 1030 (s), 940 (m), 827 (w), 809 (w), 762 (w), 692 (m), 649 (w), 624 cm⁻¹ (w). ¹H-NMR (CDCl₃): Starke Linienverbreiterung durch Aggregation. UV/Vis (EtOH): *λ*ₘₐₓ (*E*ᵣₑₗ): 596 (1.0), 569 nm (0.79). UV/Vis (Feststoff/Cellulose): *λ*ₘₐₓ (*E*ᵣₑₗ): 599 (1.0), 564 nm (0.97). UV/Vis (Feststoff/Wolle): *λ*ₘₐₓ (*E*ᵣₑₗ): 563 (1.0), 597 nm (0.94). Fluoreszenz (EtOH): *λ*ₘₐₓ (*I*ᵣₑₗ): 610 (1.0), 660 nm (0.51). Fluoreszenz (Feststoff/Cellulose): *λ*ₘₐₓ (*I*ᵣₑₗ): 654 (1.0), 633 nm (0.98). Fluoreszenz (Feststoff/Wolle): *λ*ₘₐₓ (*I*ᵣₑₗ): 627 (1.0), 662 mn (0.88). Fluoteszenzquamenausbeute (CHCl₃, *λ*_{exc} = 559 nm, *E*_{559 nm/1 cm} = 0.0145; Referenz: S-13 mit *Φ* = 1.00): 0.18.

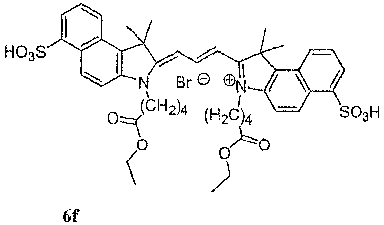

**3,3'-Di-(4-ethoxycarbonylbutyl)-1,1,1',1'-tetramethyl-1*H*-disulfobenz[*e*]indocarbocyaninbromid (6f):** als Referenz 1,1,2-Trimethyl-1H-sulfobenz[*e*]indol (**4**, 250 mg, 0.864 mmol), 3-Picolin (1.0 mL), Ethanol (0.2 mL), 5-Brompentansäureethylester (0.40 mL, 2.6 mmol, 10 min, 120 °C). Ortho-ameisensäuretriethylester (0.300 mL, 1.73 mmol) und Diethylether (20 mL) wurden analog zu 3,3'-Di-(2-carboxyethyl)-1,1,1',1'-tetramethyl-1*H-*disulfobenz[*e*]indocarbocyaninbromid (**6b**) umgesetzt (2 h, 120°C) und aufgearbeitet (der Farbstoff zersetzt sich bereits nach einem Tag unter Gelbfärbung) und durch Flashchromatographie gereinigt (RP 18, Methenol/H₂O/1 M HCl 1:1:0.4 zum Auftragen und Methanol/H₂O/1 M HCl 10:1:0.4 zur Elution des Farbstoffs). Ausb. 33.0 mg (8%) goldgläzender Feststoff, der violette, stark rot fluoreszierende Lösungen bildet, Schmp. >150 °C (Zers.). IR(ATR): *ṽ* = 3362 (w), 2971 (w), 2931 (w), 1724 (w), 1623 (w), 1564 (m), 1519 (w), 1492 (s), 1455 (m), 1416 (m), 1386 (m), 1185 (s, br), 1099 (m), 1060 (w), 1046 (w), 1027 (s), 980 (m), 929 (m), 809 (m), 763 (w), 736 (w), 691 (m), 643 cm⁻¹ (m). UV/Vis (EtOH): *λ*ₘₐₓ (*E*ᵣₑₗ): 588 (1.0), 556 nm (0.76). UV/Vis (Feststoff/Cellulose): *λ*ₘₐₓ (*E*ᵣₑₗ): 591 (1.0), 550 nm (0.98). UV/Vis (Feststoff/Wolle): *λ*ₘₐₓ (*E*ᵣₑₗ): 559 (1.0), 591 nm (0.95). Fluoreszenz (EtOH): *λ*ₘₐₓ (*I*ᵣₑₗ): 613 (1.0), 662 nm (0.40). Fluoreszenz (Feststoff/Cellulose): *λ*ₘₐₓ (*I*ᵣₑₗ): 636 (1.0), 671 nm (0.76). Fluoreszenz (Feststoff/Wolle): *λ*ₘₐₓ (*I*ᵣₑₗ): 637 (1.0), 664 nm (0.93). Fluoreszenzquantenausbeute (CHCl₃, *λ*_{exc} = 478 nm, *E*_{478 nm/1 cm} = 0.0170; Referenz: S-13 mit *Φ* = 1.00): 0.11. HRMS (ESI) (C₄₅H₅₁N₂O₁₀S₂⁻): Ber. 843.2991, Gef. 843.3797, *Δ* = 80.6 mmu.

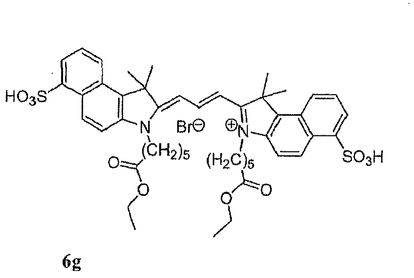

**3,3'-Di-(5-ethoxycarbonylpentyl)-1,1,1',1'-tetramethyl-1*H*-disulfobenz[*e*]indocarbocyaninbromid (6g):** als Referenz 3-(5-Ethoxycarbonylpentyl)-1,1,2-trimethyl-1*H*-sulfobenz[*e*]indol (100 mg, 0.195 mmol), 3-Picolin (1.0 mL), Methanol (0.3 mL), Orthoameisensäuretriethylester (0.06 mL, 0.39 mmol) und Diethylether (20 mL) wurden analog zu 3,3'-Di-(peatyl)-1,1,1',1'-tetramethyl-1*H*-dibeaz[*e*]indocarbocyaninbromid umgesetzt (2 h bei 120°C) und aufgearbeitet und durch Flashchromatographie gereinigt (RP 18, Methanol/H₂O/1 M HCl 1:1:0.4 zum Auftragen und Methanol/H₂O/1 M HCl 10:1:0.4 zur Elution des Farbstoffs). Ausb. 20 mg (2%) goldglänzender Feststoff, der violette, stark rot fluoreszierende Lösungen bildet, Schmp. >150°C (Zers.). IR(ATR): *ṽ* = 3410 (w), 3069 (w), 2926 (m), 2856 (w), 1710 (s), 1623 (w), 1556 (m), 1515 (m), 1480 (m), 1426 (s), 1362 (m), 1157 (s), 1135 (w), 1100 (m), 1029 (s), 1018 (s), 932 (s), 901 (w), 808 (w), 764 (w), 693 (m), 627 cm⁻¹ (w). ¹H-NMR (200 MHz, CD₃OD): *δ* = 8.79 (t, 1H, *H*_{dien}, *³J_{E}* = 13.9 Hz), 8.45-8.34 (m, 4H, *H*ₐᵣₒₘₐₜ). 8.21-8.04 (m, 4H, *H*ₐᵣₒₘₐₜ), 7.72 (t, 2H, *H*ₐᵣₒₘₐₜ, *³J* = 7.7 Hz), 6.55 (d, 2H, *H*_{dien}*.* ³*J_{E}* = 13.8 Hz), 4.35-4.24 (m, 4H, 2 x NC*H*₂), 3.88-3.77 (m, 4H, 2 x C*H*₂CH₃), 2.38-2.31 (m, 4H, 2 x C*H*₂CO₂), 2.10 (s, 12H, 4 x C*H*₃), 1.99-1.87 (m, 8H, 4 x C*H*₂), 1.78-1.61 (m, 4H, 2 x C*H*₂), 1.00-0.90 ppm (m; 6H, 2 x CH₂C*H*₃). Zusätzlich treten im NMR-Spektrum Signale der Carbonsäure als Verseifungsprodukt der Ethylester-Funktion auf. ¹³C-NMR. (100 MHz, CD₃OD): *δ* = 175.9, 142.2, 133.1, 132.7, 131.6, 129.9, 128.6, 127.7, 126.6, 125.6, 123.9, 113.3, 112.4, 52.6, 45.5, 34.7, 28.3, 27.4, 25.8, 24.5, 13.8 ppm. UV/Vis (EtOH): *λ*ₘₐₓ (*E*ᵣₑₗ): 593 (1.0), 557 nm (0.69). UV/Vis (Feststoff/Cellulose): *λ*ₘₐₓ (*E*ᵣₑₗ): 597 (1.0), 556 nm (0.97). UV/Vis (Feststoff/Wolle): *λ*ₘₐₓ (*E*ᵣₑₗ): 561 (1.0), 596 nm (0.98). Fluoreszenz (EtOH): *λ*ₘₐₓ (*I*ᵣₑₗ): 610 (1.0), 660 nm (0.51). Fluoreszenz (Feststoff/Cellulose): *λ*ₘₐₓ (*I*ᵣₑₗ): 622 (1.0), 654 mm (0.97). Fluoreszenz (Feststoff/Wolle): *λ*ₘₐₓ (*I*ᵣₑₗ): 660 (1.0), 641 nm (0.96). Fluoreszenzqueatenausbeute (CHCl₃, *λ*_{exc} = 559 nm, *E*_{559 nm/1 cm} = 0.0115; Referenz: S-13 mit *Φ* = 1.00): 0.22.

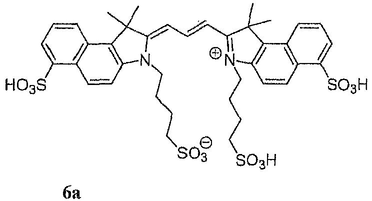

**3,3'-Di-(4-sulfobutyl)-1,1,1',1'-tetramethyl-1*H*-dissulfobenz[*e*]indocarbocyanin (6a):** als Referenz 3-(4-Sulfobutyl)-1,1,2-trimethyl-1*H*-sulfobenz[*e*]indol (140 mg, 0.276 mmol), Pyridin (1.5 mL), Orthoameisensäuretriethylester (0.090 mL, 0.55 mmol) und Diethylether (15 mL) wurden analog zu 3,3'-Di-(pentyl)1,1,1',1'-tetramethyl-1*H*-dibenz[*e*]indocarbocyaninbromid umgesetzt (2 h bei 120°C) und aufgearbeitet, mehrfach in wenig Ethanol gelöst und mit Diethylether gefällt und durch Flashchromatographie gereinigt (RP 18, Methanol/H₂O/1 M HCl 1:1:0.4 zum Auftragen und Methanol/H₂O/1 M HCl 10:1:0.4 zur Elution des Farbstoffs). Ausb. 100 mg (77%) goldglänzender Feststoff, der violette, stark rot fluoreszierende Lösungen bildet, Schmp. >150°C. IR(ATR): *ṽ* = 3774 (w), 3450 (s, br), 2976 (w), 2938 (w), 2873 (w), 2361 (w), 2331 (w), 2008 (w), 1691 (w), 1640 (w), 1556 (s), 1515 (m), 1484 (m), 1431 (s), 1364 (m), 1277 (w), 1173 (s, br), 1103 (w), 1034 (m), 940 (m), 900 (w), 812 (w), 783 (w), 756 (w), 692 (w), 636 (w), 610 cm⁻¹ (w). ¹H-NMR (200 MHz, CD₃OD): *δ* = 8.71-8.54 (m, 7H, *H*_{dien,} *H*ₐᵣₒₘₐₜ), 8.40-8.33 (m, 2H, *H*ₐᵣₒₘₐₜ), 7.80-7.64 (m, 2H, *H*ₐᵣₒₘₐₜ), 6.63 (d, 2H. *H*_{dien}, ³*J_{E}* = 13.2 Hz), 4.37-4.34 (m, 4H, 2 x NC*H*₂), 2.98-2.97 (m, 4H, 2 x C*H*₂SO₃H), 2.11-2.00 (m, 16H, 4 x C*H*₃, 2 x C*H*₂), 1.65 ppm (t, 4H, 2 x C*H*₂, ³*J* = 7.7 Hz). UV/Vis (EtOH): *λ*ₘₐₓ (*E*ᵣₑₗ): 593 (1.0), 556 nm (0.66). UV/Vis (Feststoff/Cellulose): *λ*ₘₐₓ (*E*ᵣₑₗ): 546 (1.0), 586 nm (0.94). UV/Vis (Feststoff/Wolle): *λ*ₘₐₓ (*E*ᵣₑₗ): 554 (1.0), 588 nm (0.92). Fluoreszenz (EtOH): *λ*ₘₐₓ (*I*ᵣₑₗ): 608 (1.0), 655 nm (0.47). Fluoreszenz (Feststoff/Cellulose): *λ*ₘₐₓ (*I*ᵣₑₗ): 654 (1.0), 629 nm (0.94). Fluoreszenz (Feststoff/Wolle): *λ*ₘₐₓ (*I*ᵣₑₗ): 631 (1.0), 661 nm (0.99). Fluoreszenzquantenausbeute (CHCl₃, *λ*_{exc} = 556 nm, *E*_{556 nm/1 cm} = 0.0099; Referenz: S-13 mit *Φ* = 1.00): 0.33. HRMS (ESI) (C₃₉H₄₅N₂O₁₂S₄⁺): Ber. 861.1850, Gef. 861.1851, *Δ* = 0.1 mmu.

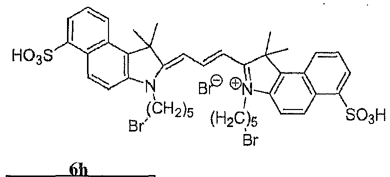

**3,3'-Di-(5-brompentyl)-1,1,1',1'-tetramethyl-1*H*-disulfobenz[*e*]indocarbocyanin-bromid (6h):** als Referenz 1,1,2-Trimethyl-1*H*-sulfobenz[*e*]indol (215 mg, 0.743 mmol), 3-Picolin (1.5 mL), Methanol (1.0 mL), 1,5-Dibrompentan (0.30 mol, 2.2 mmol, 10 min, 120°C), Orthoameisensäuretriethylester (0.250 mL, 1.45 mmol) und Diethylether (20 mL) wurden analog zu 3,3'-Di-(2-carboxyethyl)-1,1,1',1'-tetramethyl-1*H*-disulfobenz[*e*]indocarbocyaninbromid umgesetzt (2 h, 120°C) und aufgearbeitet und durch Flashchromatographie gereinigt (RP 18, Methenol/H₂O/1 M HCl 1:1:0.4 zum Auftragen und Methanol/H₂O/1 M HCl 10:1:0.4 zur Elution des Farbstoffs). Ausb. 98 mg (27%) goldglänzender Feststoff, der violette, stark rot fluoreszierende Lösungen bildet, Schmp. > 75 °C (Zers.). IR(ATR): *ṽ* = 3398 (s, br), 3056 (m), 3036 (w), 2866 (w), 2074 (w), 1634 (m), 1592 (w), 1559 (w), 1505 (s), 1483 (w), 1463 (w), 1387 (w), 1325 (w), 1250 (w), 1202 (m), 1155 (m), 1098 (w), 1048 (m), 1031 (m), 928 (w), 808 (m), 749 (w), 683 (m), 657 cm⁻¹ (w). UV/Vis (EtOH): *λ*ₘₐₓ (*E*ᵣₑₗ): 590 (1.0), 559 nm (0.78). UV/Vis (Feststoff/Cellulose): *λ*ₘₐₓ (*E*ᵣₑₗ): 600 (1.0), 565 nm (0.95). UV/Vis (Feststoff/Wolle): *λ*ₘₐₓ (*E*ᵣₑₗ): 558 (1.0), 587 nm (0.95). Fluoreszenz (EtOH): *λ*ₘₐₓ (*I*ᵣₑₗ): 611 (1.0), 659 nm (0.50). Fluoreszenz (Feststoff/Cellulose): *λ*ₘₐₓ (*I*ᵣₑₗ): 626 (1.0), 660 nm (0.83). Fluoreszenz (Feststoff/Wolle): *λ*ₘₐₓ (*I*ᵣₑₗ): 615 (1.0), 658 nm (0.60). Fluoreszenzquantenausbeute (CHCl₃, *λ*_{exc} = 553 nm, *E*_{553 nm/1 cm} = 0.0124; Referenz: **S-13** mit *Φ* = 1.00): 0.19.

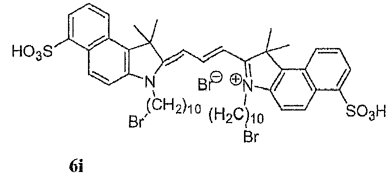

**3,3'-Di-(10-bromdecyl)-1,1,1',1'-tetramethyl-1*H*-disulfobenz[*e*]indocarbocyaninbromid** (**6i**)**:** als Referenz 1,1,2-Trimethyl-1H-sulfobenz[*e*]indol (205 mg, 0.708 mmol), 3-Picolin (1.0 mL), Methanol (0.3 mL), 1,10-Dibromdecan (0.50 mL, 2.1 mmol, 10 min,120°C), Orthoameisensäuretriethylester (0.24 mL, 1.4 mmol) und Diethylether (20 mL) wurden analog zu 3,3'-Di-(2-carboxyethyl)-1,1,1',1'-tehamethyl-1*H*-disulfobenz[*e*]indocerbocyaninbromid (**6b**) umgesetzt (2 h, 120°C) und aufgearbeitet und durch Flashchromatographie gereinigt (RP 18, Methanol/H₂O/1 M HCl 1:1:0.4 zum Auftragen und Methanol/H₂O/1 M HCl 10:1:0.4 zur Elution des Farbstoffs). Ausb. 58 mg (14%) goldglänzender Feststoff, der violette, stark rot fluoreszierende Lösungen bildet, Schmp. > 100°C (Zers.). IR(ATR): *ṽ*=3387 (s), 3059 (w), 2927 (s), 2855 (s), 1706 (m), 1635 (m), 1591 (w), 1562 (m), 1504 (m), 1489 (m), 1456 (m), 1414 (w), 1388 (w), 1364 (w), 1186 (s), 1098 (m), 1047 (w), 1029 (s), 981 (m), 932 (m), 811 (m), 765 (w), 734 (w), 688 (m), 645 cm⁻¹ (m). UV/Vis (EtOH): *λ*ₘₐₓ (*E*ᵣₑₗ): 587 (1.0), 555 nm (0.77). UV/Vis (Feststoff/Cellulose): *λ*ₘₐₓ (*E*ᵣₑₗ): 500 (1.0), 591 nm (0.75). UV/Vis (Feststoff/Wolle): *λ*ₘₐₓ (*E*ᵣₑₗ): 551 (1.0), 591 nm (0.95). Fluoreszenz (EtOH): *λ*ₘₐₓ (*I*ᵣₑₗ): 614 (1.0), 661 nm (0.42). Fluoreszenz (Feststoff/Cellulose): *λ*ₘₐₓ (*I*ᵣₑₗ): 620 (1.0), 655 nm (0.70). Fluoreszenz (Feststoff/Wolle): *λ*ₘₐₓ (*I*ᵣₑₗ): 628 (1.0), 665 nm (0.77). Fluoreazenzquantenausbeute (CHCl₃, *λ*_{exc} = 481nm, *E*_{481 nm/1 cm} = 0.0181; Referenz: S-13 mit *Φ* = 1.00): 0.13.

### Biologische Tests

**BSS plus®:** Sterile intraoculare Spüllösung, die durch 1:1 Mischung von zwei Teillösungen erhalten wird. Teillösung 1: 1 mL enthält 7.44 mg NaCl, 0.395 mg KCl, 0.433 mg Na2HPO4, in Wasser, ggf. HCl oder NaOH zur Einstellung eines physiologischen pH-Werts. Teillösung 2: 1 mL enthält 3.85 mg CaCl2 · 2 H2O, 5 mg MgCl2 · 6 H2O, 23 mg Glucose, 4.6 mg Glutathiondisulfid in Wasser. Für den Färbetest wurden jeweils 5 mg Farbstoff in 500 µL Ethanol gelöst und mit BSS plus Lösung auf 2 mL als Stammlösung aufgefüllt und dann entsprechend weiter verdünnt. Als Alternative kann man zur Herstellung von Stammlösungen des Farbstoffs beispielsweise 5 mg Farbstoff in 1 mL destilliertem Wasser lösen, zu 7 mL BSS-Lösung geben und dann mit 1 mL einer Kochsalzlösung einer Osmolarität von 620 (ca. 2%) versetzen um die ursprüngliche Osmolarität der BSS-Lösung wiederherzustellen (308). Diese Stammlösung kann entsprechend der Erfordernisse mit BSS-Lösung weiter verdünnt werden.

### Färbeuntersuchungen am Schweineauge:

Enukleierte Schweineaugen mit einer postmortem Zeit von wenigen Stunden wurden verwendet. Zur Anfärbung der Linsenkapsel wurde zunächst die Hornhaut entfernt. Anschließend wurde die Regenbogenhaut reseziert und der Zonulaapparat durchtrennt. Die Linse mit intakter Linsenkapsel wurde entnommen und in eine mit BSS gefüllte Schale platziert. Die Linsen wurden im Anschluss mit den erfindungsgemäßen Farbstoffen in verschiedenen Konzentrationen inkubiert und anschließen nach einer Minute wieder abgespült. Das Färbemuster wurde photographisch dokumentiert, wobei die Anfärbung ohne zusätzliche Beleuchtung und die Fluoreszenz mit Beleuchtung (Xenonlampe, Halogenlampe mit typischer Endoilluminationsquelle und Fiberoptic) beurteilt wurde.

Die Linsenkapsel ist für Färbungen ein sehr gutes Modell, da es sich bei der Linsenkapsel um eine Basalmembran handelt, wie auch die LLI der Netzhaut. Beide Strukturen sind wichtige Zielstrukturen in der Augenchirurgie.

Die folgende Tabelle zeigt Ergebnisse der Färbeuntersuchungen bei verschiedenen Farbstoffkonzentrationen (+++ steht für visuell beurteilte sehr starke Färbung, ++ für gute Färbung; + für ausreichende Färbung und - für schwache/nicht vorhandene Färbung).

| **Farbstoff** | **1.0** | **0.5%** | **0.25%** | **0.025%** | **0.0025%** |
|---|---|---|---|---|---|
| 3a | | ++ | ++ | + | - |
| 3c | | +++ | +++ | - | - |
| 3d | | +++ | + | - | - |
| 3e | | ++ | ++ | - | - |
| 3g | | ++ | ++ | - | - |
| 3h | | +++ | ++ | + | - |
| 3i | | ++ | + | + | - |
| 3j | | ++ | + | - | - |
| 3k | | ++ | + | - | - |
| 3l | | +++ | +++ | + | - |
| 3m | | +++ | ++ | - | - |
| 3n | | ++ | + | - | - |
| 3o | | ++ | ++ | - | - |
| 3t | | +++ | ++ | + | - |
| 3u | + | - | | | |
| 3v | - | - | | | |
| 6a | +++ | +++ | | | |
| 6b | +++ | +++ | | | |
| 6c | ++ | ++ | | | |
| 6d | Zers. | Zers. | | | |
| 6e | Zers. | Zers. | | | |
| 6f | Zers. | Zers. | | | |
| 6g | ++ | ++ | | | |
| 6h | Zers. | Zers. | | | |
| 6i | Zers. | Zers. | | | |

### Toxizitätsstudien unter Verwendung etablierter Zellkulturmodelle; MTT Assay:

Für die Untersuchung zur Einwirkung von Farbstoffen auf Zellen wurde ARPE-19 Zellmaterial bzw. primäre humane RPE-Zellen 24 h unter serumfreien Bedingungen gehalten. RPE-Zellen wurden erhalten und kultiviert wie in Eibl K.H., Banas B., Schoenfeld C.L., May C.A., Neubauer A.S., Priglinger S., Kampik A. und Welge-Lussen U., Alkylphosphocholines inhibit proliferation of human retinal pigment epithelial cells, Invest Ophthalmol Vis Sci 2003; 44: 3556-3561 beschrieben. Nach drei Waschschritten mit PBS wurden die Zellen 30, 60, 120 und 300 s lang mit 300 µL BSS plus^{®} Lösungen inkubiert, die jeweils 0.5%, 0.25% und 0.1% 3,3'-Di-(4-sulfobutyl)-1,1,1',1'-tetramethyl-1*H*-dibenz[*e*]indocarbocyanin (**3t**) enthielten. Sowohl für die ARPE 19 als auch die primären RPE-Zellen wurde ICG in den gleichen Konzentrationen und Inkubationszeiten als Referenz eingesetzt. Die verhältnismäßig langen Inkubationszeiten sind sinnvoll, um auch schwach toxische Wirkungen aufzufinden, obwohl sie nicht die klinischen Verwendungen der Farbstoffe widerspiegeln. Der überschüssige Farbstoff wurde durch dreifaches Waschen der Zellen mit BSS plus^{®} entfernt und danach der Zellwachtumsversuch (Zellproliferationsversuch) ausgeführt. Kontrollversuche wurde mit BSS plus^{®} ohne Zusätze und mit Zusatz von H₂O₂, (200µL/mL) ausgeführt.

Der Tetrazoliumfarbstoff-Reduktions-Assay (MTT; 3-(4,5-Dimethylthiazol-2-yl)-2,5-diphenyl tetrazoliumbromid) wurde verwendet, um die Überlebensrate der Zellen zu bestimmen. Der MTT-Test nach Mosmann wurde modifiziert ausgeführt (K. H. Eibl, B. Banas, C. L. Schoenfeld, C. A. May, A. S. Neubauer, S. Priglinger, A. Kampik, U. Welge-Lussen, Invest. Ophthalmol. Vis. Sci. 2003, 44, 3556-3561). Das Medium wurde entfernt, die Zellen wurden mit PBS und 1000 µL/well MTT Lösung (1.5 mL MTT Stammlösung, 2 mg/mL in PBS und 28.5 mL DMEM) versetzt, RPE Zellen wurden 1 h bei 37°C inkubiert, entstandene Formazan-Kristalle durch Zugabe von Dimethylsulfoxid gelöst (DMSO; 1000 µL/well) und die Absorption mit einem Scanning Multiwell Spektrophotometer bei 550 nm (Molecular Probes, Garching, Germany) gemessen. Die Ergebnisse wurden auf den gemittelten Prozenzsatz des Kotrollwachstums (Kontroll-Proliferation) bezogen. Die Experimente wurden in dreifacher Ausführung dreifach wiederholt. ARPE-19 Zellen der gleichen Versuchsserie bzw. RPE-Zellen inkubiert in BSS dienten als Kontrollversuch. Der statistische Vergleich der Versuche mit verschiedenen Farbstoffkonzentrationen erfolgte mit SPSS (Mann-Whitney-U-Test).

Der ausgeführte MTT Test ist für die Bestimmung der Zellvitalität etabliert, hängt aber von der colorimetrischen Messung eines blauen (550 nm) Formazan-Produkts ab. Die hierauf basierende Lichtabsorption überlappt teilweise mit der Absorption der untersuchten Farbstoffe. Es wurden daher Kontrollexperimente ausgeführt, um mögliche Störungen des Tests abzuschätzen. Zell-Monoschichten wurden wie bei den anderen Versuchen mit dem Farbstoff behandelt, aber die Auslesung der Absorption erfolgte ohne vorherige Anwendung von MTT. Wir fanden keine Unterschiede nach der Behandlung mit Farbstoffen verglichen mit den BSS Kontrollexperimenten. Verglichen mit ICG als Referenz zeigte sich eine signifikant erhöhte Überlebensrate der Zellen mit dem erfindungsgemäßen Farbstoff. Die Versuche wurden in dreifacher Ausführung dreimal wiederholt.

Figur 3 zeigt die Zahl der ARPE-19 bzw. RPE-Zellen, gemessen mit dem beschriebenen colorimetrischen Verfahren (MTT) nach der Behandlung mit dem Farbstoff: ARPE-19: A: 0.5%, B: 0.25%, C: 0.1% des Farbstoffs **3t**; RPE: D: 0.5%, E: 0.25%, F: 0.1% des Farbstoffs **3t**. AVS4 bezeichnet die Substanz **3t**.

## Patentansprüche

1. Farbstoff der Formel (IIb) 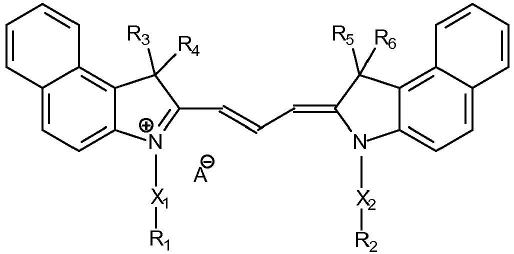
wobei X₁ und X₂ unabhängig voneinander ausgewählt sind aus einer bis 12 CH₂-Einheiten, bei denen unabhängig voneinander eine oder mehrere ersetzt sein können durch eine Carbonylgruppe, ein Sauerstoffatom, ein Schwefelatom, eine *cis* oder *trans*-CH=CH-Gruppe, bei der eine CH-Einheit auch durch ein Stickstoffatom ersetzt sein kann, eine acetylenische C=C-Gruppe, einen divalenten Phenyl-, Pyridin-, oder Thiophenrest, einen divalenten Naphthalinrest, bei dem ein oder zwei CH-Gruppen durch Stickstoffatome ersetzt sein können, einen divalenten Anthracenrest, bei dem ein oder zwei CH-Gruppen durch Stickstoffatome ersetzt sein können; und wobei bis zu 12 einzelne Wasserstoffatome der CH₂-Einheiten jeweils unabhängig voneinander auch an gleichen C-Atomen ersetzt sein können durch die Halogene Fluor, Chlor, Brom oder Jod oder die Cyanogruppe oder durch eine lineare Alkylkette mit bis zu 18 C-Atomen, bei der eine bis 6 CH₂-Einheiten unabhängig voneinander ersetzt sein können durch eine Carbonylgruppe, ein Sauerstoffatom, ein Schwefelatom, eine *cis* oder *trans-*CH=CH-Gruppe, bei der eine CH-Einheit auch durch ein Stickstoffatom ersetzt sein kann, eine acetylenische C≡C-Gruppe, einen divalenten Phenyl-, Pyridin- oder Thiophenrest, einen divalenten Naphthalinrest, bei dem ein oder zwei CH-Gruppen durch Stickstoffatome ersetzt sein können, oder einen divalenten Anthracenrest, bei dem ein oder zwei CH-Gruppen durch Stickstoffatome ersetzt sein können;
R₁ und R₂ unabhängig voneinander ausgewählt sind aus einer Carbonsäuregruppe (-COOH), einer Carbonsäureestergruppe, einer Sulfonsäuregruppe (-SO₃H) oder einem Halogenatom;
R₃, R₄, R₅ und R₆ Methylgruppen sind; und
A ein optionales Anion darstellt, das die Formalladung am positiv geladenen Stickstoffatom ausgleichen kann,
zur Anwendung zum Färben einer Basalmembran des Auges bei der chirurgischen Behandlung des Auges oder bei der Diagnostik am oder im Auge.

2. Farbstoff gemäss Anspruch 1 zur Anwendung zum Färben der LLI-Membran.

3. Farbstoff gemäss Anspruch 1 zur Anwendung zum Färben der Linsenkapsel des Auges.

4. Farbstoff gemäss einem der Ansprüche 1 bis 3, wobei es sich um einen Farbstoff der Formel (III) handelt, 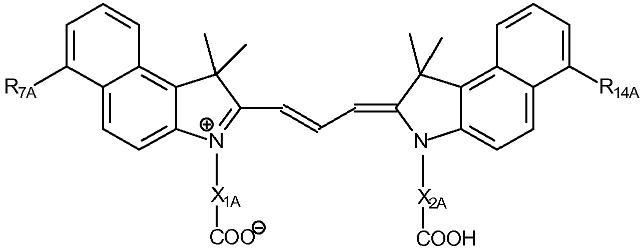
wobei X_{1A} und X_{2A} unabhängig voneinander ausgewählt sind aus einer bis 12 CH₂-Einheiten, bei denen unabhängig voneinander eine bis drei ersetzt sein können durch ein Sauerstoffatom, oder ein Schwefelatom, und/oder eine ersetzt sein kann durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Jod oder die Cyanogruppe oder durch eine lineare Alkylkette mit bis zu 6 C-Atomen; und
R_{7A} und R_{14A} Wasserstoff sind.

5. Farbstoff gemäss einem der Ansprüche 1 bis 4, wobei es sich um einen Farbstoff der Formel (IV) handelt, 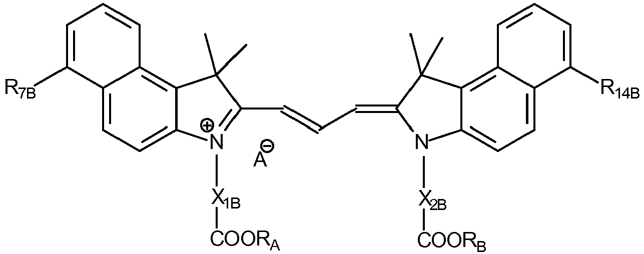
wobei X_{1B} und X_{2B} unabhängig voneinander ausgewählt sind aus einer bis 12 CH₂-Einheiten, bei denen unabhängig voneinander eine bis drei ersetzt sein können durch ein Sauerstoffatom, oder ein Schwefelatom, und/oder eine ersetzt sein kann durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Jod oder die Cyanogruppe oder durch eine lineare Alkylkette mit bis zu 6 C-Atomen;
R_{A} und R_{B} unabhängig voneinander ausgewählt sind aus C₁-C₁₂-Alkyl und einer der Reste R_{A} und R_{B} auch Wasserstoff sein kann; R_{7B} und R_{14B} Wasserstoff sind; und
A⁻ ein Anion darstellt, das die Formalladung am positiv geladenen Stickstoffatom ausgleicht.

6. Farbstoff gemäss einem der Ansprüche 1 bis 3, wobei es sich um einen Farbstoff der Formel (V) handelt: 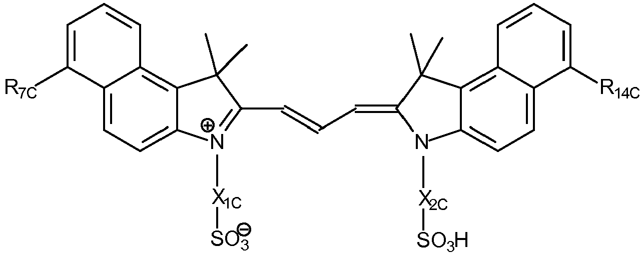
wobei X_{1C} und X_{2C} unabhängig voneinander ausgewählt sind aus einer bis 12 CH₂-Einheiten, bei denen unabhängig voneinander eine bis drei ersetzt sein können durch ein Sauerstoffatom, oder ein Schwefelatom, und/oder eine ersetzt sein kann durch einen divalenten Phenylrest; und wobei bis zu 4 einzelne Wasserstoffatome der CH₂-Einheiten jeweils unabhängig voneinander auch an gleichen C-Atomen ersetzt sein können durch die Halogene Fluor, Chlor, Brom oder Jod oder die Cyanogruppe oder durch eine lineare Alkylkette mit bis zu 6 C-Atomen; und
R_{7C} und R_{14C} Wasserstoff sind.

7. Verfahren zur Bereitstellung eines Farbstoffs gemäss einem der Ansprüche 1 bis 6, umfassend die Reinigung des Farbstoffs mit Hilfe der Reversed-Phase Chromatographie.

8. Verfahren nach Anspruch 7, wobei als stationäre Phase ein Kieselgel mit unpolaren Seitenketten eingesetzt wird.

## Claims

1. A dye of Formula (IIb) 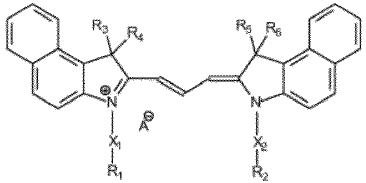
wherein X₁ and X₂ are selected independently of one another from one to 12 CH₂ units, one or more of which can be substituted independently of one another by a carbonyl group, an oxygen atom, a sulphur atom, a cis- or trans-CH=CH group, in which a CH unit can also be substituted by a nitrogen atom, an acetylenic C≡C group, a divalent phenyl, pyridine or thiophene radical, a divalent naphthalene radical, in which one or two CH groups can be substituted by nitrogen atoms, a divalent anthracene radical, in which one or two CH groups can be substituted by nitrogen atoms; and wherein up to 12 individual hydrogen atoms of the CH₂ units can each be substituted independently of one another and on the same C atoms by the halogens fluorine, chlorine, bromine or iodine or the cyano group or by a linear alkyl chain with up to 18 C atoms, in which one to 6 CH₂ units can be substituted independently of one another by a carbonyl group, an oxygen atom, a sulphur atom, a cis- or trans-CH=CH group, in which a CH unit can also be substituted by a nitrogen atom, an acetylenic C≡C group, a divalent phenyl, pyridine or thiophene radical, a divalent naphthalene radical, in which one or two CH groups can be substituted by nitrogen atoms, or a divalent anthracene radical, in which one or two CH groups can be substituted by nitrogen atoms;
R₁ and R₂ are selected independently of one another from a carboxylic acid group (-COOH), a carboxylic acid ester group, a sulphonic acid group (-SO₃H) or a halogen atom;
R₃, R₄, R₅ and R₆ are methyl groups; and
A represents an optional anion, which can balance the formal charge on the positively charged nitrogen atom,
for use in colouring a basal membrane of the eye in surgical treatment of the eye or in diagnosis on or in the eye.

2. A dye as claimed in Claim 1 for use in colouring the LLI membrane.

3. A dye as claimed in Claim 1 for use in colouring the lens capsule of the eye.

4. A dye as claimed in one of Claims 1 to 3, wherein it is a dye of Formula (III) 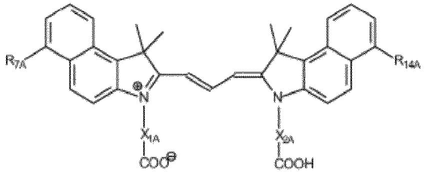
wherein X_{1A} and X_{2A} are selected independently of one another from one to 12 CH₂ units, of which one to three can be substituted independently of one another by an oxygen atom, or a sulphur atom and/or one can be substituted by a divalent phenyl radical; and wherein up to 4 individual hydrogen atoms of the CH₂ units can be substituted independently of one another and on the same C atoms by the halogens fluorine, chlorine, bromine or iodine or the cyano group or by a linear alkyl chain with up to 6 C atoms; and
R_{7A} and R_{14A} are hydrogen.

5. A dye as claimed in one of Claims 1 to 4, wherein it is a dye of Formula (IV) 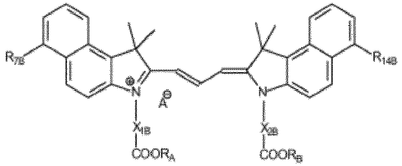
wherein X_{1B} and X_{2B} are selected independently of one another from one to 12 CH₂ units, of which one to three can be substituted independently of one another by an oxygen atom, or a sulphur atom and/or one can be substituted by a divalent phenyl radical; and wherein up to 4 individual hydrogen atoms of the CH₂ units can be substituted independently of one another and on the same C atoms by the halogens fluorine, chlorine, bromine or iodine or the cyano group or by a linear alkyl chain with up to 6 C atoms;
R_{A} and R_{B} are selected independently of one another from C₁-C₁₂ alkyl and one of the radicals R_{A} and R_{B} can also be hydrogen;
R_{7B} and R_{14B} are hydrogen; and
A⁻ represents an anion, which balances the formal charge on the positively charged hydrogen atom.

6. A dye as claimed in one of Claims 1 to 3, wherein it is a dye of Formula (V): 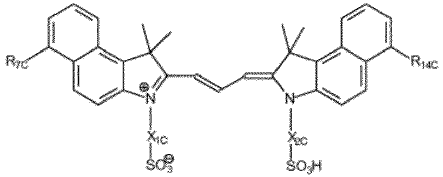
wherein X_{1C} and X_{2C} are selected independently of one another from one to 12 CH₂ units, of which one to three can be substituted independently of one another by an oxygen atom, or a sulphur atom and/or one can be substituted by a divalent phenyl radical; and wherein up to 4 individual hydrogen atoms of the CH₂ units can be substituted independently of one another and on the same C atoms by the halogens fluorine, chlorine, bromine or iodine or the cyano group or by a linear alkyl chain with up to 6 C atoms; and
R_{7C} and R_{14C} are hydrogen.

7. A method of preparing a dye as claimed in one of Claims 1 to 6, including the cleaning of the dye with the aid of Reversed-Phase Chromatography.

8. A method as claimed in Claim 7, wherein a silica gel with non-polar side chains is used as the stationary phase.

## Revendications

1. Colorant de formule (IIb) 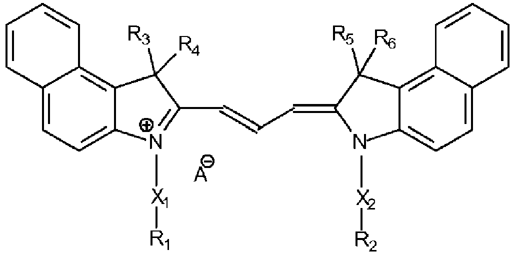 dans laquelle
X₁ et X₂ sont choisis, indépendamment l'un de l'autre, parmi une à 12 unités CH₂, parmi lesquelles, indépendamment les unes des autres, une ou plusieurs peuvent être remplacées par un groupe carbonyle, un atome d'oxygène, un atome de soufre, un groupe CH=CH cis ou trans, dans lequel une unité CH peut également être remplacée par un atome d'azote, un groupe acétylénique C≡C, un radical phényle, pyridine ou thiophène divalent, un radical naphtalène divalent, dans lequel un ou deux groupes CH peuvent être remplacés par des atomes d'azote, un radical anthracène divalent, dans lequel un ou deux groupes CH peuvent être remplacés par des atomes d'azote ; jusqu'à 12 atomes d'hydrogène individuels des unités CH₂ pouvant être remplacés, à chaque fois indépendamment les uns des autres, également sur les mêmes atomes de carbone, par les halogènes fluor, chlore, brome ou iode ou par le groupe cyano ou par une chaîne alkyle linéaire comprenant jusqu'à 18 atomes de carbone, dans laquelle une à 6 unités CH₂ peuvent être remplacées, indépendamment les unes des autres, par un groupe carbonyle, un atome d'oxygène, un atome de soufre, un groupe CH=CH cis ou trans, dans lequel une unité CH peut également être remplacée par un atome d'azote, un groupe acétylénique C≡C, un radical phényle, pyridine ou thiophène divalent, un radical naphtalène divalent, dans lequel un ou deux groupes CH peuvent être remplacés par des atomes d'azote, ou un radical anthracène divalent, dans lequel un ou deux groupes CH peuvent être remplacés par des atomes d'azote ;
R₁ et R₂ sont choisis, indépendamment l'un de l'autre, parmi un groupe acide carboxylique (-COOH), un groupe ester d'acide carboxylique, un groupe acide sulfonique (-SO₃H) ou un atome d'halogène ;
R₃, R₄, R₅ et R₆ représentent des groupes méthyle ; et
A représente un anion facultatif, qui peut compenser la charge formelle sur l'atome d'azote chargé positivement,
pour une utilisation pour la coloration d'une membrane basale des yeux lors du traitement chirurgical des yeux ou lors du diagnostic au niveau des yeux ou dans ceux-ci.

2. Colorant selon la revendication 1 pour une utilisation pour la coloration de la membrane LLI (lame laminaire interne).

3. Colorant selon la revendication 1 pour une utilisation pour la coloration de la capsule du cristallin.

4. Colorant selon l'une quelconque des revendications 1 à 3, le colorant étant un colorant selon la formule (III) 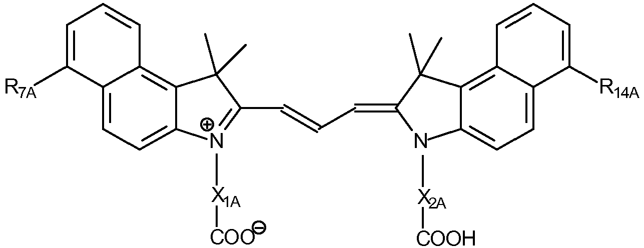 dans laquelle
X_{1A} et X_{2A} sont choisis, indépendamment l'un de l'autre, parmi une à 12 unités CH₂, parmi lesquelles, indépendamment les unes des autres, une à trois unités peuvent être remplacées par un atome d'oxygène ou un atome de soufre et/ou une unité peut être remplacée par un radical phényle divalent ; jusqu'à 4 atomes d'hydrogène individuels des unités CH₂ pouvant être remplacés, à chaque fois indépendamment les uns des autres, également sur les mêmes atomes de carbone, par les halogènes fluor, chlore, brome ou iode ou par le groupe cyano ou par une chaîne alkyle linéaire comprenant jusqu'à 6 atomes de carbone ; et
R_{7A} et R_{14A} représentent hydrogène.

5. Colorant selon l'une quelconque des revendications 1 à 4, le colorant étant un colorant selon la formule (IV) 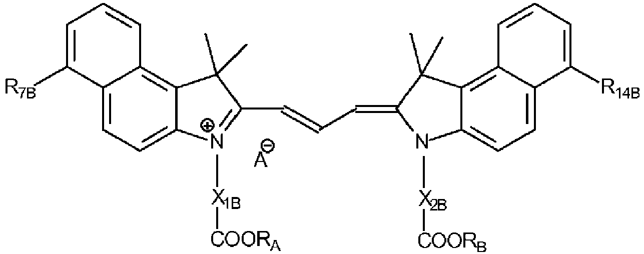 dans laquelle
X_{1B} et X_{2B} sont choisis, indépendamment l'un de l'autre, parmi une à 12 unités CH₂, parmi lesquelles, indépendamment les unes des autres, une à trois unités peuvent être remplacées par un atome d'oxygène ou un atome de soufre et/ou une unité peut être remplacée par un radical phényle divalent ; et jusqu'à 4 atomes d'hydrogène individuels des unités CH₂ pouvant être remplacés, à chaque fois indépendamment les uns des autres, également sur les mêmes atomes de carbone, par les halogènes fluor, chlore, brome ou iode ou par le groupe cyano ou par une chaîne alkyle linéaire comprenant jusqu'à 6 atomes de carbone ;
R_{A} et R_{B} sont choisis, indépendamment l'un de l'autre, parmi C₁-C₁₂-alkyle et un des radicaux R_{A} et R_{B} peut également être hydrogène ;
R_{7B} et R_{14B} représentent hydrogène ; et
A⁻ représente un anion, qui compense la charge formelle sur l'atome d'azote chargé positivement.

6. Colorant selon l'une quelconque des revendications 1 à 3, le colorant étant un colorant selon la formule (V) 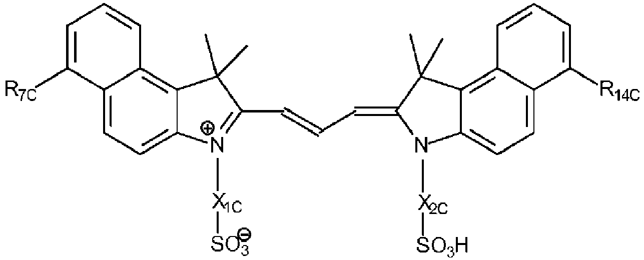 dans laquelle
X_{1C} et X_{2C} sont choisis, indépendamment l'un de l'autre, parmi une à 12 unités CH₂, parmi lesquelles, indépendamment les unes des autres, une à trois unités peuvent être remplacées par un atome d'oxygène ou un atome de soufre et/ou une unité peut être remplacée par un radical phényle divalent ; jusqu'à 4 atomes d'hydrogène individuels des unités CH₂ pouvant être remplacés, à chaque fois indépendamment les uns des autres, également sur les mêmes atomes de carbone, par les halogènes fluor, chlore, brome ou iode ou par le groupe cyano ou par une chaîne alkyle linéaire comprenant jusqu'à 6 atomes de carbone ; et
R_{7C} et R_{14C} représentent hydrogène.

7. Procédé pour la préparation ou mise à disposition d'un colorant selon l'une quelconque des revendications 1 à 6, comprenant la purification du colorant à l'aide de la chromatographie en phase inverse.

8. Procédé selon la revendication 7, un gel silicique présentant des chaînes latérales non polaires étant utilisé comme phase stationnaire.
